# EUROPEAN PATENT SPECIFICATION

(11) **EP 4 169 525 B1**
(45) Date of publication and mention of the grant of the patent: **02.12.2026**
(21) Application number: 22163636.8
(22) Date of filing: 06.07.2012
(51) Int. Cl.: A61K 38/37, A61K 47/60, A61K 47/68, A61P 7/00, A61P 7/02, A61P 7/04, A61P 17/02, A61P 19/00, A61P 43/00, G01N 33/86, C07K 14/755

(54) **FACTOR VIII CHIMERIC AND HYBRID POLYPEPTIDES, AND METHODS OF USE THEREOF**
CHIMÄRE UND HYBRIDE FAKTOR-VIII-POLYPEPTIDE UND VERFAHREN ZUR VERWENDUNG DAVON
POLYPEPTIDES CHIMÉRIQUES ET HYBRIDES DE FACTEUR VIII ET LEURS PROCÉDÉS D'UTILISATION

(30) Priority: 08.07.2011 US 201161506015 P; 11.08.2011 US 201161522647 P; 30.09.2011 US 201161541561 P; 09.12.2011 US 201161569158 P; 13.01.2012 US 201261586443 P; 11.04.2012 US 201261622789 P; 08.06.2012 US 201261657641 P
(43) Date of publication of application: 26.04.2023
(62) Document number(s) of the earlier application(s) in accordance with Art. 76 EPC: 18211156.7 / 3 513 804; 12811808.0 / 2 729 161
(73) Proprietor: Bioverativ Therapeutics Inc., Waltham MA 02451 (US)
(72) Inventor: DUMONT, Jennifer, Groton, 01450 (US); LOW, Susan, Pepperill, 01463 (US); BITONTI, Alan J., Acton, 01720 (US); GLENN, Pierce, Rancho Santa Fee, 92067 (US); LUK, Alvin, Boston, 02114-1897 (US); JIANG, Haiyan, Belmont, 02478 (US); MCKINNEY, Byron, San Diego, 92122 (US); OTTMER, Matt, Newton, 02459 (US); SOMMER, Jurg, Wayland, 01788 (US); NUGENT, Karen, Princes Risborough, Buckinghamshire, HP27 9RH (GB); LI, Lian, Lexington, 02420 (US); PETERS, Robert, West Roxbury, 02132 (US)
(74) Representative: Zwicker, Jörk

(56) References cited:
- WO-A2-2011/069164

## Description

### Field of the Invention

The present invention relates generally to the field of therapeutics for hemostatic disorders.

### Background Art

Hemophilia A is an X-linked bleeding disorder caused by mutations and/or deletions in the factor VIII (FVIII) gene resulting in a deficiency of FVIII activity (Peyvandi, F. et al. Haemophilia 12:82-89 (2006). The disease is characterized by spontaneous hemorrhage and excessive bleeding after trauma. Over time, the repeated bleeding into muscles and joints, which often begins in early childhood, results in hemophilic arthropathy and irreversible joint damage. This damage is progressive and can lead to severely limited mobility of joints, muscle atrophy and chronic pain (Rodriguez-Merchan, E.C., Semin. Thromb. Hemost. 29:87-96 (2003).

The A2 domain is necessary for the procoagulant activity of the factor VIII molecule. Studies show that porcine factor VIII has six-fold greater procoagulant activity than human factor VIII (Lollar, P., and E. T. Parker, J. Biol. Chem. 266:12481-12486 (1991)), and that the difference in coagulant activity between human and porcine factor VIII appears to be based on a difference in amino acid sequence between one or more residues in the human and porcine A2 domains (Lollar, P., et al., J. Biol. Chem. 267:23652-23657 (1992)).

Treatment of hemophilia A is by replacement therapy targeting restoration of FVIII activity to 1 to 5 % of normal levels to prevent spontaneous bleeding (Mannucci, P.M., et al., N. Engl. J. Med. 344:1773-1779 (2001)). There are plasma-derived and recombinant FVIII products available to treat bleeding episodes on-demand or to prevent bleeding episodes from occurring by treating prophylactically. Based on the short half-life of these products, however, e.g., 8-12 hours, treatment regimens require the administration of frequent intravenous injections. Such frequent administration is painful and inconvenient.

Reduced mortality, prevention of joint damage and improved quality of life have been important achievements due to the development of plasma-derived and recombinant FVIII. Prolonged protection from bleeding would represent another key advancement in the treatment of hemophilia A patients. However, to date, no products that allow for prolonged hemostatic protection have been developed. Therefore, there remains a need for improved methods of treating hemophilia due to factor VIII deficiency that are more tolerable, longer lasting, and more effective than current therapies. WO 2011/069164 discloses methods of administering Factor VIII to a subject in need thereof, comprising administering to the subject a therapeutic dose of a polypeptide comprising a Factor VIII and an Fc.

### BRIEF SUMMARY OF THE INVENTION

The present invention is defined in the appended claims. Specifically, the present invention provides a pharmaceutical composition comprising a chimeric polypeptide, which comprises a Factor VIII (FVIII) portion and a second portion, and at least one pharmaceutically acceptable excipient, wherein in 1% to 40% of the FVIII portion, the FVIII is in the form of a single chain FVIII, and wherein in 60 to 99% of the FVIII portion, the FVIII portion is processed, and wherein the single chain FVIII comprises a FVIII heavy chain and a FVIII light chain on a single polypeptide chain, wherein said single chain Factor VIII comprises an amino acid sequence at least 90% or 95% identical to amino acids 20 to 1457 of SEQ ID NO: 2, and wherein the second portion comprises an amino acid sequence at least 90% or 95% identical to amino acids 21 to 247 of SEQ ID NO: 4. In one embodiment, the polypeptide has a half-life at least one and one-half to six times longer, one and one-half to five times longer, one and one-half to four times longer, one and one-half to three times longer, or one and one-half to two times longer to a polypeptide consisting of said Factor VIII.

In one embodiment the subject has the blood serotype A, B, or AB.

In one embodiment, the plasma half-life of FVIII-Fc in said subject is at least about 1.3, 1.4, 1.5, 1.6, 1.7, 1.8, 1.9, 2.0, 2.1, 2.2, 2.3, 2.4, or 2.5-fold times greater than the plasma half-life of FVIII-Fc when administered to a subject having average levels of VWF. In another embodiment, the FVIII-Fc plasma half-life is between 20-40 hours. In another embodiment, the long-acting FVIII protein has a half-life of about 21 hours, 22 hours, 23 hours, 24 hours, 25 hours, 26 hours, 27 hours, 28 hours, 29 hours, 30 hours, 31 hours, 31 hours, 32 hours, 33 hours, 34 hours, 35 hours, 36 hours, 37 hours, 38 hours, 39 hours, or 40 hours. In another embodiment, the long-acting FVIII protein has a half-life of between about 20 and 27 hours.

The invention also provides the pharmaceutical composition of the invention for use in reducing bleeding in a subject who has hemophilia A. The invention also provides the pharmaceutical composition of the invention for use in preventing, decreasing, or treating a bleeding episode in the subject, for use in prophylactic treatment of a bleeding episode in the subject, and for use in on-demand treatment of a bleeding episode in the subject.

In one embodiment, the subject is a human. In another embodiment, the subject is a pediatric subject. In another embodiment, the subject has the blood serotype A, B, or AB.

In one embodiment, the administration prevents a bleeding episode in the subject. In one embodiment, the bleeding episode is spontaneous. In one embodiment, the administration resolves greater than 80-100%, greater than 80-90%, greater than 85-90%, greater than 90-100%, greater than 90-95%, or greater than 95-100% of bleeding episodes. In one embodiment, the administration maintains homeostatis in the population of the subjects in need of a surgery. In one embodiment, the FVIII-Fc protein is administered prior to, during, or after the surgery. In one embodiment, the surgery is minor surgery, major surgery, tooth extraction, tonsillectomy, inguinal herniotomy, synovectomy, total knee replacement, craniotomy, osteosynthesis, trauma surgery, intracranial surgery, intra-abdominal surgery, intrathoracic surgery, or joint replacement surgery. In one embodiment the surgery is an emergency surgery.

Further aspects and embodiments of the invention are disclosed in the accompanying claims.

### BRIEF DESCRIPTION OF THE DRAWINGS/FIGURES

FIG. 1. Schematic Representation of rFVIIIFc monomer.
FIGS. 2A-E. A-B. Non-reducing and reducing SDS-PAGE analysis of rFVIIIFc (processed or single chain). C. rFVIIIFc structure analyzed by LC/UV and LC/MS. D. Total Ion Current (TIC) chromatogram (LC/MS map) of rFVIIIFc after thrombin cleavage. Major digestion products are indicated. E. Deconvoluted Mass Spectrum of the A2 domain of rFVIIIFc and rBDDFVIII. Major products and their cognate masses are indicated, corresponding to thrombin-cleaved A2 domain (S373 to R740) and two truncated products, S373 to Y729 and S373 to E720.
FIGS. 3A-C. Biochemical characterization of rFVIII-Fc: A. Activation of Factor X as a function of phospholipid vesicle concentration; B. Activation of Factor X as a function of FX concentration. C. Activation of Factor X as a function of Factor IXa concentration.
FIG 4. Activation of Factor X following cleavage by activated Protein C.

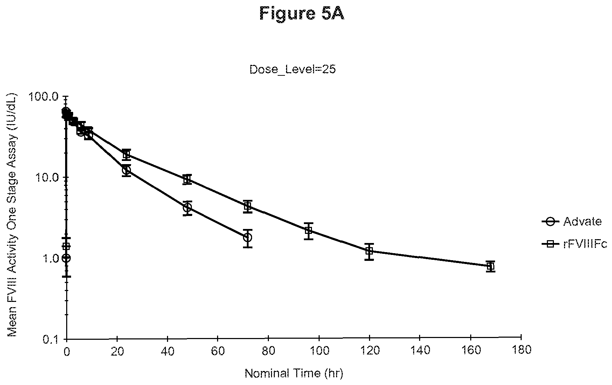

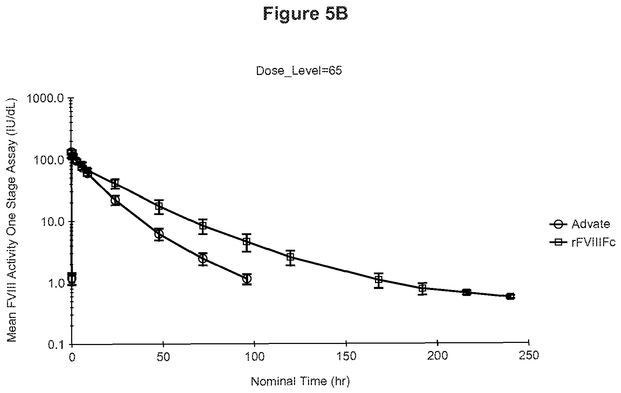

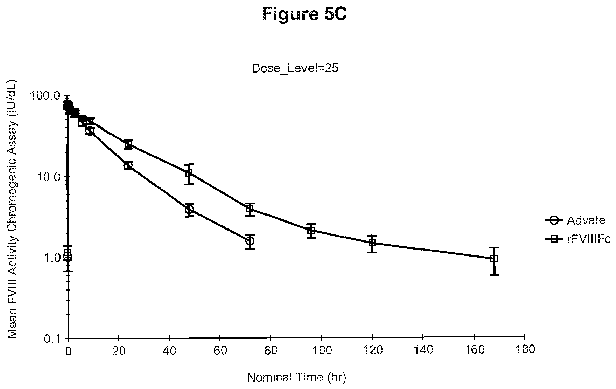

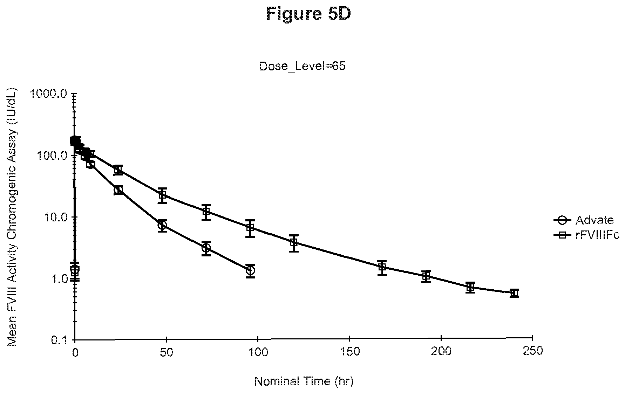

FIGS. 5A-D. Observed group mean FVIII activity (±SE) versus time profiles, sorted by dose level, grouped by compound (one stage assay, 25 IU/kg (A) or 65 IU/kg (B); and chromogenic assay, 25 IU/kg (C) or 65 IU/kg (D)) versus time.

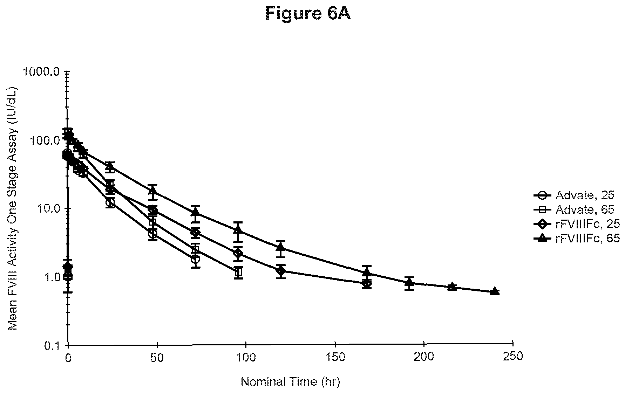

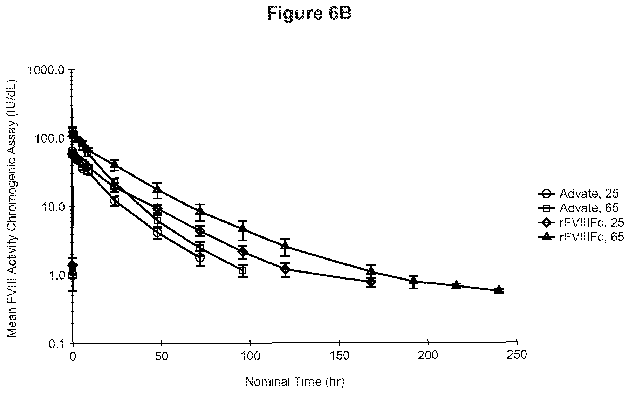

FIGS. 6A-B. Observed group mean FVIII activity (±SE) versus time profiles, grouped by dose level and compound (one stage assay (A) or chromogenic assay (B)) versus time.
FIGS. 7(A)-(C). *In Vivo* Efficacy of Single Chain FVIII:Fc in HemA Mouse Tail Vein Transection Model. (A) Single chain rFVIII:Fc doses are shown as squares, and processed rFVIII:Fc doses are shown as circles. (B) Percent survival following tail vein transection of 4.6 µg/kg, 1.38 µg/kg, and 0.46 µg/kg of rFVIIIFc or SC rFVIIIFc. (C) Percent of non-bleeders following tail vein transection of 4.6 µg/kg (black circle or inverted triangle), 1.38 µg/kg (triangle or diamond), and 0.46 µg/kg (square and gray circle) of rFVIIIFc or SC rFVIIIFc, respectively.
FIG. 8. Study Design. Fig. 8 depicts the study design of the phase 1/2a study, which was a dose-escalation, sequential design to evaluate the safety and PK of rFVIIIFc compared with ADVATE^{®} after a single intravenous dose of either 25 IU/kg (low dose Cohort A) or 65 IU/kg (high dose Cohort B).
FIG. 9. Correlation of rFVIII Activity by One-Stage (aPTT) and Chromogenic Assays. Correlation between one-stage clotting (aPTT) and chromogenic assay results measuring FVIII activity (IU/mL) following injection of ADVATE^{®} (◆) and rFVIIIFc (□).
FIGS. 10(A)-(B). Group Mean Plasma FVIII Activity Pharmacokinetic Profiles for Low-Dose and High-Dose Cohorts. The plasma FVIII activity (one stage aPTT assay) versus time curve after a single intravenous injection of rFVIIIFc or ADVATE^{®} are shown for (A) 25 IU/kg (low-dose cohort, n=6); and (B) 65 IU/kg (high dose cohort, n=10 [ADVATE^{®}]; n=9 [rFVIIIFc]). Results presented are group mean ± standard error of mean (SEM).

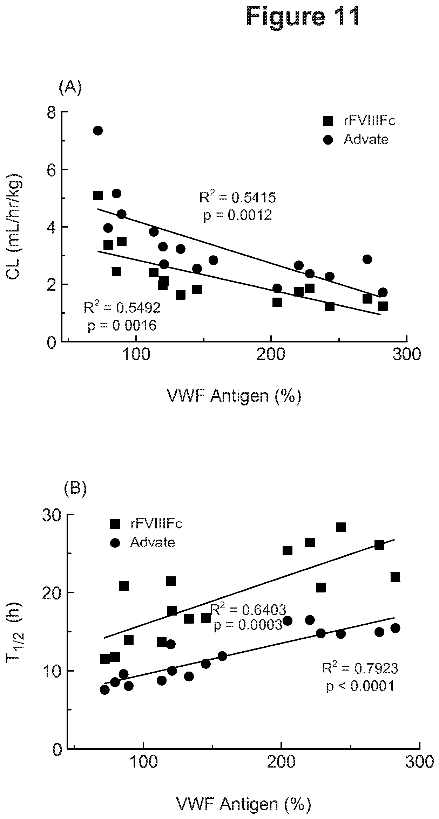

FIGS. 11(A)-(B). Effect of VWF Antigen Levels on Cl and t_{1/2} of FVIII Activity after Injection of ADVATE^{®} or rFVIIIFc. Correlation between VWF antigen levels and (A) the weight-adjusted Cl of ADVATE^{®} (R²=0.5415 and p=0.0012) and rFVIIIFc (R²=0.5492 and p=0.0016); and (B) the t_{1/2} of ADVATE^{®} (R²=0.7923 and p<0.0001) and rFVIIIFc (R²= 0.6403 and p=0.0003). Each dot represents an individual subject.
FIGS. 12(A)-(B). Ex Vivo Whole Blood ROTEM^{®} Results for Individual Subjects After Injection of ADVATE^{®} or rFVIIIFc. Blood was sampled from subjects prior to and after treatment at doses of (A) 25 IU/kg ADVATE^{®} and rFVIIIFc; and (B) 65 IU/kg ADVATE^{®} and rFVIIIFc at specified time points. Clotting time was determined by NATEM initiated with Ca++ on a ROTEM^{®} instrument. Results presented are mean ± standard error of mean (SEM) from triplicate channel readings for each individual sample.
FIGS. 13(A)-(B). Activity comparison in thrombin generation assay (TGA). (A) SC rFVIIIFc showed a reduced endogenous thrombin potential (ETP), and (B) a reduced peak thrombin compared to rFVIIIFc.
FIGS. 14(A)-(C): In vitro ROTEM data. ROTEM (NATEM) results (Mean ± SD) for varying concentratios of XYNTHA, ADVATE< and rFVIIIFc spked in pooled whole blood obtained from naive HemA mice. (A). Average clot time (CT) (Fig. 14A), (B). clot formation time (CFT) , and (C). alpha angle.
FIGS. 15(A)-(C). Ex vivo ROTEM data. ROTEM (NATEM) results (Mean ± SD) from HemA mice following a single intravenous administration of 50 IU/kg of XYNTHA, ADVATE, or rFVIIIFc at 5 min, 24, 48, 72, and 96 hours after dosing. (A). Average clot time (CT), (B). Clot formation time (CFT), and (C). alpha angle.

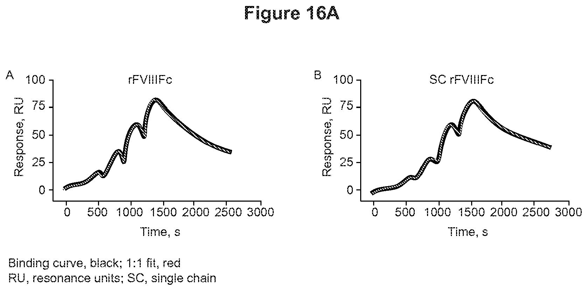

FIGS. 16(A)-(E): Real-time evaluation of the interaction of rFVIIIFc and single chain (SC) rFVIIIFc with vWF, and real-time evaluation of thrombin mediated release of rFVIIIFc and SC rFVIIIFc from vWF. (A). Surface plasmon resonance (SPR) analysis of rFVIIIFc and SC rFVIIIFc affinity for vWF. Depicted are the binding curve and the 1:1 fit interaction model. The x-axis shows time in seconds and the y-axis shows response in response units (RU). (B). Reference subtracted sensograms of thrombin-mediated release of activated rFVIIIFc, SC rFVIIIFc, and B-domain deleted rFVIII lacking Fc moieties (rBDD FVIII) at 25° C (top) and 37° C (bottom). The x-axis shows time in seconds and the y-axis shows response in response units (RU). Individual lines indicate the response at different α-thrombin concentrations. The uppermost line is the response at 0 U/mL α-thrombin, and each subsequent line runs in order for α-thrombin concentrations of 0.005, 0.01, 0.02, 0.04, 0.08, 0.16, 0.31, 0.63, 1.3, 2.5, 5, 10, and 20 U/mL. (C). Double reference subtracted sensograms of thrombin mediated release phase for rFVIIIFc, SC rFVIIIFc, and rBDD FVIII at 25° C (top) and 37° C (bottom). The x-axis shows time in seconds and the y-axis shows response in response units (RU). Individual lines indicate response at different α-thrombin concentrations. The uppermost line is the response at 0 U/mL α-thrombin, and each subsequent line runs in order for α-thrombin concentrations of 0.005, 0.01, 0.02, 0.04, 0.08, 0.16, 0.31, 0.63, 1.3, 2.5, 5.0, 10, and 20 U/mL. (D). Thrombin-mediated release rate as a function of time for rFVIIIFc, SC rFVIIIFc, and rBDD FVIII at 25° C (top) and 37° C (bottom). The x-axis shows time in seconds and the y-axis shows response in response units (RU). Individual lines indicate response at different α-thrombin concentrations. The uppermost line is the response at 20 U/mL α-thrombin, and each subsequent line runs in order for α-thrombin concentrations of 10, 5, 2.5, 1.3, 0.63, 0.31, 0.16, 0.08, 0.04, 0.02, 0.01, and 0.005 U/mL. (E). Peak thrombin-mediated release rate as a function of thrombin concentration for rFVIIIFc, SC rFVIIIFc, and rBDD FVIII at 25° C (top) and 37° C (bottom). EC₅₀ is half maximal effective concentration. The x-axis is α-thrombin concentration in U/mL and the y-axis is maximum release rate in RU/second.

### DETAILED DESCRIPTION OF THE INVENTION

The present invention provides a pharmaceutical composition comprising (i) a chimeric polypeptide, which comprises a Factor VIII (FVIII) portion and a second portion, and (ii) at least one pharmaceutically acceptable excipient, wherein in 1% to 40% of the FVIII portion, the FVIII is in the form of a single chain FVIII, and wherein in 60 to 99% of the FVIII portion, the FVIII portion is processed, and wherein the single chain FVIII comprises a FVIII heavy chain and a FVIII light chain on a single polypeptide chain, wherein said single chain Factor VIII comprises an amino acid sequence at least 90% or 95% identical to amino acids 20 to 1457 of SEQ ID NO: 2, and wherein the second portion comprises an amino acid sequence at least 90% or 95% identical to amino acids 21 to 247 of SEQ ID NO: 4. The present invention further provides the pharmaceutical composition of the invention for use in reducing bleeding in a subject who has hemophilia A. The present invention further provides the pharmaceutical composition of the invention for use in preventing, decreasing, or treating a bleeding episode in the subject, prophylactic treatment of a bleeding episode in the subject, on-demand treatment of a bleeding episode in the subject.

Treatment of hemophilia A is by replacement therapy targeting restoration of FVIII activity to 1 to 5 % of normal levels to prevent spontaneous bleeding (Mannucci, P.M. et al., N. Engl. J. Med. 344:1773-9 (2001)). There are plasma-derived and recombinant FVIII products available to treat bleeding episodes on-demand or to prevent bleeding episodes from occurring by treating prophylactically. Based on the short half-life of these products (8-12 hr) (White G.C., et al., Thromb. Haemost. 77:660-7 (1997); Morfini, M., Haemophilia 9 (suppl 1):94-99; discussion 100 (2003)), treatment regimens require frequent intravenous administration, commonly two to three times weekly for prophylaxis and one to three times daily for on-demand treatment (Manco-Johnson, M.J., et al., N. Engl. J. Med. 357:535-544 (2007)). Such frequent administration is painful and inconvenient.

A dosing interval may be at least about one and one-half to six times longer, one and one-half to five times longer, one and one-half to four times longer, one and one-half to three times longer, or one and one-half to two times longer, than the dosing interval required for an equivalent dose of said Factor VIII without the non-Factor VIII portion (a polypeptide consisting of said Factor VIII portion), e.g., without the Fc portion. The dosing interval may be at least about one and one-half, two, two and one-half, three, three and one-half, four, four and one-half, five, five and one-half or six times longer than the dosing interval required for an equivalent dose of said Factor VIII without the non-Factor VIII portion (a polypeptide consisting of said Factor VIII portion), e.g., without the Fc portion. The dosing interval may be about every three, four, five, six, seven, eight, nine, ten, eleven, twelve, thirteen, or fourteen days or longer.

The dosing interval may be at least about one and one-half to 5, one and one-half, 2, 3, 4, or 5 days or longer.

"Administering," as used herein, means to give a pharmaceutically acceptable Factor VIII polypeptide of the invention to a subject via a pharmaceutically acceptable route. Routes of administration can be intravenous, e.g., intravenous injection and intravenous infusion. Additional routes of administration include, e.g., subcutaneous, intramuscular, oral, nasal, and pulmonary administration. Chimeric polypeptides and hybrid proteins may be administered as part of a pharmaceutical composition comprising at least one excipient.

"Area under the plasma concentration versus time curve (AUC)," as used herein, is the same as the term of art in pharmacology, and is based upon the rate and extent of absorption of Factor VIII following administration. AUC is determined over a specified time period, such as 12, 18, 24, 36, 48, or 72 hours, or for infinity using extrapolation based on the slope of the curve. Unless otherwise specified herein, AUC is determined for infinity. The determination of AUC may be carried out in a single subject, or in a population of subjects for which the average is calculated.

A "B domain" of Factor VIII, as used herein, is the same as the B domain known in the art that is defined by internal amino acid sequence identity and sites of proteolytic cleavage by thrombin, e.g., residues Ser741-Arg1648 of full length human factor VIII. The other human factor VIII domains are defined by the following amino acid residues: A1, residues Ala1-Arg372; A2, residues Ser373-Arg740; A3, residues Ser1690-Ile2032; C1, residues Arg2033-Asn2172; C2, residues Ser2173-Tyr2332. The A3-C1-C2 sequence includes residues Ser1690-Tyr2332. The remaining sequence, residues Glu1649-Arg1689, is usually referred to as the factor VIII light chain activation peptide. The locations of the boundaries for all of the domains, including the B domains, for porcine, mouse and canine factor VIII are also known in the art. In one embodiment, the B domain of Factor VIII is deleted ("B domain deleted factor VIII" or "BDD FVIII"). An example of a BDD FVIII is REFACTO^{®} (recombinant BDD FVIII), which has the same sequence as the Factor VIII portion of the sequence in Table 2A(i) (amino acids 1 to 1457 or 20 to 1457 of SEQ ID NO:2). In another embodiment, the B domain deleted Factor VIII contains an intact intracellular processing site, which corresponds to Arginine at residue 754 of B domain deleted Factor VIII, which corresponds to Arginine residue 773 of SEQ ID NO: 2, or residue 1648 of full-length Factor VIII, which corresponds to Arginine residue 1667 of SEQ ID NO: 6. The sequence residue numbers used herein without referring to any SEQ ID Numbers correspond to the Factor VIII sequence without the signal peptide sequence (19 amino acids) unless otherwise indicated. For example, S743/Q1638 of full-length Factor VIII corresponds to S762/Q1657 of SEQ ID NO: 6 due to the 19 amino acid signal peptide sequence. In other embodiments, the B domain deleted FVIII comprises a substitution or mutation at an amino acid position corresponding to Arginine 1645, a substitution or mutation at an amino acid position corresponding to Arginine 1648, or a substitution or mutation at amino acid positions corresponding to Arginine 1645 and Arginine 1648 in full-length Factor VIII. In some embodiments, the amino acid substituted at the amino acid position corresponding to Arginine 1645 is a different amino acid from the amino acid substituted at the amino acid position corresponding to Arginine 1648. In certain embodiments, the substitution or mutation is an amino acid other than arginine, e.g., alanine.

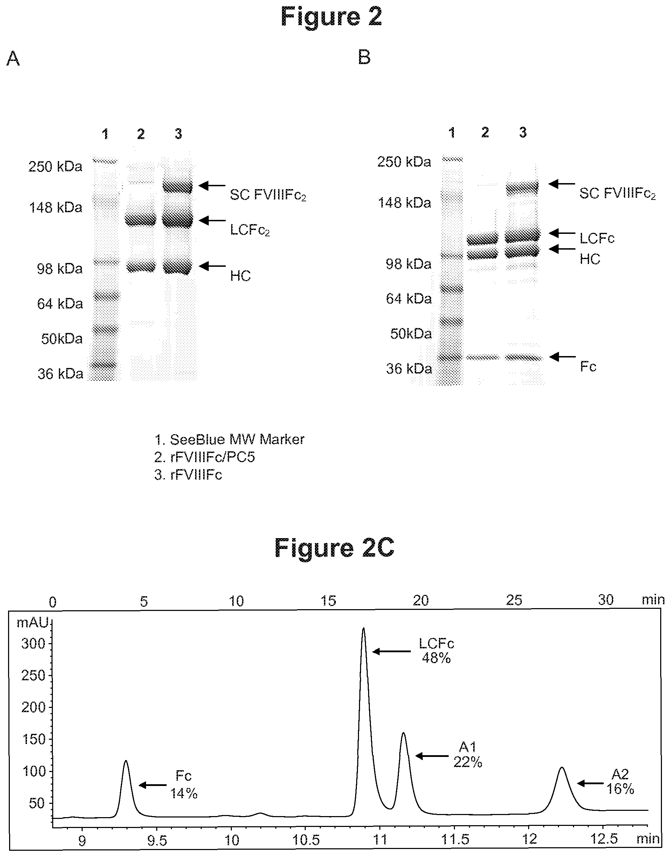

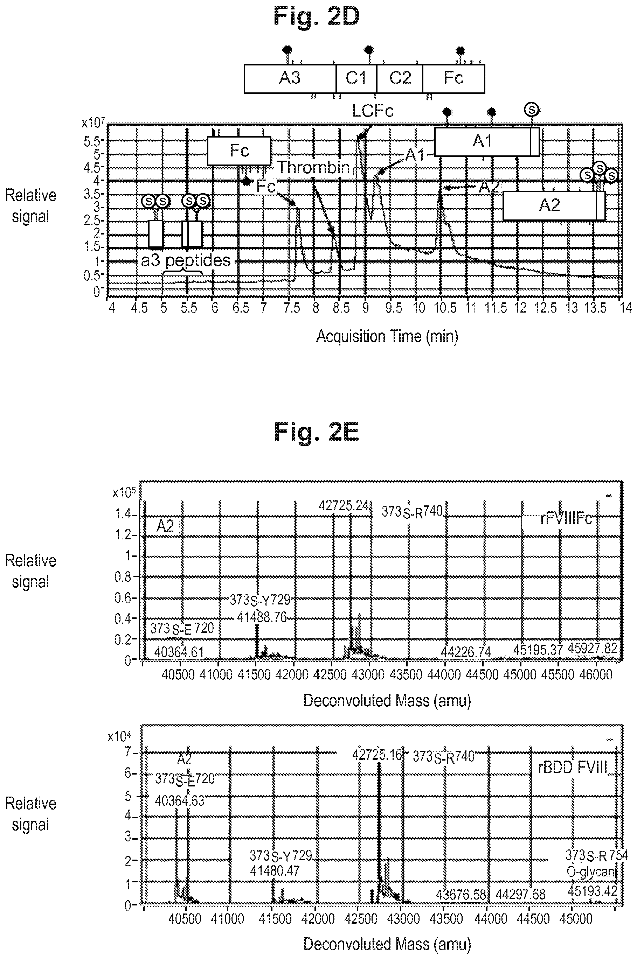

A "B domain deleted factor VIII" may have the full or partial deletions disclosed in U.S. Patent Nos. 6,316,226, 6,346,513, 7,041,635, 5,789,203, 6,060,447, 5,595,886, 6,228,620, 5,972,885, 6,048,720, 5,543,502, 5,610,278, 5,171,844, 5,112,950, 4,868,112, and 6,458,563. In some embodiments, a B domain deleted factor VIII sequence of the present invention comprises any one of the deletions disclosed at col. 4, line 4 to col. 5, line 28 and examples 1-5 of U.S. Patent No. 6,316,226 (also in US 6,346,513). In some embodiments, a B domain deleted factor VIII of the present invention has a deletion disclosed at col. 2, lines 26-51 and examples 5-8 of U.S. Patent No. 5,789,203 (also US 6,060,447, US 5,595,886, and US 6,228,620). In some embodiments, a B domain deleted factor VIII has a deletion described in col. 1, lines 25 to col. 2, line 40 of US Patent No. 5,972,885; col. 6, lines 1-22 and example 1 of U.S. Patent no. 6,048,720; col. 2, lines 17-46 of U.S. Patent No. 5,543,502; col. 4, line 22 to col. 5, line 36 of U.S. Patent no. 5,171,844; col. 2, lines 55-68, figure 2, and example 1 of U.S. Patent No. 5,112,950; col. 2, line 2 to col. 19, line 21 and table 2 of U.S. Patent No. 4,868,112; col. 2, line 1 to col. 3, line 19, col. 3, line 40 to col. 4, line 67, col. 7, line 43 to col. 8, line 26, and col. 11, line 5 to col. 13, line 39 of U.S. Patent no. 7,041,635; or col. 4, lines 25-53, of U.S. Patent No. 6,458,563. In some embodiments, a B domain deleted factor VIII has a deletion of most of the B domain, but still contains amino-terminal sequences of the B domain that are essential for *in vivo* proteolytic processing of the primary translation product into two polypeptide chain (i.e., intracellular processing site), as disclosed in WO 91/09122. In some embodiments, a B domain deleted factor VIII is constructed with a deletion of amino acids 747-1638, i.e., virtually a complete deletion of the B domain. Hoeben R.C., et al. J. Biol. Chem. 265 (13): 7318-7323 (1990). A B domain deleted factor VIII may also contain a deletion of amino acids 771-1666 or amino acids 868-1562 of factor VIII. Meulien P., et al. Protein Eng. 2(4): 301-6 (1988). Additional B domain deletions that are part of the invention include, e.g.,: deletion of amino acids 982 through 1562 or 760 through 1639 (Toole et al., Proc. Natl. Acad. Sci. U.S.A. 83:5939-5942 (1986)), 797 through 1562 (Eaton et al., Biochemistry 25:8343-8347 (1986)), 741 through 1646 (Kaufman (PCT published application No. WO 87/04187)), 747-1560 (Sarver et al., DNA 6:553-564 (1987)), 741 through 1648 (Pasek (PCT application No.88/00831)), 816 through 1598 or 741 through 1689 (Lagner (Behring Inst. Mitt. (1988) No 82:16-25, EP 295597)). Each of the foregoing deletions may be made in any Factor VIII sequence.

In one embodiment, the B domain deleted Factor VIII portion in the chimeric polypeptide is processed into two chains connected (or associated) by a metal bond, the first chain comprising a heavy chain (A1-A2-partial B) and a second chain comprising a light chain (A3-C1-C2) . In another embodiment, the B domain deleted Factor VIII portion is a single chain Factor VIII. The single chain Factor VIII can comprise an intracellular processing site, which corresponds to Arginine at residue 754 of B domain deleted Factor VIII (residue 773 of SEQ ID NO: 2) or at residue 1648 of full-length Factor VIII (residue 1657 of SEQ ID NO: 6).

The metal bond between the heavy chain and the light chain can be any metal known in the art. For example, the metals useful for the invention can be a divalent metal ion. The metals that can be used to associate the heavy chain and light chain include, but not limited to, Ca²⁺, Mn²⁺, or Cu²⁺. Fatouros et al., Intern. J. Pharm. 155(1): 121-131 (1997); Wakabayashi et al., JBC. 279(13): 12677-12684 (2004).

"Chimeric polypeptide," as used herein, means a polypeptide that includes within it at least two polypeptides (or subsequences or peptides) from different sources. Chimeric polypeptides may include, e.g., two, three, four, five, six, seven, or more polypeptides from different sources, such as different genes, different cDNAs, or different animal or other species. Chimeric polypeptides may include, e.g., one or more linkers joining the different subsequences. Thus, the subsequences may be joined directly or they may be joined indirectly, via linkers, or both, within a single chimeric polypeptide. Chimeric polypeptides may include, e.g., additional peptides such as signal sequences and sequences such as 6His and FLAG that aid in protein purification or detection. In addition, chimeric polypeptides may have amino acid or peptide additions to the N- and/or C-termini.

The chimeric polypeptide comprises a Factor VIII portion and a non-Factor VIII portion, wherein this portion comprises an amino acid sequence at least 90% or 95% identical to amino acids 21 to 247 of SEQ ID NO: 4.

As discussed above, exemplary chimeric polypeptides also include Factor VIII fused to one or more albumin polypeptides, albumin binding polypeptides, or albumin-binding small molecules. In one embodiment, the albumin is human albumin. The albumin or albumin binding protein can be fused to either the N-terminal end of FVIII or to the C-terminal end of FVIII or inserted between two amino acids in FVIII. Examples of albumin, e.g., fragments thereof, that may be used in the present invention are known. e.g., U.S. Patent No. 7,592,010; U.S. Patent No. 6,686,179; and Schulte, Thrombosis Res. 124 Suppl. 2:S6-S8 (2009).

The albumin binding polypeptides can compromise, without limitation, bacterial albumin-binding domains, albumin-binding peptides, or albumin-binding antibody fragments that can bind to albumin. Domain 3 from streptococcal protein G, as disclosed by Kraulis et al., FEBS Lett. 378:190-194 (1996) and Linhult et al., Protein Sci. 11:206-213 (2002) is an example of a bacterial albumin-binding domain. Examples of albumin-binding peptides include a series of peptides having the core sequence DICLPRWGCLW (SEQ ID NO: 7). See, e,g., Dennis et al., J. Biol. Chem. 2002, 277: 35035-35043 (2002). Examples of albumin-binding antibody fragments are disclosed in Muller and Kontermann, Curr. Opin. Mol. Ther. 9:319-326 (2007); Rooverset et al., Cancer Immunol. Immunother. 56:303-317 (2007), and Holt et al., Prot. Eng. Design Sci., 21:283-288 (2008).

In certain aspects, a recombinant FVIII polypeptide of the invention comprises at least one attachment site for a non-polypeptide small molecule, variant, or derivative that can bind to albumin thereof. An example of such albumin binding moieties is 2-(3-maleimidopropanamido)-6-(4-(4-iodophenyl)butanamido)hexanoate ("Albu" tag) as disclosed by Trusselet et al., Bioconjugate Chem. 20:2286-2292 (2009).

As discussed above, exemplary chimeric polypeptides also include Factor VIII fused to at least one β subunit of the C-terminal peptide (CTP) of human chorionic gonadotropin or fragment, variant, or derivative thereof. The CTP can be fused to Factor VIII either the N-terminal end of FVIII or to the C-terminal end of FVIII or inserted between two amino acids in FVIII. One or more CTP peptides fused to or inserted into a recombinant protein is known to increase the *in vivo* half-life of that protein. *See, e.g.,* U.S. Patent No. 5,712,122. Exemplary CTP peptides include DPRFQDSSSSKAPPPSLPSPSRLPGPSDTPIL (SEQ ID NO: 8) or SSSSKAPPPSLPSPSRLPGPSDTPILPQ. (SEQ ID NO: 9). *See, e.g.,* U.S. Patent Application Publication No. US 2009/0087411 A1.

As discussed above, exemplary chimeric polypeptides also include Factor VIII fused to at least one PAS sequence or fragment, variant, or derivative thereof. The PAS sequence can be fused to either the N-terminal end of FVIII or to the C-terminal end of FVIII or inserted between two amino acids in FVIII. A PAS peptide or PAS sequence, as used herein, means an amino acid sequence comprising mainly alanine and serine residues or comprising mainly alanine, serine, and proline residues, the amino acid sequence forming random coil conformation under physiological conditions. Accordingly, the PAS sequence is a building block, an amino acid polymer, or a sequence cassette comprising, consisting essentially of, or consisting of alanine, serine, and proline which can be used as a part of the heterologous moiety in the chimeric protein. An amino acid polymer also can form random coil conformation when residues other than alanine, serine, and proline are added as a minor constituent in the PAS sequence. By "minor constituent" is meant that that amino acids other than alanine, serine, and proline can be added in the PAS sequence to a certain degree, *e.g.,* up to about 12%, *i.e.,* about 12 of 100 amino acids of the PAS sequence, up to about 10%, up to about 9%, up to about 8%, about 6%, about 5%, about 4%, about 3%, *i.e.* about 2%, or about 1%, of the amino acids. The amino acids different from alanine, serine and proline cab be selected from the group consisting of Arg, Asn, Asp, Cys, Gln, Glu, Gly, His, Ile, Leu, Lys, Met, Phe, Thr, Trp, Tyr, and Val. Under physiological conditions, a PAS peptide forms a random coil conformation and thereby can mediate an increased *in vivo* and/or *in vitro* stability to a recombinant protein of the invention, and has procoagulant activity.

Non-limiting examples of the PAS peptides include ASPAAPAPASPAAPAPSAPA (SEQ ID NO: 10), AAPASPAPAAPSAPAPAAPS (SEQ ID NO: 11), APSSPSPSAPSSPSPASPSS (SEQ ID NO: 12), APSSPSPSAPSSPSPASPS (SEQ ID NO: 13), SSPSAPSPSSPASPSPSSPA (SEQ ID NO: 14), AASPAAPSAPPAAASPAAPSAPPA (SEQ ID NO: 15), ASAAAPAAASAAASAPSAAA (SEQ ID NO: 16) or any variants, derivatives, fragments, or combinations thereof. Additional examples of PAS sequences are known from, e.g., US Pat. Publ. No. 2010/0292130 A1 and PCT Appl. Publ. No. WO 2008/155134 A1. European issued patent EP2173890.

As discussed above, exemplary chimeric polypeptides also include Factor VIII fused to at least one transferrin peptide or fragment, variant, or derivative thereof. At least one transferrin peptide can be fused to either the N-terminal end of FVIII or to the C-terminal end of FVIII or inserted between two amino acids in FVIII. Any transferrin can be fused to or inserted into a recombinant FVIII protein of the invention. As an example, wild-type human Tf (Tf) is a 679 amino acid protein, of approximately 75 KDa (not accounting for glycosylation), with two main domains, N (about 330 amino acids) and C (about 340 amino acids), which appear to originate from a gene duplication. *See* GenBank accession numbers NM001063, XM002793, M12530, XM039845, XM 039847 and S95936 (www.ncbi.nlm.nih.gov).

Transferrin transports iron through transferrin receptor (TfR)-mediated endocytosis. After the iron is released into an endosomal compartment and Tf-TfR complex is recycled to cell surface, the Tf is released back extracellular space for next cycle of iron transporting. Tf possesses a long half-life that is in excess of 14-17 days (Li et al., Trends Pharmacol. Sci. 23:206-209 (2002)). Transferrin fusion proteins have been studied for half-life extension, targeted deliver for cancer therapies, oral delivery and sustained activation of proinsulin (Brandsma et al., Biotechnol. Adv., 29: 230-238 (2011); Bai et al., Proc. Natl. Acad. Sci. USA 102:7292-7296 (2005); Kim et al., J. Pharmacol. Exp. Ther., 334:682-692 (2010); Wang et al., J. Controlled Release 155:386-392 (2011)).

As discussed above, exemplary chimeric polypeptides also include Factor VIII fused to at least one polyethylene glycol (PEG) moieties.

PEGylated FVIII can refer to a conjugate formed between FVIII and at least one polyethylene glycol (PEG) molecule. PEG is commercially available in a large variety of molecular weights and average molecular weight ranges. Typical examples of PEG average molecular weight ranges include, but are not limited to, about 200, about 300, about 400, about 600, about 1000, about 1300-1600, about 1450, about 2000, about 3000, about 3000-3750, about 3350, about 3000-7000, about 3500-4500, about 5000-7000, about 7000-9000, about 8000, about 10000, about 8500-11500, about 16000-24000, about 35000, about 40000, about 60000, and about 80000 daltons. These average molecular weights are provided merely as examples and are not meant to be limiting in any way.

A recombinant FVIII protein of the invention can be PEGylated to include mono- or poly-(e.g., 2-4) PEG moieties. PEGylation can be carried out by any of the PEGylation reactions known in the art. Methods for preparing a PEGylated protein product will generally include (i) reacting a polypeptide with polyethylene glycol (such as a reactive ester or aldehyde derivative of PEG) under conditions whereby the peptide of the invention becomes attached to one or more PEG groups; and (ii) obtaining the reaction product(s). In general, the optimal reaction conditions for the reactions will be determined case by case based on known parameters and the desired result.

There are a number of PEG attachment methods available to those skilled in the art, for example Malik F et al., Exp. Hematol. 20:1028-35 (1992); Francis, Focus on Growth Factors 3(2):4-10 (1992); European Pat. Pub. Nos. EP0401384, EP0154316, and EP0401384; and International Pat. Appl. Pub. Nos. WO92/16221 and WO95/34326. As a non-limiting example, FVIII variants can contain cysteine substitutions in one or more insertion sites in FVIII, and the cysteines can be further conjugated to PEG polymer. See Mei et al., Blood 116:270-279 (2010) and U.S. Patent No. 7,632,921.

As discussed above, exemplary chimeric polypeptides also include Factor VIII fused to at least one hydroxyethyl starch (HES) polymer. HES is a derivative of naturally occurring amylopectin and is degraded by alpha-amylase in the body. HES exhibits advantageous biological properties and is used as a blood volume replacement agent and in hemodilution therapy in the clinics. See, *e.g.,* Sommermeyer et al., Krankenhauspharmazie 8:271-278 (1987); and Weidler et al., Arzneim.-Forschung/Drug Res. 41: 494-498 (1991).

HES is mainly characterized by the molecular weight distribution and the degree of substitution. HES has a mean molecular weight (weight mean) of from 1 to 300 kD, from 2 to 200kD, from 3 to 100 kD, or from 4 to 70kD. Hydroxyethyl starch can further exhibit a molar degree of substitution of from 0.1 to 3, from 0.1 to 2,from 0.1 to 0.9, or from 0.1 to 0.8, and a ratio between C2:C6 substitution in the range of from 2 to 20 with respect to the hydroxyethyl groups. HES with a mean molecular weight of about 130 kD is Voluven^{®} from Fresenius. Voluven^{®} is an artificial colloid, employed, *e.g.,* for volume replacement used in the therapeutic indication for therapy and prophylaxis of hypovolaemia. There are a number of HES attachment methods available to those skilled in the art, *e.g.,* the same PEG attachment methods described above.

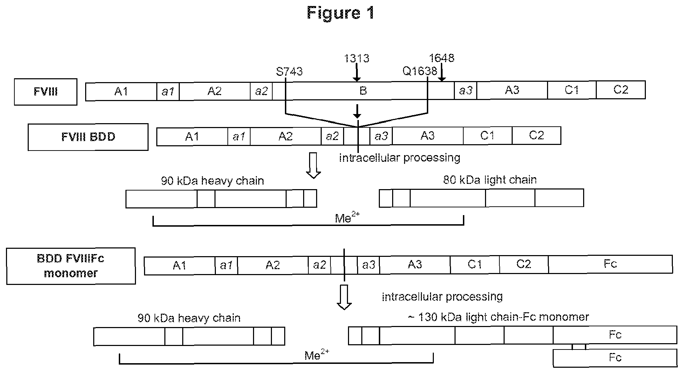

In some embodiments, a chimeric polypeptide comprising a Factor VIII portion has an increased half-life (t1/2) over a polypeptide consisting of the same Factor VIII portion without the non Factor VIII portion. A chimeric Factor VIII polypeptide with an increased t1/2 may be referred to herein as a long-acting Factor VIII. Long-acting chimeric Factor VIII polypeptides include, e.g., Factor VIII fused to Fc (including, e.g., chimeric Factor VIII polypeptides in the form of a hybrid such as a FVIIIFc monomer dimer hybrid; see Example 1, Fig. 1, and Table 2A; and US Patent Nos. 7,404,956 and 7,348,004), Factor VIII fused to XTEN, and Factor VIII fused to albumin.

"Culture," "to culture" and "culturing," as used herein, means to incubate cells under in vitro conditions that allow for cell growth or division or to maintain cells in a living state. "Cultured cells," as used herein, means cells that are propagated in vitro.

"Factor VIII," as used herein, means functional factor VIII polypeptide in its normal role in coagulation, unless otherwise specified. Factor VIII proteins can be the human, porcine, canine, and murine factor VIII proteins. As described in the Background Art section, the full length polypeptide and polynucleotide sequences are known, as are many functional fragments, mutants and modified versions. Examples of human factor VIII sequences are shown as subsequences in SEQ ID NOs:2 or 6 (Table 2). Factor VIII polypeptides include, e.g., full-length factor VIII, full-length factor VIII minus Met at the N-terminus, mature factor VIII (minus the signal sequence), mature factor VIII with an additional Met at the N-terminus, and/or factor VIII with a full or partial deletion of the B domain. Factor VIII variants include B domain deletions, whether partial or full deletions.

A great many functional factor VIII variants are known, as is discussed above and below. In addition, hundreds of nonfunctional mutations in factor VIII have been identified in hemophilia patients, and it has been determined that the effect of these mutations on factor VIII function is due more to where they lie within the 3-dimensional structure of factor VIII than on the nature of the substitution (Cutler et al., Hum. Mutat. 19:274-8 (2002)). In addition, comparisons between factor VIII from humans and other species have identified conserved residues that are likely to be required for function (Cameron et al., Thromb. Haemost. 79:317-22 (1998); US 6,251,632).

The human factor VIII gene was isolated and expressed in mammalian cells (Toole, J. J., et al., Nature 312:342-347 (1984); Gitschier, J., et al., Nature 312:326-330 (1984); Wood, W. I., et al., Nature 312:330-337 (1984); Vehar, G. A., et al., Nature 312:337-342 (1984); WO 87/04187; WO 88/08035; WO 88/03558; U.S. Pat. No. 4,757,006), and the amino acid sequence was deduced from cDNA. Capon et al., U.S. Pat. No. 4,965,199, discloses a recombinant DNA method for producing factor VIII in mammalian host cells and purification of human factor VIII. Human factor VIII expression in CHO (Chinese hamster ovary) cells and BHKC (baby hamster kidney cells) has been reported. Human factor VIII has been modified to delete part or all of the B domain (U.S. Pat. Nos. 4,994,371 and 4,868,112), and replacement of the human factor VIII B domain with the human factor V B domain has been performed (U.S. Pat. No. 5,004,803). The cDNA sequence encoding human factor VIII and predicted amino acid sequence are shown in SEQ ID NOs:1 and 2, respectively, of US Application Publ. No. 2005/0100990.

U.S. Pat. No. 5,859,204, Lollar, J. S., reports functional mutants of factor VIII having reduced antigenicity and reduced immunoreactivity. U.S. Pat. No. 6,376,463, Lollar, J. S., also reports mutants of factor VIII having reduced immunoreactivity. US Application Publ. No. 2005/0100990, Saenko et al., reports functional mutations in the A2 domain of factor VIII.

A number of functional factor VIII molecules, including B-domain deletions, are disclosed in the following patents US 6,316,226 and US 6,346,513, both assigned to Baxter; US 7,041,635 assigned to In2Gen; US 5,789,203, US 6,060,447, US 5,595,886, and US 6,228,620 assigned to Chiron; US 5,972,885 and US 6,048,720 assigned to Biovitrum, US 5,543,502 and US 5,610,278 assigned to Novo Nordisk; US 5,171,844 assigned to Immuno Ag; US 5,112,950 assigned to Transgene S.A.; US 4,868,112 assigned to Genetics Institute.

The porcine factor VIII sequence is published, (Toole, J. J., et al., Proc. Natl. Acad. Sci. USA 83:5939-5942 (1986)), and the complete porcine cDNA sequence obtained from PCR amplification of factor VIII sequences from a pig spleen cDNA library has been reported (Healey, J. F. et al., Blood 88:4209-4214 (1996)). Hybrid human/porcine factor VIII having substitutions of all domains, all subunits, and specific amino acid sequences were disclosed in U.S. Pat. No. 5,364,771 by Lollar and Runge, and in WO 93/20093. More recently, the nucleotide and corresponding amino acid sequences of the A1 and A2 domains of porcine factor VIII and a chimeric factor VIII with porcine A1 and/or A2 domains substituted for the corresponding human domains were reported in WO 94/11503. U.S. Pat. No. 5,859,204, Lollar, J. S., also discloses the porcine cDNA and deduced amino acid sequences. US Pat. No. 6,458,563, assigned to Emory discloses a B-domain deleted porcine Factor VIII.

The single chain Factor VIII (or Factor VIII portion of the chimeric polypeptide) comprises an amino acid sequence at least 90% or 95% identical to amino acids 20 to 1457 of SEQ ID NO:2.

"Equivalent dose," as used herein, means the same dose of Factor VIII activity as expressed in International Units, which is independent of molecular weight of the polypeptide in question. One International Unit (IU) of factor VIII activity corresponds approximately to the quantity of factor VIII in one milliliter of normal human plasma. Several assays are available for measuring Factor VIII activity, including the European Pharmacopoeia chromogenic substrate assay and a one stage clotting assay.

"Fc," as used herein, means functional neonatal Fc receptor (FcRn) binding partners, unless otherwise specified. An FcRn binding partner is any molecule that can be specifically bound by the FcRn receptor with consequent active transport by the FcRn receptor of the FcRn binding partner. Thus, the term Fc includes any variants of IgG Fc that are functional. The region of the Fc portion of IgG that binds to the FcRn receptor has been described based on X-ray crystallography (Burmeister et al. , Nature 372:379 (1994)). The major contact area of the Fc with the FcRn is near the junction of the CH2 and CH3 domains. Fc-FcRn contacts are all within a single Ig heavy chain. The FcRn binding partners include, e.g., whole IgG, the Fc fragment of IgG, and other fragments of IgG that include the complete binding region of FcRn. The major contact sites include amino acid residues 248, 250-257, 272, 285, 288, 290-291, 308-311, and 314 of the CH2 domain and amino acid residues 385-387, 428, and 433-436 of the CH3 domain. References made to amino acid numbering of immunoglobulins or immunoglobulin fragments, or regions, are all based on Kabat et al. 1991, Sequences of Proteins of Immunological Interest, U. S. Department of Public Health, Bethesda; MD. (The FcRn receptor has been isolated from several mammalian species including humans. The sequences of the human FcRn, rat FcRn, and mouse FcRn are known (Story et al., J. Exp. Med. 180: 2377 (1994)). An Fc may comprise the CH2 and CH3 domains of an immunoglobulin with or without the hinge region of the immunoglobulin. Exemplary Fc variants are provided in WO 2004/101740 and WO 2006/074199.

Fc (or Fc portion of a chimeric polypeptide) may contain one or more mutations, and combinations of mutations.

Fc (or Fc portion of a chimeric polypeptide) may contain mutations conferring increased half-life such as M252Y, S254T, T256E, and combinations thereof, as disclosed in Oganesyan et al., Mol. Immunol. 46:1750 (2009); H433K, N434F, and combinations thereof, as disclosed in Vaccaro et al., Nat. Biotechnol. 23:1283 (2005); the mutants disclosed at pages 1-2, paragraph [0012], and Examples 9 and 10 of US 2009/0264627 A1; and the mutants disclosed at page 2, paragraphs [0014] to [0021] of US 20090163699 A1.

Fc (or Fc portion of a chimeric polypeptide) may also include, e.g., the following mutations: The Fc region of IgG can be modified according to well recognized procedures such as site directed mutagenesis and the like to yield modified IgG or Fc fragments or portions thereof that will be bound by FcRn. Such modifications include, e.g., modifications remote from the FcRn contact sites as well as modifications within the contact sites that preserve or even enhance binding to the FcRn. For example the following single amino acid residues in human IgG1 Fc (Fcy1) can be substituted without significant loss of Fc binding affinity for FcRn: P238A, S239A, K246A, K248A, D249A, M252A, T256A, E258A, T260A, D265A, S267A, H268A, E269A, D270A, E272A, L274A, N276A, Y278A, D280A, V282A, E283A, H285A, N286A, T289A, K290A, R292A, E293A, E294A, Q295A, Y296F, N297A, S298A, Y300F, R301A, V303A, V305A, T307A, L309A, Q311A, D312A, N315A, K317A, E318A, K320A, K322A, S324A, K326A, A327Q, P329A, A330Q, A330S, P331A, P331S, E333A, K334A, T335A, S337A, K338A, K340A, Q342A, R344A, E345A, Q347A, R355A, E356A, M358A, T359A, K360A, N361A, Q362A, Y373A, S375A D376A, A378Q, E380A, E382A, S383A, N384A, Q386A, E388A, N389A, N390A, Y391F, K392A, L398A, S400A, D401A, D413A, K414A, R416A, Q418A, Q419A, N421A, V422A, S424A, E430A, N434A, T437A, Q438A, K439A, S440A, S444A, and K447A, where for example P238A represents wildtype proline substituted by alanine at position number 238. In addition to alanine other amino acids may be substituted for the wildtype amino acids at the positions specified above. Mutations may be introduced singly into Fc giving rise to more than one hundred FcRn binding partners distinct from native Fc. Additionally, combinations of two, three, or more of these individual mutations may be introduced together, giving rise to hundreds more FcRn binding partners. Certain of these mutations may confer new functionality upon the FcRn binding partner. For example, one embodiment incorporates N297A, removing a highly conserved N-glycosylation site. The effect of this mutation is to reduce immunogenicity, thereby enhancing circulating half-life of the FcRn binding partner, and to render the FcRn binding partner incapable of binding to FcyRI, FcyRIIA, FcyRIIB, and FcyRIIIA, without compromising affinity for FcRn (Routledge et al. 1995, Transplantation 60:847; Friend et al. 1999, Transplantation 68:1632; Shields et al. 1995, J. Biol. Chem. 276:6591). Additionally, at least three human Fc gamma receptors appear to recognize a binding site on IgG within the lower hinge region, generally amino acids 234-237. Therefore, another example of new functionality and potential decreased immunogenicity may arise from mutations of this region, as for example by replacing amino acids 233-236 of human IgG1 "ELLG" to the corresponding sequence from IgG2 "PVA" (with one amino acid deletion). It has been shown that FcyRI, FcyRII, and FcyRIII which mediate various effector functions will not bind to IgG1 when such mutations have been introduced (Ward and Ghetie, Therapeutic Immunology 2:77 (1995); and Armour et al., Eur. J. Immunol. 29:2613 (1999)). As a further example of new functionality arising from mutations described above affinity for FcRn may be increased beyond that of wild type in some instances. This increased affinity may reflect an increased "on" rate, a decreased "off" rate or both an increased "on" rate and a decreased "off" rate. Mutations believed to impart an increased affinity for FcRn include, e.g., T256A, T307A, E380A, and N434A (Shields et al. , J. Biol. Chem. 276:6591 (2001)).

The Fc (or Fc portion of a chimeric polypeptide) comprises an amin acid sequence at least 90% or 95% identical to amino acids 21 to 247 of SEQ ID NO:4.

"Hybrid" polypeptides and proteins, as used herein, means a combination of a chimeric polypeptide with a second polypeptide. The chimeric polypeptide and the second polypeptide in a hybrid may be associated with each other via protein-protein interactions, such as charge-charge or hydrophobic interactions. The chimeric polypeptide and the second polypeptide in a hybrid may be associated with each other via disulfide or other covalent bond(s). Hybrids are described in WO 2004/101740 and WO 2006/074199. See also U.S. Patent Nos. 7,404,956 and 7,348,004. The second polypeptide may be a second copy of the same chimeric polypeptide or it may be a non-identical chimeric polypeptide. See, e.g., Figure 1, Example 1, and Table 2. In one embodiment, the second polypeptide is a polypeptide comprising an Fc. In another embodiment, the chimeric polypeptide is a chimeric Factor VIII-Fc polypeptide and the second polypeptide consists essentially of Fc, e.g., the hybrid polypeptide of Example 1, which is a rFVIIIFc recombinant fusion protein consisting of a single molecule of recombinant B-domain deleted human FVIII (BDD-rFVIII) fused to the dimeric Fc domain of the human IgG1, with no intervening linker sequence. This hybrid polypeptide is referred to herein as FVIIIFc monomeric Fc fusion protein, FVIIIFc monomer hybrid, monomeric FVIIIIFc hybrid, and FVIIIFc monomer-dimer. See Example 1, Fig. 1, and Table 2A. The Examples provide preclinical and clinical data for this hybrid polypeptide.

The second polypeptide in a hybrid may comprise or consist essentially of a sequence at least 90% or 95% identical to the amino acid sequence shown in Table 2A(ii) without a signal sequence (amino acids 21 to 247 of SEQ ID NO:4) or at least 90% or 95% identical to the amino acid sequence shown in Table 2A(ii) with a signal sequence (amino acids 1 to 247 of SEQ ID NO-:4). The second polypeptide may comprise or consist essentially of a sequence identical to the amino acid sequence shown in Table 2A(ii) without a signal sequence (amino acids 21 to 247 of SEQ ID NO:4) or identical to the amino acid sequence shown in Table 2A(ii) with a signal sequence (amino acids 1 to 247 of SEQ ID NO:4).

Figure 1 is a schematic showing the structure of a B domain deleted factor VIII-Fc chimeric polypeptide, and its association with a second polypeptide that is an Fc polypeptide. To obtain this hybrid, the coding sequence of human recombinant B-domain deleted FVIII was obtained by reverse transcription-polymerase chain reaction (RT-PCR) from human liver poly A RNA (Clontech) using FVIII-specific primers. The FVIII sequence includes the native signal sequence for FVIII. The B-domain deletion was from serine 743 (S743; 2287 bp) to glutamine 1638 (Q1638; 4969 bp) for a total deletion of 2682 bp. Then, the coding sequence for human recombinant Fc was obtained by RT-PCR from a human leukocyte cDNA library (Clontech) using Fc specific primers. Primers were designed such that the B-domain deleted FVIII sequence was fused directly to the N-terminus of the Fc sequence with no intervening linker. The FVIIIFc DNA sequence was cloned into the mammalian dual expression vector pBUDCE4.1 (Invitrogen) under control of the CMV promoter. A second identical Fc sequence including the mouse Igk signal sequence was obtained by RT-PCR and cloned downstream of the second promoter, EF1α, in the expression vector pBUDCE4.1.

The rFVIIIFc expression vector was transfected into human embryonic kidney 293 cells (HEK293H; Invitrogen) using Lipofectamine 2000 transfection reagent (Invitrogen). Stable clonal cell lines were generated by selection with Zeocin (Invitrogen). One clonal cell line, 3C4-22 was used to generate FVIIIFc for characterization in vivo. Recombinant FVIIIFc was produced and purified (McCue et al. 2009) at Biogen Idec (Cambridge, MA). The transfection strategy described above was expected to yield three products, i.e., monomeric rFVIIIFc hybrids, dimeric rFVIIIFc hybrids and dimeric Fc. However, there was essentially no dimeric rFVIIIFc detected in the conditioned medium from these cells. Rather, the conditioned medium contained Fc and monomeric rFVIIIFc. It is possible that the size of dimeric rFVIIIFc was too great and prevented efficient secretion from the cell. This result was beneficial since it rendered the purification of the monomer less complicated than if all three proteins had been present. The material used in these studies had a specific activity of approximately 9000 IU/mg.

The polypeptides as disclosed herein can be administered to a patient who expresses high level of von Willebrand factor (VWF). "Subject" or "patient" as used herein means a human individual. A subject can be a patient who is currently suffering from a bleeding disorder or is expected to be in need of such a treatment. "Subject" can include an adult or a pediatric subject. The pediatric subject can be a pediatric patient under the age of 12. The term "pediatrics" as used herein is the branch of medicine that deals with the care of infants and children and the treatment of their diseases. In one embodiment, the subject is a pediatric patient who has a diagnosis of severe hemophilia A. For example, pediatric subjects can be treated with a long-acting Factor VIII polypeptide as disclosed herein.

VWF is a plasma protein having a multimer structure in which the molecular weight of the various forms varies between approximately 230 kDa for each monomer subunit and up to more than 20 million Da in the multimer forms of greater molecular weight, thus forming the largest known soluble protein. Its plasma concentration is approximately around 5-10 µg/ml (Siedlecki et al., Blood, vol 88: 2939-2950 (1996)) and the plasma form of smaller size is that corresponding to the dimer, with an approximate size of 500 kDa.

VWF has an essential role to play in primary haemostasis, being responsible for the adhesion of platelets to damaged vascular surfaces and therefore formation of the platelet plug on which the mechanisms for formation of the fibrin coagulate develop. It is suggested that the higher molecular weight multimers support platelet adhesion mechanisms to the sub-endothelium with greater efficiency and the clinical efficacy of VWF concentrates has been related to the concentration of these multimers of higher molecular weight (Metzner et al., Haemophilia 4:25-32 (1998).

Therefore, subjects expressing high levels of VWF would require less frequent dosing of FVIII compared to a subject who expresses lower or normal levels of VWF. The average range of VWF in plasma is between about 50 IU/dL and about 200 IU/dL. In one embodiment, the average level of VWF in plasma is about 50 IU/dL. In another embodiment, a VWF level in plasma of at least about 100 IU/dL is considered a high VWF level. In another embodiment, a high level of VWF in plasma is between about 100 IU/dL and about 200 IU/dL. In another embodiment a high level of VWF in plasma is at least about 110 IU/dL, about 120 IU/dL, about 130 IU/dL, about 140 IU/dL, about 150 IU/dL, about 160 IU/dL, about 170 IU/dL, about 180 IU/dL, about 190 IU/dL, or about 200 IU/dL.

Therefore, subjects expressing at least about 100 IU/dL of plasma VWF can be administered a long-acting FVIII polypeptide disclosed herein at a long interval dosing regimen. The long-acting FVIII polypeptide can be administered at a dosing interval of at least about 3 days, or at a dosing interval of at least about once every week, about once every two weeks, about once every 15 days, about once every 20 days, about once every three weeks, about once every 25 days, about once every four weeks, or about once every one month.

In one embodiment, the subjects were previously identified as having high levels of VWF. In certain embodiments, subjects having a blood serotype other than O (*i.e.,* A, B, or AB) require less frequent dosing of long-acting FVIII because the long-acting FVIII has a longer half-life in these subjects. In these subjects, the increased half-life is due to their elevated VWF levels.

Moreover, pharmacokinetic data, defined as the study of the time course of drug absorption, distribution, metabolism, and excretion, can be used as an identifier of subjects eligible for longer or shorter dosing intervals using a long-acting FVIII polypeptide of the invention. Cinical pharmacokinetics is the application of pharmacokinetic principles to the safe and effective therapeutic management of drugs in an individual patient. The primary goals of clinical pharmacokinetics include enhancing efficacy and decreasing toxicity of a patient's drug therapy. The development of strong correlations between drug concentrations and their pharmacologic responses has enabled clinicians to apply pharmacokinetic principles to actual patient situations.

Thus, the half-life of a FVIII-Fc polypeptide can be used to identify patients who express high levels of VWF. The range of half-life of FVIII-Fc is between about 10 and about 40 hours, depending at least in part on the levels of VWF also present. On average however, the half-life of FVIII-Fc is about 18 hours. Generally, FVIII-Fc exhibits an increased half-life of at least about 1.2-fold in patients having high levels of VWF compared to the half-life of FVIII-Fc when administered to individuals having average levels of VWF. In one embodiment, FVIII-Fc exhibits an increased half-life of at least about 1.3, 1.4, 1.5, 1.6, 1.7, 1.8, 1.9, 2.0, 2.1, 2.2, 2.3, 2.4, or 2.5-fold compared to the half-life of FVIII-Fc when administered to individuals having average levels of VWF. In subjects expressing high levels of VWF, the half-life of FVIII-Fc can be between at least about 20 hours and about 40 hours, at least about 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 37 hours, 38 hours, 39 hours, or 40 hours, or the half-life of FVIII-Fc is between about 20 and about 27 hours in subjects having high levels of VWF. An increased half-life of FVIII-Fc compared to average values can be indicative of a subject that is eligible for a longer dosing interval with a long-acting FVIII polypeptide of the invention.

Alternatively, the half-life of a short-acting FVIII polypeptide can be used to identify patients who express high levels of VWF. As used herein, the term "short-acting FVIII" refers to a FVIII polypeptide in which no extenders of half-life have been added. Short-acting FVIII polypeptides may consist of full-length or B domain-deleted FVIII. Examples of short-acting FVIII polypeptides are Advate^{®} and ReFacto^{®}.

Since the half-life of short-acting FVIII also varies depending at least in part on VWF levels, short-acting FVIII polypeptides can also be used to identify patients that are eligible for a longer dosing interval of a long-acting FVIII polypeptide disclosed herein. The short-acting FVIII may exhibit an increased half-life of at least about 1.2-fold in individuals expressing high levels of VWF compared to the half-life of the short-acting FVIII when administered to individuals having average levels of VWF, or an increased half-life of at least about 1.3, 1.4, 1.5, 1.6, 1.7, 1.8, 1.9, 2.0, 2.1, 2.2, 2.3, 2.4, or 2.5-fold in individuals expressing high levels of VWF compared to the half-life of the short-acting FVIII when administered to individuals having average levels of VWF. Thus, individuals that demonstrate an increased half-life of at least about 1.2-fold when they are administered a short-acting FVIII are eligible for a longer dosing interval with a long-acting FVIII polypeptide of the invention.

"Dosing interval," as used herein, means the dose of time that elapses between multiple doses being administered to a subject. The comparison of dosing interval may be carried out in a single subject or in a population of subjects and then the average obtained in the population may be calculated.

The dosing interval when administering a chimeric Factor VIII polypeptide, e.g., a chimeric Factor VIII-Fc polypeptide (a polypeptide comprising a Factor VIII or a hybrid) disclosed herein may be at least about one and one-half times longer than the dosing interval required for an equivalent dose of said Factor VIII without the non-Factor VIII portion, e.g., without the Fc portion (a polypeptide consisting of said Factor VIII). The dosing interval may be at least about one and one-half to six times longer, one and one-half to five times longer, one and one-half to four times longer, one and one-half to three times longer, or one and one-half to two times longer, than the dosing interval required for an equivalent dose of said Factor VIII without the non-Factor VIII portion, e.g., without the Fc portion (a polypeptide consisting of said Factor VIII). The dosing interval may be at least about one and one-half, two, two and one-half, three, three and one-half, four, four and one-half, five, five and one-half or six times longer than the dosing interval required for an equivalent dose of said Factor VIII without the non-Factor VIII portion, e.g., without the Fc portion (a polypeptide consisting of said Factor VIII).. The dosing interval may be about every three, four, five, six, seven, eight, nine, ten, eleven, twelve, thirteen, or fourteen days or longer. The dosing interval may be at least about one and one-half to 5, one and one-half, 2, 3, 4, or 5 days or longer. For on-demand treatment, the dosing interval of said chimeric polypeptide or hybrid is about once every 24-36, 24-48, 24-72, 24-96, 24-120, 24-144, 24-168, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, or 72 hours or longer.

In one embodiment, the effective dose is 25-80 IU/kg (25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 62, 64, 65, 66, 67, 68, 69, 70, 71, 72, 73, 74, 75, 76, 77, 78, 79, or 80 IU/kg) and the dosing interval is once every 3-5, 3-6, 3-7, 3, 4, 5, 6, 7, or 8 or more days, or three times per week, or no more than three times per week. In one embodiment, the effective dose is 80 IU/kg and the dosing interval is once every 3 days. In a further embodiment, the effective dose of 80 IU/kg given at a dosing interval of every 3 days is administered to a pediatric subject. In another embodiment, the effective dose is 65 IU/kg and the dosing interval is once weekly, or once every 6-7 days. The doses can be administered repeatedly as long as they are necessary (e.g., at least 10, 20, 28, 30, 40, 50, 52, or 57 weeks, at least 1, 2, 3, 4, 5, 6, 7, 8, 9, or 10 years).

In certain embodiments, the effective dose for on-demand treatment is 20-50IU/Kg (20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 IU/kg). The on-demand treatment can be one time dosing or repeated dosing. For repeated dosing, the dosing interval can be every 12-24 hours, every 24-36 hours, every 24-48 hours, every 36-48 hours, or every 48-72 hours.

"Long-acting Factor VIII" is a Factor VIII having an increased half-life (also referred to herein as t1/2, t1/2 beta, elimination half-life and HL) over a reference Factor VIII. The increased half-life of a long-acting Factor VIII may be due to fusion to one or more non-Factor VIII polypeptides such as, e.g., Fc, XTEN, albumin, a PAS sequence, transferrin, CTP (28 amino acid C-terminal peptide (CTP) of hCG with its 4 O-glycans), polyethylene glycol (PEG), hydroxyethyl starch (HES), albumin binding polypeptide, albumin-binding small molecules, or two or more combinations thereof. The increased half-life may be due to one or more modification, such as, e.g., pegylation. Exemplary long-acting Factor VIII polypeptides include, e.g., chimeric Factor VIII polypeptides comprising Fc, chimeric Factor VIII polypeptides comprising XTEN and chimeric Factor VIII polypeptides comprising albumin. Additional exemplary long-acting Factor VIII polypeptides include, e.g., pegylated Factor VIII.

The "reference" polypeptide, in the case of a long-acting chimeric Factor VIII polypeptide, is a polypeptide consisting essentially of the Factor VIII portion of the chimeric polypeptide, e.g., the same Factor VIII portion without the Fc portion, without the XTEN portion, or without the albumin portion. Likewise, the reference polypeptide in the case of a modified Factor VIII is the same Factor VIII without the modification, e.g., a Factor VIII without the pegylation.

A long-acting Factor VIII may have one or more of the following properties when administered to a subject:
a mean residence time (MRT) (activity) in said subject of about 14-41.3 hours;
a clearance (CL) (activity) in said subject of about 1.22-5.19 mL/hour/kg or less;
a t1/2beta (activity) in said subject of about 11-26.4 hours;
an incremental recovery (K value) (activity; observed) in said subject of about 1.38-2.88 IU/dL per IU/kg;
a Vss (activity) in said subject of about 37.7-79.4 mL/kg; and
an AUC/dose in said subject of about 19.2-81.7 IU*h/dL per IU/kg.

A long-acting Factor VIII may have one or more of the following properties when administered to a patient population:
a mean incremental recovery (K-Value) (activity; observed) greater that 1.38 IU/dL per IU/kg;
a mean incremental recovery (K-Value) (activity; observed) of at least about 1.5, at least about 1.85, or at least about 2.46 IU/dL per IU/kg;
a mean clearance (CL) (activity) in said patient population of about 2.33 ± 1.08 mL/hour/kg or less;
a mean clearance (CL) (activity) in said patient population of about 1.8-2.69 mL/hour/kg;
a mean clearance (CL) (activity) in said patient population that is about 65% of the clearance of a polypeptide comprising said Factor VIII without modification;
a mean mean residence time (MRT) (activity) in said patient population of at least about 26.3 ± 8.33 hours;
a mean MRT (activity) in said patient population of about 25.9 - 26.5 hours;
a mean MRT (activity) in said patent population that is about 1.5 fold longer than the mean MRT of a polypeptide comprising said Factor VIII without modification;
a mean t1/2beta (activity) in said patient population of about 18.3 ± 5.79 hours;
a mean t1/2beta (activity)in said patient population that is about 18 - 18.4 hours;
a mean t1/2beta (activity) in said patient population that is about 1.5 fold longer than the mean t1/2beta of a polypeptide comprising said Factor VIII without modification;
a mean incremental recovery (K value) (activity; observed) in said patient population of about 2.01 ± 0.44 IU/dL per IU/kg;
a mean incremental recovery (K value) (activity; observed) in said patient population of about 1.85 - 2.46 IU/dL per IU/kg;
a mean incremental recovery (K value) (activity; observed) in said patient population that is about 90 % of the mean incremental recovery of a polypeptide comprising said Factor VIII without modification;
a mean Vss (activity) in said patient population of about 55.1 ± 12.3 mL/kg;
a mean Vss (activity) in said patient population of about 45.3 - 56.1 mL/kg;
a mean AUC/dose (activity) in said patient population of about 49.9 ± 18.2 IU*h/dL per IU/kg;
a mean AUC/dose (activity) in said patient population of about 44.8 - 57.6 IU*h/dL per IU/kg.

The long-acting Factor VIII may have one or more of the following properties when administered to a patient population:
a C_{max_}OBS in said subject administered with the chimeric polypeptide is comparable to the C_{max_}OBS in a subject administered with the same amount of a polypeptide consisting of the full-length, mature Factor VIII when measured by a one stage (aPTT) assay or a two stage (chromogenic) assay;
a C_{max_}OBS in said subject of about 60.5 IU/dL, about 60.5 ± 1 IU/dL, about 60.5 ± 2 IU/dL, about 60.5 ± 3 IU/dL, about 60.5 ± 4 IU/dL, about 60.5 ± 5 IU/dL, about 60.5 ± 6 IU/dL, about 60.5 ± 7 IU/dL, about 60.5 ± 8 IU/dL, about 60.5 ± 9 IU/dL, or about 60.5 ± 10 IU/dL as measured by a one stage (aPTT) assay when about 25 IU/kg of the chimeric polypeptide is administered;
a C_{max_}OBS in said subject of about 53.1 - 69 IU/dL as measured by a one stage (aPTT) assay when about 25 IU/kg of the chimeric polypeptide is administered;
a C_{max_}OBS in said subject of about 119 IU/dL, about 119 ± 1 IU/dL, about 119 ± 2 IU/dL, about 119 ± 3 IU/dL, about 119 ± 4 IU/dL, about 119 ± 5 IU/dL, about 119 ± 6 IU/dL, about 119 ± 7 IU/dL, about 119 ± 8 IU/dL, about 119 ± 9 IU/dL, about 119 ± 10 IU/dL, about 119 ± 11 IU/dL, about 119 ± 12 IU/dL, about 119 ± 13 IU/dL, about 119 ± 14 IU/dL, about 119 ± 15 IU/dL, about 119 ± 16 IU/dL, about 119 ± 17 IU/dL, or about 119 ± 18 IU/dL, as measured by a one stage (aPTT) assay when about 65 IU/kg of the chimeric polypeptide is administered;
a C_{max_}OBS in said subject of about 103 - 136 IU/dL as measured by a one stage (aPTT) assay when about 65 IU/kg of the chimeric polypeptide is administered;
a C_{max_}OBS in said subject of about 76.5 IU/dL, about 76.5 ± 1 IU/dL, about 76.5 ± 2 IU/dL, about 76.5 ± 3 IU/dL, about 76.5 ± 4 IU/dL, about 76.5 ± 5 IU/dL, about 76.5 ± 6 IU/dL, about 76.5 ± 7 IU/dL, about 76.5 ± 8 IU/dL, about 76.5 ± 9 IU/dL, about 76.5 ± 10 IU/dL, about 76.5 ± 11 IU/dL, about 76.5 ± 12 IU/dL, about 76.5 ± 13 IU/dL, about 76.5 ± 14 IU/dL, or about 76.5 ± 15 IU/dL, as measured by a two stage (chromogenic) assay when about 25 IU/kg of the chimeric polypeptide is administered;
a C_{max_}OBS in said subject of about 64.9 - 90.1 IU/dL as measured by a two stage (chromogenic) assay when about 25 IU/kg of the chimeric polypeptide is administered;
a C_{max_}OBS in said subject of about 182 IU/dL, about 182 ± 2 IU/dL, about 182 ± 4 IU/dL, about 182 ± 6 IU/dL, about 182 ± 8 IU/dL, about 182 ± 10 IU/dL, about 182 ± 12 IU/dL, about 182 ± 14 IU/dL, about 182 ± 16 IU/dL, about 182 ± 18 IU/dL, or about 182 ± 20 IU/dL as measured by a two stage (chromogenic) assay when about 65 IU/kg of the chimeric polypeptide is administered; or
a C_{max_}OBS in said subject of about 146 - 227 IU/dL, about 146 ± 5 IU/dL, about 146 ± 10 IU/dL, about 227 ± 5 IU/dL, or about 146 ± 10 IU/dL as measured by a two stage (chromogenic) assay when about 65 IU/kg of the chimeric polypeptide is administered.

The long-acting Factor VIII may have one or more of the following properties when administered to a patient population:
a t1/2beta (activity) in said subject that is at least 1.48, 1.49, 1.50, 1.51, 1.52, 1.53, 1.54, 1.55, 1.56, 1.57, 1.58, 1.59, 1.60, 1.61, 1.62, 1.63, 1.64, 1.65, 1.66, 1.67, 1.68, 1.69, 1.70, 1.71, 1.72, 1.73, 1.74, 1.75, 1.76, 1.77, 1.78, 1.79, 1.80, 1.81, 1.82, 1.83, 1.84, 1.85, 1.86, 1.87, 1.88, 1.89, or 1.90 times higher than the t1/2beta (activity) in a subject administered with the same amount of a polypeptide consisting of the full-length, mature Factor VIII when measured by a one stage (aPTT) assay or a two stage (chromogenic) assay;
a t1/2beta (activity) in said subject of about 18.8 hours, 18.8 ± 1 hours, 18.8 ± 1 hours, 18.8 ± 2 hours, 18.8 ± 3 hours, 18.8 ± 4 hours, 18.8 ± 5 hours, 18.8 ± 6 hours, 18.8 ± 7 hours, 18.8 ± 8 hours, 18.8 ± 9 hours, 18.8 ± 10 hours, or 18.8 ± 11 hours as measured by a one stage (aPTT) assay;
a t1/2beta (activity) in said subject of about 14.3 - 24.5 hours as measured by a one stage (aPTT) assay;
a t1/2beta (activity) in said subject of about 16.7 hours, 16.7 ± 1 hours, 16.7 ± 2 hours, 16.7 ± 3 hours, 16.7 ± 4 hours, 16.7 ± 5 hours, 16.7 ± 6 hours, 16.7 ± 7 hours, 16.7 ± 8 hours, 16.7 ± 9 hours, 16.7 ± 10 hours, or 16.7 ± 11 hours as measured by a two stage (chromogenic) assay;
a t1/2beta (activity) in said subject of about 13.8 - 20.1 hours as measured by a two stage (chromogenic) assay;
a t1/2beta (activity) in said subject of about 19.8 hours, 19.8 ± 1 hours, 19.8 ± 2 hours, 19.8 ± 3 hours, 19.8 ± 4 hours, 19.8 ± 5 hours, 19.8 ± 6 hours, 19.8 ± 7 hours, 19.8 ± 8 hours, 19.8 ± 9 hours, 19.8 ± 10 hours, or 19.8 ± 11 hours as measured by a two stage (chromogenic) assay; or
a t1/2beta (activity) in said subject of about 14.3 - 27.5 hours as measured by a two stage (chromogenic) assay.

In certain embodiments, the long-acting Factor VIII has one or more of the following properties when administered to a patient population:
a clearance (CL) (activity) in said subject is 0.51, 0.52, 0.53, 0.54, 0.55, 0.56, 0.57, 0.58, 0.59, 0.60, 0.61, 0.62, 0.63, 0.64, 0.65, 0.66, 0.67, 0.68, 0.69, or 0.70 times lower than the clearance in a subject administered with the same amount of a polypeptide consisting of the full-length, mature Factor VIII when measured by a one stage (aPTT) assay or a two stage (chromogenic) assay;
a clearance (CL) (activity) in said subject of about 1.68 mL/hour/kg, 1.68 ± 0.1 mL/hour/kg, 1.68 ± 0.2 mL/hour/kg, 1.68 ± 0.3 mL/hour/kg, 1.68 ± 0.4 mL/hour/kg, 1.68 ± 0.5 mL/hour/kg, 1.68 ± 0.6 mL/hour/kg, or 1.68 ± 0.7 mL/hour/kg, as measured by a one stage (aPTT) assay when about 25 IU/kg of the chimeric polypeptide is administered;
a clearance (CL) (activity) in said subject of about 1.31 - 2.15 mL/hour/kg as measured by a one stage (aPTT) assay when about 25 IU/kg of the chimeric polypeptide is administered;
a clearance (CL) (activity) in said subject of about 2.32 mL/hour/kg, 2.32 ± 0.1 mL/hour/kg, 2.32 ± 0.2 mL/hour/kg, 2.32 ± 0.3 mL/hour/kg, 2.32 ± 0.4 mL/hour/kg, 2.32 ± 0.5 mL/hour/kg, 2.32 ± 0.6 mL/hour/kg, or 2.32 ± 0.7 mL/hour/kg as measured by a one stage (aPTT) assay when about 65 IU/kg of the chimeric polypeptide is administered;
a clearance (CL) (activity) in said subject of about 1.64 - 3.29 mL/hour/kg as measured by oa ne stage (aPTT) assay when about 65 IU/kg of the chimeric polypeptide is administered;
a clearance (CL) (activity) in said subject of about 1.49 mL/hour/kg, 1.49 ± 0.1 mL/hour/kg, 1.49 ± 0.2 mL/hour/kg, 1.49 ± 0.3 mL/hour/kg, 1.49 ± 0.4 mL/hour/kg, 1.49 ± 0.5 mL/hour/kg, 1.49 ± 0.6 mL/hour/kg, or 1.49 ± 0.7 mL/hour/kg as measured by a two stage (chromogenic) assay when about 25 IU/kg of the chimeric polypeptide is administered;
a clearance (CL) (activity) in said subject of about 1.16 - 1.92 mL/hour/kg as measured by a two stage (chromogenic) assay when about 25 IU/kg of the chimeric polypeptide is administered;
a clearance (CL) (activity) in said subject of about 1.52 mL/hour/kg, 1.52 ± 0.1 mL/hour/kg, 1.52 ± 0.2 mL/hour/kg, 1.52 ± 0.3 mL/hour/kg, 1.52 ± 0.4 mL/hour/kg, 1.52 ± 0.5 mL/hour/kg, 1.52 ± 0.6 mL/hour/kg, or 1.52 ± 0.7 mL/hour/kg as measured by a two stage (chromogenic) assay when about 65 IU/kg of the chimeric polypeptide is administered; or
a clearance (CL) (activity) in said subject of about 1.05-2.20 mL/hour/kg as measured by a two stage (chromogenic) assay when about 65 IU/kg of the chimeric polypeptide is administered.

The long-acting Factor VIII may have one or more of the following properties when administered to a patient population:
a MRT in said subject is at least 1.46, 1.47, 1.48, 1.49, 1.50, 1.51, 1.52, 1.53, 1.54, 1.55, 1.56, 1.57, 1.58, 1.59, 1.60, 1.61, 1.62, 1.63, 1.64, 1.65, 1.66, 1.67, 1.68, 1.69, 1.70, 1.71, 1.72, 1.73, 1.74, 1.75, 1.76, 1.77, 1.78, 1.79, 1.80, 1.81, 1.82, 1.83, 1.84, 1.85, 1.86, 1.87, 1.88, 1.89, 1.90, 1.91, 1.92, or 1.93 times higher than the MRT in a subject administered with the same amount of a polypeptide consisting of the full-length, mature Factor VIII when meansured by a one stage (aPTT) assay or a two stage (chromogenic) assay;
a MRT (activity) in said subject of about 27 hours, 27 ± 1 hours, 27 ± 2 hours, 27 ± 3 hours, 27 ± 4 hours, 27 ± 5 hours, 27 ± 6 hours, 27 ± 7 hours, 27 ± 8 hours, 27 ± 9 hours, or 27 ± 10 hours as meansured by a one stage (aPTT) assay;
a MRT (activity) in said subject of about 20.6 - 35.3 hours as meansured by a one stage (aPTT) assay;
a MRT (activity) in said subject of about 23.9 - 28.5 hours as measured by a two stage (chromogenic) assay;
a **MRT (activity)** in said subject of about 19.8 - 28.9 hours as measured by a two stage (chromogenic) assay; or
a MRT (activity) in said subject of about 20.5 - 39.6 hours as measured by a two stage (chromogenic) assay.

The long-acting Factor VIII may have one or more of the following properties when administered to a patient population:
an incremental recovery in said subject that is comparable to the Incremental Recovery in a subject administered with the same amount of a polypeptide consisting of the full-length, mature Factor VIII when measured by a one stage (aPTT) assay or a two stage (chromogenic) assay;
an incremental recovery in said subject of about 2.44 IU/dL per IU/kg, 2.44 ± 0.1 IU/dL per IU/kg, 2.44 ± 0.2 IU/dL per IU/kg, 2.44 ± 0.3 IU/dL per IU/kg, 2.44 ± 0.4 IU/dL per IU/kg, 2.44 ± 0.5 IU/dL per IU/kg, 2.44 ± 0.6 IU/dL per IU/kg, 2.44 ± 0.7 IU/dL per IU/kg, 2.44 ± 0.8 IU/dL per IU/kg, 2.44 ± 0.9 IU/dL per IU/kg, 2.44 ± 1.0 IU/dL per IU/kg, 2.44 ± 1.1 IU/dL per IU/kg, or 2.44 ± 1.2 IU/dL per IU/kg as measured by a one stage (aPTT) assay when about 25 IU/kg of the chimeric polypeptide is administered;
an incremental recovery in said subject of about 2.12 - 2.81 IU/dL per IU/kg as measured by a one stage (aPTT) assay when about 25 IU/kg of the chimeric polypeptide is administered;
an incremental recovery in said subject of about 1.83 IU/dL per IU/kg, 1.83 ± 0.1 IU/dL per IU/kg, 1.83 ± 0.2 IU/dL per IU/kg, 1.83 ± 0.3 IU/dL per IU/kg, 1.83 ± 0.4 IU/dL per IU/kg, 1.83 ± 0.5 IU/dL per IU/kg, 1.83 ± 0.6 IU/dL per IU/kg, 1.83 ± 0.7 IU/dL per IU/kg, 1.83 ± 0.8 IU/dL per IU/kg, 1.83 ± 0.9 IU/dL per IU/kg, 1.83 ± 1.0 IU/dL per IU/kg, or 1.83 ± 1.1 IU/dL per IU/kg as measured by a one stage (aPTT) assay when about 65 IU/kg of the chimeric polypeptide is administered;
an incremental recovery in said subject of about 1.59 - 2.10 IU/dL per IU/kg as measured by a one stage (aPTT) assay when about 65 IU/kg of the chimeric polypeptide is administered;
an incremental recovery in said subject of about 3.09 IU/dL per IU/kg, 3.09 ± 0.1 IU/dL per IU/kg, 3.09 ± 0.2 IU/dL per IU/kg, 3.09 ± 0.3 IU/dL per IU/kg, 3.09 ± 0.4 IU/dL per IU/kg, 3.09 ± 0.5 IU/dL per IU/kg, 3.09 ± 0.6 IU/dL per IU/kg, 3.09 ± 0.7 IU/dL per IU/kg, 3.09 ± 0.8 IU/dL per IU/kg, 3.09 ± 0.9 IU/dL per IU/kg, 3.09 ± 1.0 IU/dL per IU/kg, 3.09 ± 1.1 IU/dL per IU/kg, 3.09 ± 1.2 IU/dL per IU/kg, or 3.09 ± 1.3 IU/dL per IU/kg, as measured by a two stage (chromogenic) assay when about 25 IU/kg of the chimeric polypeptide is administered;
an incremental recovery in said subject of about 2.80 IU/dL per IU/kg, 2.80 ± 0.1 IU/dL per IU/kg, 2.80 ± 0.2 IU/dL per IU/kg, 2.80 ± 0.3 IU/dL per IU/kg, 2.80 ± 0.4 IU/dL per IU/kg, 2.80 ± 0.5 IU/dL per IU/kg, 2.80 ± 0.6 IU/dL per IU/kg, 2.80 ± 0.7 IU/dL per IU/kg, 2.80 ± 0.8 IU/dL per IU/kg, 2.80 ± 0.9 IU/dL per IU/kg, 2.80 ± 1.0 IU/dL per IU/kg, 2.80 ± 1.1 IU/dL per IU/kg, or 2.80 ± 1.2 IU/dL per IU/kg, as measured by a two stage (chromogenic) assay when about 65 IU/kg of the chimeric polypeptide is administered;
an incremental recovery in said subject of about 2.61-3.66 IU/dL per IU/kg as measured by a two stage (chromogenic) assay when about 25 IU/kg of the chimeric polypeptide is administered; or
an incremental recovery in said subject of about 2.24-3.50 IU/dL per IU/kg as measured by a two stage (chromogenic) assay when about 65 IU/kg of the chimeric polypeptide is administered.

The long-acting Factor VIII may have one or more of the following properties when administered to a patient population:
a Vss (activity) in said subject that is comparable to the Vss (activity) in a subject administered with the same amount of a polypeptide consisting of the full-length, mature Factor VIII when measured by a one stage (aPTT) assay or a two stage (chromogenic) assay;
a Vss (activity) in said subject of about 45.5 mL/kg, 45.5 ± 1 mL/kg, 45.5 ± 2 mL/kg, 45.5 ± 3 mL/kg, 45.5 ± 4 mL/kg, 45.5 ± 5 mL/kg, 45.5 ± 6 mL/kg, 45.5 ± 7 mL/kg, 45.5 ± 8 mL/kg, 45.5 ± 9 mL/kg, 45.5 ± 10 mL/kg, or 45.5 ± 11 mL/kg, as measured by a one stage (aPTT) assay when about 25 IU/kg of the chimeric polypeptide is administered;
a Vss (activity) in said subject of about 39.3 - 52.5 mL/kg as measured by a one stage (aPTT) assay when about 25 IU/kg of the chimeric polypeptide is administered;
a Vss (activity) in said subject of about 62.8 mL/kg, 62.8 ± 1 mL/kg, 62.8 ± 2 mL/kg, 62.8 ± 3 mL/kg, 62.8 ± 4 mL/kg, 62.8 ± 5 mL/kg, 62.8 ± 6 mL/kg, 62.8 ± 7 mL/kg, 62.8 ± 8 mL/kg, 62.8 ± 9 mL/kg, 62.8 ± 10 mL/kg, 62.8 ± 11 mL/kg, 62.8 ± 12 mL/kg, 62.8 ± 13 mL/kg, 62.8 ± 14 mL/kg, 62.8 ± 15 mL/kg, or 62.8 ± 16 mL/kg as measured by a one stage (aPTT) assay when about 65 IU/kg of the chimeric polypeptide is administered;
a Vss (activity) in said subject of about 55.2 - 71.5 mL/kg as measured by a one stage (aPTT) assay when about 65 IU/kg of the chimeric polypeptide is administered;
a Vss (activity) in said subject of about 35.9 mL/kg, 35.9 ± 1 mL/kg, 35.9 ± 2 mL/kg, 35.9 ± 3 mL/kg, 35.9 ± 4 mL/kg, 35.9 ± 5 mL/kg, 35.9 ± 6 mL/kg, 35.9 ± 7 mL/kg, 35.9 ± 8 mL/kg, 35.9 ± 9 mL/kg, 35.9 ± 10 mL/kg, 35.9 ± 11 mL/kg, 35.9 ± 12 mL/kg, or 35.9 ± 13 mL/kg, as measured by a two stage (chromogenic) assay when about 25 IU/kg of the chimeric polypeptide is administered;
a Vss (activity) in said subject of about 30.4-42.3 mL/kg as measured by a two stage (chromogenic) assay when about 25 IU/kg of the chimeric polypeptide is administered;
a Vss (activity) in said subject of about 43.4 mL/kg, 43.4 ± 1 mL/kg, 43.4 ± 2 mL/kg, 43.4 ± 3 mL/kg, 43.4 ± 4 mL/kg, 43.4 ± 5 mL/kg, 43.4 ± 6 mL/kg, 43.4 ± 7 mL/kg, 43.4 ± 8 mL/kg, 43.4 ± 9 mL/kg, 43.4 ± 10 mL/kg, 43.4 ± 11 mL/kg, 43.4 ± 12 mL/kg, 43.4 ± 13 mL/kg, 43.4 ± 14 mL/kg, 43.4 ± 15 mL/kg, or 43.4 ± 16 mL/kg, as measured by a two stage (chromogenic) assay when about 65 IU/kg of the chimeric polypeptide is administered; or
a Vss (activity) in said subject of about 38.2-49.2 mL/kg as measured by a two stage (chromogenic) assay when about 65 IU/kg of the chimeric polypeptide is administered.

The long-acting Factor VIII may have one or more of the following properties when administered to a patient population:
an AUC_{INF} in said subject that is at least 1.45 1.46, 1.47, 1.48, 1.49, 1.50, 1.51, 1.52, 1.53, 1.54, 1.55, 1.56, 1.57, 1.58, 1.59, 1.60, 1.61, 1.62, 1.63, 1.64, 1.65, 1.66, 1.67, 1.68, 1.69, 1.70, 1.71, 1.72, 1.73, 1.74, 1.75, 1.76, 1.77, 1.78, 1.79, 1.80, 1.81, 1.82, 1.83, 1.84, 1.85, 1.86, 1.87, 1.88, 1.89, 1.90 times higher than the AUC_{INF} in a subject administered with the same amount of a polypeptide consisting of the full-length, mature Factor VIII when meansured by a one stage (aPTT) assay or a two stage (chromogenic) assay;
an AUC_{INF} in said subject of about 1440 ± 316 hr*IU/dL per IU/kg as measured by a one stage (aPTT) assay when about 25 IU/kg of the chimeric polypeptide is administered;
an AUC_{INF} in said subject of about 1160 - 1880 hr*IU/dL per IU/kg as measured by a one stage (aPTT) assay when about 25 IU/kg of the chimeric polypeptide is administered;
an AUC_{INF} in said subject of about 1480 hr*IU/dL per IU/kg, 1480 ± 100 hr*IU/dL per IU/kg, 1480 ± 200 hr*IU/dL per IU/kg, 1480 ± 300 hr*IU/dL per IU/kg, 1480 ± 400 hr*IU/dL per IU/kg, 1480 ± 500 hr*IU/dL per IU/kg, 1480 ± 600 hr*IU/dL per IU/kg, 1480 ± 700 hr*IU/dL per IU/kg, 1480 ± 800 hr*IU/dL per IU/kg, 1480 ± 900 hr*IU/dL per IU/kg, or 1480 ± 1000 hr*IU/dL per IU/kg, as measured by a one stage (aPTT) assay when about 25 IU/kg of the chimeric polypeptide is administered;
an AUC_{INF} in said subject of about 2910 ± 1320 hr*IU/dL per IU/kg as measured by a one stage (aPTT) assay when about 65 IU/kg of the chimeric polypeptide is administered;
an AUC_{INF} in said subject of about 1980 - 3970 hr*IU/dL per IU/kg as measured by a one stage (aPTT) assay when about 65 IU/kg of the chimeric polypeptide is administered;
an AUC_{INF} in said subject of about 2800 hr*IU/dL per IU/kg, 2800 ± 100 hr*IU/dL per IU/kg, 2800 ± 200 hr*IU/dL per IU/kg, 2800 ± 300 hr*IU/dL per IU/kg, 2800 ± 400 hr*IU/dL per IU/kg, 2800 ± 500 hr*IU/dL per IU/kg, 2800 ± 600 hr*IU/dL per IU/kg, 2800 ± 700 hr*IU/dL per IU/kg, 2800 ± 800 hr*IU/dL per IU/kg, 2800 ± 900 hr*IU/dL per IU/kg, or 2800 ± 1000 hr*IU/dL per IU/kg as measured by a one stage (aPTT) assay when about 65 IU/kg of the chimeric polypeptide is administered;
an AUC_{INF} in said subject of about 1660 hr*IU/dL per IU/kg, 1660 ± 100 hr*IU/dL per IU/kg, 1660 ± 200 hr*IU/dL per IU/kg, 1660 ± 300 hr*IU/dL per IU/kg, 1660 ± 400 hr*IU/dL per IU/kg, 1660 ± 500 hr*IU/dL per IU/kg, 1660 ± 600 hr*IU/dL per IU/kg, 1660 ± 700 hr*IU/dL per IU/kg, 1660 ± 800 hr*IU/dL per IU/kg, 1660 ± 900 hr*IU/dL per IU/kg, or 1660 ± 1000 hr*IU/dL per IU/kg as measured by a two stage (chromogenic) assay when about 25 IU/kg of the chimeric polypeptide is administered;
an AUC_{INF} in said subject of about 1300 - 2120 hr*IU/dL per IU/kg as measured by a two stage (chromogenic) assay when about 25 IU/kg of the chimeric polypeptide is administered;
an AUC_{INF} in said subject of about 4280 hr*IU/dL per IU/kg, 4280 ± 100 hr*IU/dL per IU/kg, 4280 ± 200 hr*IU/dL per IU/kg, 4280 ± 300 hr*IU/dL per IU/kg, 4280 ± 400 hr*IU/dL per IU/kg, 4280 ± 500 hr*IU/dL per IU/kg, 4280 ± 600 hr*IU/dL per IU/kg, 4280 ± 700 hr*IU/dL per IU/kg, 4280 ± 800 hr*IU/dL per IU/kg, 4280 ± 900 hr*IU/dL per IU/kg, 4280 ± 1000 hr*IU/dL per IU/kg, 4280 ± 1100 hr*IU/dL per IU/kg, 4280 ± 1200 hr*IU/dL per IU/kg, 4280 ± 1300 hr*IU/dL per IU/kg, 4280 ± 1400 hr*IU/dL per IU/kg, 4280 ± 1500 hr*IU/dL per IU/kg, or 4280 ± 1600 hr*IU/dL per IU/kg as measured by a two stage (chromogenic) assay when about 65 IU/kg of the chimeric polypeptide is administered; or
an AUC_{INF} in said subject of about 2960 - 6190 hr*IU/dL per IU/kg as measured by a two stage (chromogenic) assay when about 65 IU/kg of the chimeric polypeptide is administered.

"On-demand treatment," as used herein, means treatment that is intended to take place over a short course of time and is in response to an existing condition, such as a bleeding episode, or a perceived need such as planned surgery. Conditions that may require on-demand treatment include, e.g., a bleeding episode, hemarthrosis, muscle bleed, oral bleed, hemorrhage, hemorrhage into muscles, oral hemorrhage, trauma, trauma capitis, gastrointestinal bleeding, intracranial hemorrhage, intra-abdominal hemorrhage, intrathoracic hemorrhage, bone fracture, central nervous system bleeding, bleeding in the retropharyngeal space, bleeding in the retroperitoneal space, or bleeding in the illiopsoas sheath. The subject may be in need of surgical prophylaxis, peri-operative management, or treatment for surgery. Such surgeries include, e.g., minor surgery, major surgery, tooth extraction, tonsillectomy, inguinal herniotomy, synovectomy, total knee replacement, craniotomy, osteosynthesis, trauma surgery, intracranial surgery, intra-abdominal surgery, intrathoracic surgery, or joint replacement surgery.

**In** one embodiment, on-demand treatment resolves greater than 80% (greater than 80%, greater than 81%, greater than 82%, greater than 83%, greater than 84%, greater than 85%, greater than 86%, greater than 87%, greater than 88%, greater than 89%, greater than 90%, greater than 91%, greater than 92%, greater than 93%, greater than 94%, greater than 95%, greater than 96%, greater than 97%, greater than 98%, greater than 99%, or 100%) or 80-100%, 80-90%, 85-90%, 90-100%, 90-95%, or 95-100% of bleeds (e.g., spontaneous bleeds) in a single dose. In another embodiment, greater than 80% (greater than 81%, greater than 82%, greater than 83%, greater than 84%, greater than 85%, greater than 86%, greater than 87%, greater than 88%, greater than 89%, greater than 90%, greater than 91%, greater than 92%, greater than 93%, greater than 94%, greater than 95%, greater than 96%, greater than 97%, greater than 98%, or 100%) or 80-100%, 80-90%, 85-90%, 90-100%, 90-95%, or 95-100% of bleeding episodes are rated excellent or good by physicians after on-demand treatment. In other embodiments, greater than 5%, (greater than 6%, greater than 7%, greater than 8%, greater than 9%, greater than 10%, greater than 11%, greater than 12%, greater than 13%, greater than 14%, greater than 15%, greater than 16%, greater than 17%, greater than 18%, greater than 19%, greater than 20%), or 5-20%, 5-15%, 5-10%, 10-20%, or 10-15% of bleeding episodes are rated as fair by physicians after on-demand treatment.

"Polypeptide," "peptide" and "protein" are used interchangeably and refer to a polymeric compound comprised of covalently linked amino acid residues.

"Polynucleotide" and "nucleic acid" are used interchangeably and refer to a polymeric compound comprised of covalently linked nucleotide residues. Polynucleotides may be DNA, cDNA, RNA, single stranded, or double stranded, vectors, plasmids, phage, or viruses. Polynucleotides include, e.g., those in Table 1, which encode the polypeptides of Table 2 (see Table 1). Polynucleotides also include, e.g., fragments of the polynucleotides of Table 1, e.g., those that encode fragments of the polypeptides of Table 2, such as the Factor VIII, Fc, signal sequence, 6His and other fragments of the polypeptides of Table 2.

"Prophylactic treatment," as used herein, means administering a Factor VIII polypeptide in multiple doses to a subject over a course of time to increase the level of Factor VIII activity in a subject's plasma. The increased level can be sufficient to decrease the incidence of spontaneous bleeding or to prevent bleeding, e.g., in the event of an unforeseen injury. During prophylactic treatment, the plasma protein level in the subject may not fall below the baseline level for that subject, or below the level of Factor VIII that characterizes severe hemophilia (<1 IU/dl [1%]).

In one embodiment, the prophylaxis regimen is "tailored" to the individual patient, for example, by determining PK data for each patient and administering Factor VIII of the invention at a dosing interval that maintains a trough level of 1-3% FVIII activity. Adjustments may be made when a subject experiences unacceptable bleeding episodes defined as ≥2 spontaneous bleeding episodes over a rolling two-month period. In this case, adjustment will target trough levels of 3-5%. In another embodiment, prophylactic treatment results in prevention and control of bleeding, sustained control of bleeding, sustained protection from bleeding, and/or sustained benefit. Prophylaxis, e.g., sustained protection can be demonstrated by an increased AUC to last measured time point (AUC-LAST) and reduced clearance, resulting in increased terminal t1/2 compared to short acting FVIII. Prophylaxis can be demonstrated by better Cmax, better Tmax, and/or greater mean residence time versus short-acting FVIII. In some embodiments, prophylaxis results in no spontaneous bleeding episodes within about 24, 36, 48, 72, or 96 hours (e.g., 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 96, 87, 88, 89, 90, 91, 92, 93, 94, 95, or 96 hours), after injection (e.g., the last injection). In certain embodiments, prophylaxis results in greater than 30% (e.g., greater than 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 96, 87, 88, 89, or 90%, for example, greater than 50%), mean reduction in annualized bleeding episodes with once weekly dosing (e.g., at 65 IU/kg). "Therapeutic dose," as used herein, means a dose that achieves a therapeutic goal, as described herein. The calculation of the required dosage of factor VIII is based upon the empirical finding that, on average, 1 IU of factor VIII per kg body weight raises the plasma factor VIII activity by approximately 2 IU/dL. The required dosage is determined using the following formula: 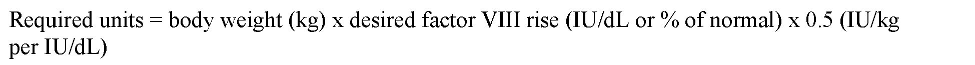 Required units = body weight (kg) x desired factor VIII rise (IU/dL or % of normal) x 0.5 (IU/kg per IU/dL)

The therapeutic doses that may be used in the methods of the invention are about 10-100 IU/kg, more specifically, 10-20, 20-30, 30-40, 40-50, 50-60, 60-70, 70-80, 80-90, or 90-100 IU/kg, and more specifically, 10, 15, 20, 25, 30, 35, 40, 45, 50, 55, 60, 65, 70, 75, 80, 85, 90, 95, or 100 IU/kg.

Additional therapeutic doses that may be used in the methods of the invention are about 10 to about 150 IU/kg, more specifically, about 100-110, 110-120, 120-130, 130-140, 140-150 IU/kg, and more specifically, about 110, 115, 120, 125, 130, 135, 140, 145, or 150 IU/kg.

"Variant," as used herein, refers to a polynucleotide or polypeptide differing from the original polynucleotide or polypeptide, but retaining essential properties thereof, e.g., factor VIII coagulant activity or Fc (FcRn binding) activity. Generally, variants are overall closely similar, and, in many regions, identical to the original polynucleotide or polypeptide. Variants include, e.g., polypeptide and polynucleotide fragments, deletions, insertions, and modified versions of original polypeptides.

Variant polynucleotides may comprise, or alternatively consist of, a nucleotide sequence which is at least 85%, 90%, 95%, 96%, 97%, 98% or 99% identical to, for example, the nucleotide coding sequence in SEQ ID NO:1, 3, or 5 (the factor VIII portion, the Fc portion, individually or together) or the complementary strand thereto, the nucleotide coding sequence of known mutant and recombinant factor VIII or Fc such as those disclosed in the publications and patents cited herein or the complementary strand thereto, a nucleotide sequence encoding the polypeptide of SEQ ID NO:2, 4, or 6 (the factor VIII portion, the Fc portion, individually or together), and/or polynucleotide fragments of any of these nucleic acid molecules (e.g., those fragments described herein). Polynucleotides which hybridize to these nucleic acid molecules under stringent hybridization conditions or lower stringency conditions are also included as variants, as are polypeptides encoded by these polynucleotides as long as they are functional.

Variant polypeptides may comprise, or alternatively consist of, an amino acid sequence which is at least 85%, 90%, 95%, 96%, 97%, 98%, 99% identical to, for example, the polypeptide sequence shown in SEQ ID NOS:2, 4, or 6 (the factor VIII portion, the Fc portion, individually or together), and/or polypeptide fragments of any of these polypeptides (e.g., those fragments described herein).

By a nucleic acid having a nucleotide sequence at least, for example, 95% "identical" to a reference nucleotide sequence, it is intended that the nucleotide sequence of the nucleic acid is identical to the reference sequence except that the nucleotide sequence may include up to five point mutations per each 100 nucleotides of the reference nucleotide sequence. In other words, to obtain a nucleic acid having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. The query sequence may be, for example, the entire sequence shown in SEQ ID NO:1 or 3, the ORF (open reading frame), or any fragment specified as described herein.

As a practical matter, whether any particular nucleic acid molecule or polypeptide is at least 85%, 90%, 95%, 96%, 97%, 98% or 99% identical to a nucleotide sequence or polypeptide of the present invention can be determined conventionally using known computer programs. In one embodiment, a method for determining the best overall match between a query sequence (reference or original sequence) and a subject sequence, also referred to as a global sequence alignment, can be determined using the FASTDB computer program based on the algorithm of Brutlag et al., Comp. App. Biosci. 6:237-245 (1990). In a sequence alignment the query and subject sequences are both DNA sequences. An RNA sequence can be compared by converting U's to T's. The result of said global sequence alignment is in percent identity. In another embodiment, parameters used in a FASTDB alignment of DNA sequences to calculate percent identity are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the length of the subject nucleotide sequence, whichever is shorter.

If the subject sequence is shorter than the query sequence because of 5' or 3' deletions, not because of internal deletions, a manual correction must be made to the results. This is because the FASTDB program does not account for 5' and 3' truncations of the subject sequence when calculating percent identity. For subject sequences truncated at the 5' or 3' ends, relative to the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present invention. Only bases outside the 5' and 3' bases of the subject sequence, as displayed by the FASTDB alignment, which are not matched/aligned with the query sequence, are calculated for the purposes of manually adjusting the percent identity score.

For example, a 90 base subject sequence is aligned to a 100 base query sequence to determine percent identity. The deletions occur at the 5' end of the subject sequence and therefore, the FASTDB alignment does not show a matched/alignment of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequence are manually corrected for. No other manual corrections are to made for the purposes of the present invention.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, (indels) or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 85%, 90%, 95%, 96%, 97%, 98% or 99% identical to, for instance, the amino acid sequences of SEQ ID NO:2 (the factor VIII portion, the Fc portion, individually or together) or 4, or a known factor VIII or Fc polypeptide sequence, can be determined conventionally using known computer programs. In one embodiment, a method for determining the best overall match between a query sequence (reference or original sequence) and a subject sequence, also referred to as a global sequence alignment, can be determined using the FASTDB computer program based on the algorithm of Brutlag et al., Comp. App. Biosci. 6:237-245 (1990). In a sequence alignment the query and subject sequences are either both nucleotide sequences or both amino acid sequences. The result of said global sequence alignment is in percent identity. In another embodiment, parameters used in a FASTDB amino acid alignment are: Matrix=PAM 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This is because the FASTDB program does not account for N- and C-terminal truncations of the subject sequence when calculating global percent identity. For subject sequences truncated at the N- and C-termini, relative to the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C- termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequence are manually corrected for. No other manual corrections are to made for the purposes of the present invention.

The polynucleotide variants may contain alterations in the coding regions, non-coding regions, or both. In one embodiment, the polynucleotide variants contain alterations which produce silent substitutions, additions, or deletions, but do not alter the properties or activities of the encoded polypeptide. In another embodiment, nucleotide variants are produced by silent substitutions due to the degeneracy of the genetic code. In other embodiments, variants in which 5-10, 1-5, or 1-2 amino acids are substituted, deleted, or added in any combination. Polynucleotide variants can be produced for a variety of reasons, e.g., to optimize codon expression for a particular host (change codons in the human mRNA to others, e.g., a bacterial host such as *E*. *coli*)*.*

Naturally occurring variants are called "allelic variants," and refer to one of several alternate forms of a gene occupying a given locus on a chromosome of an organism (Genes II, Lewin, B., ed., John Wiley & Sons, New York (1985)). These allelic variants can vary at either the polynucleotide and/or polypeptide level and are included in the present invention. Alternatively, non-naturally occurring variants may be produced by mutagenesis techniques or by direct synthesis.

Using known methods of protein engineering and recombinant DNA technology, variants may be generated to improve or alter the characteristics of the polypeptides. For instance, one or more amino acids can be deleted from the N-terminus or C-terminus of the secreted protein without substantial loss of biological function. Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein (Dobeli et al., J. Biotechnology 7:199-216 (1988).

Moreover, ample evidence demonstrates that variants often retain a biological activity similar to that of the naturally occurring protein. For example, Gayle and coworkers (J. Biol. Chem 268:22105-22111 (1993), conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See Abstract.) In fact, only 23 unique amino acid sequences, out of more than 3,500 nucleotide sequences examined, produced a protein that significantly differed in activity from wild-type.

As stated above, polypeptide variants include, e.g., modified polypeptides. Modifications include, e.g., acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation (Mei et al., Blood 116:270-79 (2010)), proteolytic processing, phosphorylation, prenylation, racemization, selenoylation, sulfation, transfer-RNA mediated addition of amino acids to proteins such as arginylation, and ubiquitination. In some embodiments, Factor VIII is modified, e.g., pegylated, at any convenient location. In some embodiments, Factor VIII is pegylated at a surface exposed amino acid of Factor VIII, e.g., a surface exposed cysteine, which may be an engineered cysteine. Id.. In some embodiments, modified Factor VIII, e.g., pegylated Factor VIII, is a long-acting Factor VIII.

"Volume of distribution at steady state (Vss)," as used herein, has the same meaning as the term used in pharmacology, which is the apparent space (volume) into which a drug distributes. Vss = the amount of drug in the body divided by the plasma concentration at steady state.

"About," as used herein for a range, modifies both ends of the range. Thus, "about 10-20" means "about 10 to about 20."

"Processed Factor VIII," as used herein means Factor VIII that has been cleaved at Arginine 1648 (for full-length Factor VIII) or Arginine 754 (for B-domain deleted Factor VIII), i.e., intracellular processing site. Due to the cleavage at the intracellular processing site, processed Factor VIII comprises two polypeptide chains, the first chain being a heavy chain and the second chain being a light chain. For example, the processed Factor VIII-Fc fusion protein (i.e., Heavy chain and Light chain fused to Fc) run at approximately 90 kDa and 130 kDa on a non-reducing SDS-PAGE, respectively, and 90 kDa and 105 kDa on a reducing SDS-PAGE, respectively. Therefore, 60%, about 70%, about 75%, about 80%, about 85%, about 90%, about 95%, about 96%, about 97%, about 98%, or about 99% of the Factor VIII portion in the chimeric polypeptide is processed Factor VIII.

"Single chain Factor VIII," "SC Factor VIII," or "SCFVIII" as used herein means Factor VIII that has not been cleaved at the Arginine site (residue 1648 for full-length Factor VIII (i.e., residue 1667 of SEQ ID NO: 6) or residue 754 for B-domain deleted Factor VIII (i.e., residue 773 of SEQ ID NO: 2). Therefore, single chain Factor VIII in the chimeric polypeptide used herein comprises a single chain. In one embodiment, the single chain Factor VIII contains an intact intracellular processing site. In another embodiment, the single chain Factor VIII of the invention comprises a substitution or mutation at an amino acid position corresponding to Arginine 1645, a substitution or mutation at an amino acid position corresponding to Arginine 1648, or a substitution or mutation at amino acid positions corresponding to Arginine 1645 and Arginine 1648 in full-length Factor VIII. In other embodments, the amino acid substituted at the amino acid position corresponding to Arginine 1645 is a different amino acid from the amino acid substituted at the amino acid position corresponding to Arginine 1648. In certain embodiments, the substitution or mutation is an amino acid other than arginine, e.g., isoleucine, leucine, lysine, methionine, phenylalanine, threonine, tryptophan, valine, alanine, asparagine, aspartic acid, cysteine, glutamic acid, glutamine, glycine, proline, selenocysteine, serine, tyrosine, histidine, ornithine, pyrrolysine, or taurine. The single chain Factor VIII-Fc fusion protein can run at approximately 220 kDa on a non reducing SDS-PAGE and at approximately 195 kDa on a reducing SDS-PAGE.

Between 1% and 40% of the Factor VIII portion of the chimeric polypeptide is single chain Factor VIII. In other embodiments, about 1%-about 10%, about 5%-about 15%, about 10%-about 20%, about 15%-about 25%, about 20%-about 30%, about 25%-about 35%, about 30%-about 40% of the Factor VIII portion of the chimeric polypeptide used herein is single chain Factor VIII. In a particular embodiment, about 1%, about 5%, about 10%, about 15%, about 20% about 25%, about 30%, about 35% of the Factor VIII portion of the chimeric polypeptide used herein is single chain Factor VIII. In some embodiments, the ratio of the single chain Factor VIII to the processed Factor VIII of the chimeric polypeptide is (a) about 25% of single chain Factor VIII and about 75% of processed Factor VIII; (b) about 20% of single chain Factor VIII and about 80% of processed Factor VIII; (c) about 15% of single chain Factor VIII and about 85% of processed Factor VIII; (d) about 10% of single chain Factor VIII and about 90% of processed Factor VIII; (e) about 5% of single chain Factor VIII and about 95% of processed Factor VIII; (f) about 1% of single chain Factor VIII and about 99% of processed Factor VIII; (g) about 30% of single chain Factor VIII and about 70% of processed Factor VIII, (h) about 35% of single chain Factor VIII and about 65% of processed Factor VIII, or (i) about 40% of single chain Factor VIII and about 60% of processed Factor VIII. In other embodiments, the ratio of the single chain Factor VIII to the processed Factor VIII of the chimeric polypeptide is (a) about 30% of single chain Factor VIII and about 70% of processed Factor VIII; or (b) about 40% of single chain Factor VIII and about 60% of processed Factor VIII.

The Factor VIII portion in the chimeric polypeptide used herein has Factor VIII activity. Factor VIII activity can be measured by any known methods in the art. For example, one of those methods can be a chromogenic assay. The chromogenic assay mechanism is based on the principles of the blood coagulation cascade, where activated Factor VIII accelerates the conversion of Factor X into Factor Xa in the presence of activated Factor IX, phospholipids and calcium ions. The Factor Xa activity is assessed by hydrolysis of a p-nitroanilide (pNA) substrate specific to Factor Xa. The initial rate of release of p-nitroaniline measured at 405 nM is directly proportional to the Factor Xa activity and thus to the Factor VIII activity in the sample. The chromogenic assay is recommended by the Factor VIII and Factor IX Subcommittee of the Scientific and Standardization Committee (SSC) of the International Society on Thrombosis and Hemostatsis (ISTH). Since 1994, the chromogenic assay has also been the reference method of the European Pharmacopoeia for the assignment of FVIII concentrate potency. Thus, in one embodiment, the chimeric polypeptide comprising single chain Factor VIII has Factor VIII activity comparable to a chimeric polypeptide comprising processed Factor VIII (e.g., a chimeric polypeptide consisting essentially of or consisting of two Fc portions and processed Factor VIII, wherein said processed Factor VIII is fused to one of the two Fc portions), when the Factor VIII activity is measured in vitro by a chromogenic assay.

In another embodiment, the chimeric polypeptide comprising single chain Factor VIII of this invention has a Factor Xa generation rate comparable to a chimeric polypeptide comprising processed Factor VIII (e.g., a chimeric polypeptide consisting essentially of or consisting of two Fc portions and processed Factor VIII, wherein the processed Factor VIII is fused to one Fc of the two Fc portions).

In order to activate Factor X to Factor Xa, activated Factor IX (Factor IXa) hydrolyses one arginine-isoleucine bond in Factor X to form Factor Xa in the presence of Ca2+, membrane phospholipids, and a Factor VIII cofactor. Therefore, the interaction of Factor VIII with Factor IX is critical in coagulation pathway. In certain embodiments, the chimeric polypeptide comprising single chain factor VIII can interact with Factor IXa at a rate comparable to a chimeric polypeptide comprising processed Factor VIII (e.g., a chimeric polypeptide consisting essentially of or consisting of two Fc portions and processed Factor VIII, wherein the processed Factor VIII is fused to one Fc of the two Fc portions).

In addition, Factor VIII is bound to von Willebrand Factor while inactive in circulation. Factor VIII degrades rapidly when not bound to vWF and is released from vWF by the action of thrombin. In some embodiments, the chimeric polypeptide comprising single chain Factor VIII binds to von Willebrand Factor at a level comparable to a chimeric polypeptide comprising processed Factor VIII (e.g., a chimeric polypeptide consisting essentially of or consisting of two Fc portions and processed Factor VIII, wherein the processed Factor VIII is fused to one Fc of the two Fc portions).

Factor VIII can be inactivated by activated protein C in the presence of calcium and phospholipids. Activated protein C cleaves Factor VIII heavy chain after Arginine 336 in the A1 domain, which disrupts a Factor X substrate interaction site, and cleaves after Arginine 562 in the A2 domain, which enhances the dissociation of the A2 domain as well as disrupts an interaction site with the Factor IXa. This cleavage also bisects the A2 domain (43 kDa) and generates A2-N (18 kDa) and A2-C (25 kDa) domains. Thus, activated protein C can catalyze multiple cleavage sites in the heavy chain. In one embodiment, the chimeric polypeptide comprising single chain Factor VIII is inactivated by activated Protein C at a level comparable to a chimeric polypeptide comprising processed Factor VIII (e.g., a chimeric polypeptide consisting essentially of or consisting of two Fc portions and processed Factor VIII, wherein the processed Factor VIII is fused to one Fc of the two Fc portions).

In other embodiments, the chimeric polypeptide comprising single chain Factor VIII has Factor VIII activity in vivo comparable to a chimeric polypeptide comprising processed Factor VIII (e.g., a chimeric polypeptide consisting essentially of or consisting of two Fc portions and processed Factor VIII, wherein the processed Factor VIII is fused to one Fc of the two Fc portions). In a particular embodiment, the chimeric polypeptide comprising single chain Factor VIII is capable of protecting a HemA mouse at a level comparable to a chimeric polypeptide comprising processed Factor VIII (e.g., a chimeric polypeptide consisting essentially of or consisting of two Fc portions and processed Factor VIII, wherein said processed Factor VIII is fused to one Fc of the two Fc portions) in a HemA mouse tail vein transection model.

The term "comparable" as used herein means a compared rate or level resulted from using the chimeric polypeptide is equal to, substantially equal to, or similar to the reference rate or level. The term "similar" as used herein means a compared rate or level has a difference of no more than 10% or no more than 15% from the reference rate or level (e.g., FXa generation rate by a chimeric polypeptide consisting essentially of or consisting of two Fc portions and processed Factor VIII, wherein said processed Factor VIII is fused to one Fc of the two Fc portions). The term "substantially equal" means a compared rate or level has a difference of no more than 0.01%, 0.5% or 1% from the reference rate or level.

In certain embodiments, the composition of the present invention has Factor VIII activity comparable to the composition comprising processed Factor VIII (e.g., a composition comprising a chimeric polypeptide, which consists essentially of or consists of two Fc portions and processed Factor VIII, wherein said processed Factor VIII is fused to one of the two Fc portions), when the Factor VIII activity is measured in vitro by a chromogenic assay.

In other embodiments, the composition of the invention has a Factor Xa generation rate comparable to a composition comprising processed Factor VIII (e.g., a composition comprising a chimeric polypeptide, which consists essentially of or consists of two Fc portions and processed Factor VIII, wherein the processed Factor VIII is fused to one Fc of the two Fc portions). In still other embodiments, the composition comprising single chain factor VIII can interact with Factor IXa at a rate comparable to a composition comprising processed Factor VIII (e.g., a composition comprising a chimeric polypeptide, which consists essentially of or consists of two Fc portions and processed Factor VIII, wherein the processed Factor VIII is fused to one Fc). In further embodiments, the single chain Factor VIII in the chimeric polypeptide of the present composition is inactivated by activated Protein C at a level comparable to processed Factor VIII in a chimeric polypeptide of a composition (e.g., a composition comprising a chimeric polypeptide, which consists essentially of or consists of two Fc portions and processed Factor VIII, wherein the processed Factor VIII is fused to one Fc of the two Fc portions). In a particular embodiment, the composition comprising single chain Factor VIII has Factor VIII activity in vivo comparable to the composition comprising processed Factor VIII (e.g., a composition comprising a chimeric polypeptide, which consists essentially of or consists of two Fc portions and processed Factor VIII, wherein the processed Factor VIII is fused to one Fc of the two Fc portions). In some embodiments, the composition comprising single chain Factor VIII of the invention is capable of protecting HemA mouse at a level comparable to the composition comprising processed Factor VIII (e.g., a composition comprising a chimeric polypeptide, which consists essentially of or consists of two Fc portions and processed Factor VIII, wherein said processed Factor VIII is fused to one Fc of the two Fc portions) in HemA mouse tail vein transection model.

The present invention further provides the pharmaceutical composition for usein reducing bleeding in a (human) subject. This use may comprise administering to the subject in need thereof a therapeutically effective amount of a pharmaceutical composition/formulation comprising the chimeric polypeptide.

The bleeding condition can be caused by a blood coagulation disorder. A blood coagulation disorder can also be referred to as a coagulopathy. In one example, the blood coagulation disorder, which can be treated with a pharmaceutical composition of the current disclosure, is hemophilia or von Willebrand disease (vWD). In another example, the blood coagulation disorder, which can be treated with a pharmaceutical composition of the present disclosure is hemophilia A.

In some embodiments, the type of bleeding associated with the bleeding condition is selected from hemarthrosis, muscle bleed, oral bleed, hemorrhage, hemorrhage into muscles, oral hemorrhage, trauma, trauma capitis, gastrointestinal bleeding, intracranial hemorrhage, intra-abdominal hemorrhage, intrathoracic hemorrhage, bone fracture, central nervous system bleeding, bleeding in the retropharyngeal space, bleeding in the retroperitoneal space, and bleeding in the illiopsoas sheath.

In other embodiments, the subject suffering from bleeding condition is in need of treatment for surgery, including, e.g., surgical prophylaxis or peri-operative management. In one example, the surgery is selected from minor surgery and major surgery. Exemplary surgical procedures include tooth extraction, tonsillectomy, inguinal herniotomy, synovectomy, craniotomy, osteosynthesis, trauma surgery, intracranial surgery, intra-abdominal surgery, intrathoracic surgery, joint replacement surgery (e.g., total knee replacement, hip replacement, and the like), heart surgery, and caesarean section.

In another example, the subject is concomitantly treated with FIX. Because the compounds of the invention are capable of activating FIXa, they could be used to pre-activate the FIXa polypeptide before administration of the FIXa to the subject.

The pharmaceutical compositions may be formulated for any appropriate manner of administration, including, for example, topical (e.g., transdermal or ocular), oral, buccal, nasal, vaginal, rectal or parenteral administration.

The term parenteral as used herein includes subcutaneous, intradermal, intravascular (e.g., intravenous), intramuscular, spinal, intracranial, intrathecal, intraocular, periocular, intraorbital, intrasynovial and intraperitoneal injection, as well as any similar injection or infusion technique The composition can be also for example a suspension, emulsion, sustained release formulation, cream, gel or powder. The composition can be formulated as a suppository, with traditional binders and carriers such as triglycerides.

In one example, the pharmaceutical formulation is a liquid formulation, e.g., a buffered, isotonic, aqueous solution. In another example, the pharmaceutical composition has a pH that is physiologic, or close to physiologic. In other examples, the aqueous formulation has a physiologic or close to physiologic osmolarity and salinity. It can contain sodium chloride and/or sodium acetate. In some examples, the composition of the present invention is lyophilized.

Having now described the present invention in detail, the same will be more clearly understood by reference to the following examples, which are included herewith for purposes of illustration only and are not intended to be limiting of the invention.

### Examples

### Example 1

### Cloning, expression and purification of rFVIIIFc

All molecular biology procedures were performed following standard techniques. The coding sequence of human FVIII (Genbank accession number NM_000132), including its native signal sequence, was obtained by reverse transcription-polymerase chain reactions (RT-PCR) from human liver polyA RNA. Due to the large size of FVIII, the coding sequence was obtained in several sections from separate RT-PCR reactions, and assembled through a series of PCR reactions, restriction digests and ligations into an intermediate cloning vector containing a B domain deleted (BDD) FVIII coding region with a fusion of serine 743 (S743) to glutamine 1638 (Q1638), eliminating 2682 bp from the B domain of full length FVIII. The human IgG1 Fc sequence (e.g., GenBank accession number Y14735) was obtained by PCR from a leukocyte cDNA library, and the final expression cassette was made in such a way that the BDD FVIII sequence was fused directly to the N-terminus of the Fc sequence (hinge, CH2 and CH3 domains, beginning at D221 of the IgG1 sequence, EU numbering) with no intervening linker. For expression of the Fc chain alone, the mouse Igκ (kappa) light chain signal sequence was created with synthetic oligonucleotides and added to the Fc coding sequence using PCR to enable secretion of this protein product. The FVIIIFc and Fc chain coding sequences were cloned into a dual expression vector, pBudCE4.1 (Invitrogen, Carlsbad, CA).

HEK 293H cells (Invitrogen, Carlsbad, CA) were transfected with the pSYN-FVIII-013 plasmid using Lipofectamine transfection reagent (Invitrogen, Carlsbad, CA)), and a stable cell line was selected with zeocin. Cells were grown in serum free suspension culture, and rFVIIIFc protein purified from clarified harvest media using a four column purification process, including a FVIII-specific affinity purification step (McCue J. et al., J. Chromatogr. A., 1216(45): 7824-30 (2009)), followed by a combination of anion exchange columns and a hydrophobic interaction column.

### Example 2

### Biochemical characterization

Processed recombinant FVIII-Fc (rFVIIIFc) is synthesized as two polypeptide chains, one chain consisting of BDD-FVIII (S743-Q1638 fusion, 1438 amino acids) fused to the Fc domain (hinge, CH2 and CH3 domains) of IgG1 (226 amino acids, extending from D221 to G456, EU numbering), for a total chain length of 1664 amino acids, the other chain consisting of the same Fc region alone (226 amino acids). Though cells transfected with the FVIIIFc/Fc dual expression plasmid were expected to secrete three products (FVIIIFc dimer, FVIIIFc monomer, and Fc dimer), only the FVIIIFc monomer and Fc dimer were detected in conditioned media. Purified FVIIIFc was analyzed by non-reducing and reducing SDS-PAGE analysis (Figure 2A and, B). For the nonreduced SDS-PAGE, bands were found migrating at approximately 90 kDa and 130 kDa, consistent with the predicted molecular weights of the FVIIIFc heavy chain (HC) and light chain-dimeric Fc fusion (LCFc2) (Figure 2A, lane 3). A third band was also detected at approximately 220 kDa, consistent with the predicted molecular weight for single chain FVIIIFc (SC FVIIIFc; HC+LCFc2), in which the arginine residue at position 754 (1648 with respect to the full length sequence) is not cleaved during secretion. For the reduced SDS-PAGE analysis, major bands were seen migrating at approximately 25 kDa, 90 kDa, 105 kDa, and 195 kDa, consistent with the predicted molecular weights for the single chain Fc, HC, LCFc, and SC FVIIIFc (Figure 2B, lane 3). Cotransfection with human PC5, a member of the proprotein convertase subtlisin/kexin (PCSK) type proteases, resulted in full processing of the rFVIIIFc product (Figure 2A, B, lane 2).

Densitometry analysis of several batches of rFVIIIFc after SDS-PAGE indicated greater than 98% purity of the expected bands. Size exclusion chromatography (SEC) was also used to assess the degree of aggregation present, and all batches were found to have aggregate levels at 0.5% or less.

rFVIIIFc structure was further analyzed by thrombin cleavage, reduction, and analysis by LC/UV and LC/MS. The four Factor VIII fragments generated by thrombin (by cleavages at three arginine residues, at positions 372, 740 and 795 (795 corresponds to 1689 with respect to the full length FVIII sequence), can be detected by UV absorbance (Figure 2C), corresponding to the following segments of the protein: Fc (peak 1), light-chain-Fc (peak 2); the A1 domain from the heavy chain (peak 3) and the A2 domain from the heavy chain (peak 4). The 14 amino acid B domain linker and ~ 6 kDa a3-related peptides are not detected by UV absorbance due to their small size.

Analysis of the thrombin digestion of rFVIIIFc by HPLC/MS provided further detailed information of the four main domains as well as the ~6 kDa a3 related peptides, and was compared to REFACTO^{®}, a CHO-derived recombinant BDD-FVIII protein (rBDD FVIII), using the same methods. FVIII samples were passed through Detergent-OUTDTG-100X columns (GBioscience, Maryland Heights, MO) for the removal of Tween, fully digested with thrombin, reduced and analyzed either by RP-HPLC-UV (POROS R1/10, Applied Biosystems) or RP-HPLC-MS (Agilent 1200 coupled to an Agilent 6210TOF mass spectrometer) using gradients of acetonitrile in water + 0.1% formic acid. Peptide sequence was also confirmed with LysC peptide mapping, analyzed by RP-HPLC/MS (Thermo Finnigan LTQ-XL-ETD).

As expected, the total ion current (TIC) chromatogram of rFVIIIFc (Figure 2D) appears similar to the UV chromatogram (Figure 2C). Five of the expected six products can be detected by LC/MS, including two forms of the a3 acidic region generated from the processed and single chain isoforms, as well as the thrombin used for the digestion. An additional truncated form of the a3 acidic region was also observed, and is described more fully below. rBDD FVIII yielded a similar TIC chromatogram, but without the free Fc chain and having a different mass for the LC compared to the rFVIIIFc LC-Fc, consistent with the lack of an Fc region (data not shown).

Due to the heterogeneity of glycosylation over much of the protein, the deconvoluted mass spectra for the A1, LC-Fc, and Fc regions are complex and therefore the identity of all of the molecular ions have not been established. However, the observed mass for the three major peaks from the Fc region were found to match the G0, G1, and G2 isoforms found on IgG molecules, corresponding to biantennary oligosaccharides terminating in 0, 1 or 2 galactose residues. The deconvoluted mass spectra of the a3-related peptides and the A2 domain provide the most definitive data, as there are no heterogeneity in the posttranslational modifications in these regions, allowing the expected masses to be identified unambiguously.

The 6 kDa N-terminal peptide released from the LC after cleavage of R1689 is predicted to comprise the a3 acidic region (amino acids E1649 to R1689) if derived from the processed isoform, and the 14 amino acid truncated B domain fused to the a3 acidic region if derived from the single chain isoform. Both rFVIIIFc and rBDD FVIII were found to contain both forms of the a3 region, proportional to the expected levels based on SDS-PAGE analysis. In addition, both proteins contained a truncated form of the a3 region corresponding to amino acids D1658-R1689, as has been reported for other FVIII products, though this was found in greater abundance in rBDD FVIII than in rFVIIIFc.

The A2 domain contains three potential tyrosine sulfation sites, but no glycosylation sites that could result in complex heterogeneity, and therefore the exact masses of this region can be calculated. In addition to the primary expected peak in the deconvoluted mass spectrum of rFVIIIFc correlating to the mass of the S373 to R740 sequence (Figure 2E), two additional forms were identified corresponding to known truncations of the FVIII HC, correlating with an A2 domain truncated at E720 and Y729. These reported truncated forms were also observed directly in the deconvoluted spectrum of rBDD FVIII A2 (Figure 2E). Both rFVIIIFc and rBDD FVIII A2 contained similar relative amounts of the form truncated at Y729 while the rBDD FVIII A2 domain contained a notably greater level of the form truncated at E720 as compared to the same form in rFVIIIFc (Figure 2E).

The primary sequence of rFVIIIFc was confirmed by peptide mapping with lysyl endopeptidase (Lys-C) digests followed by UV and mass spectrometric detection. Of the 99 theoretical peptides produced from rFVIIIFc, 81 were detected, corresponding to 98% of the total sequence. The posttranslational modifications of rFVIIIFc were also characterized by this method. FVIII contains 6 potential tyrosine sulfation sites, corresponding to positions 346, 718, 719, 723, 1664, and 1680. Fully sulfated peptides corresponding to these six sites were found, with trace amounts of non-sulfated peptide corresponding to position 1680 as assessed by integration of the total ion chromatogram in the mass spectra, and no detectable non-sulfated peptides corresponding to the other positions. BDD FVIII also contains 6 potential N-glycosylation sites, four of which have been reported to be glycosylated in recombinant FVIII products. Consistent with this, rFVIIIFc was found to have the same 4 sites glycosylated; N239 and N2118 were found to contain high mannose structures, while N41 and N1810 were found to contain more complex carbohydrates, similar to those found on rBDD FVIII. The Fc region N-linked glycosylation was found to match the G0, G1, and G2 isoforms found with the thrombin map by LC/MS. FVIII has been reported to have O-glycosylation sites at Ser 741 and 743 that are partially occupied, and this was found to be the case with rFVIIIFc as well.

The rFVIIIFc polypeptide produced without cotransfected processing enzymes exhibited 15-25% single chain FVIIIFc (SC FVIIIFc), which differs from processed rFVIIIFc by a single peptide bond between R754 and E755 (R1648/E1649 with respect to the full length FVIII). This isoform was purified and characterized in all of the biochemical assays described above, and found to be comparable to rFVIIIFc as shown below. The activity of purified single chain FVIIIFc was found to be similar to rFVIIIFc in a chromogenic assay as well as by the various functional assays described below.

### Measurement of FVIII Activity by Chromogenic and One-Stage aPTT Assays

FVIII activity was measured by a FVIII chromogenic assay. The average specific activity from four separate batches of rFVIIIFc was found to be 9762 ± 449 IU/mg by the chromogenic assay, corresponding to 2148±99 IU/nmol. The average specific activity from fourteen separate batches of rFVIIIFc was found to be 8460 ± 699 IU/mg by the aPTT assay, and 9348 ± 1353 IU/mg by the chromogenic assay, corresponding to 1861±154 and 2057±298 IU/nmol, respectively. FVIII activity of single chain FVIII:Fc was also measured by the chromogenic assay and compared to the completely processed rFVIII:Fc or rFVIII:Fc DS (containing about 25% single chain rFVIII:Fc). As Table 3A shows, single chain rFVIIIFc showed no significant difference in FVIII activity compared to the Factor VIII activity of completely processed FVIIIFc or rFVIIIFc DS by the chromogenic assay, both in the presence and the absence of von Willebrand Factor (VWF). Table 3B shows that full activity of SCrFVIIIFc, as measured by one-stage activated partial thromboplastin time (aPTT) assay, was observed in the absence of VWF.

**TABLE_3A_FVIII Activity by Chromogenic Assay**

| Matrix | Sample | Chromogenic Specific Activity(IU/mg) | %CV* |
|---|---|---|---|
| FVIII depleted plasma | rFVIIIFcDS (25% NP) (RECD-19189-09-013) | 9066 | 2.49 |
| | Single chain rFVIIIFc (purified from RECD 19189-09-013) | 8194 | 2.72 |
| | Completely Processed rFVIIIFc (purified from an engineered cell line) | 9577 | 8.34 |
| FVIII and vWF depleted plasma | rFVIIIFcDS (25% NP) (RECD-19189-09-013) | 10801 | 8.92 |
| | Single chain rFVIIIFc (purified from RECD 19189-09-013) | 9498 | 4.70 |
| | Completely Processed rFVIIIFc (purified from an engineered cell line) | 9569 | 4.54 |
| FVIII/VWF-depleted plasma supplemented with human VWF | rFVIIIFc | 9982 | 4.3 |
| | SC rFVIIIFc | 8984 | 4.6 |
| | Completely-processed rFVIIIFc | 8275 | 8.2 |

| | | | |
|---|---|---|---|
| *CV=coefficient of variation | | | |

**TABLE _3B_FVIII Activity by aPTT assay**

| Matrix | Sample | Coagulation (aPTT) Specific Activity (IU/mg) | %CV |
|---|---|---|---|
| FVIII-depleted plasma | rFVIIIFcDS (25% NP) (RECD-19189-09-013) | 8210 | 5.88 |
| | Single chain rFVIIIFc (purified from RECD 19189-09-013) | 3108 | 6.57 |
| | Completely Processed rFVIIIFc (purified from an engineered cell line) | 8683 | 3.57 |
| FVIII and vWF depleted plasma | rFVIIIFcDS (25% NP) (RECD-19189-09-013) | 15621 | 6.47 |
| | Single chain rFVIIIFc (purified from RECD 19189-09-013) | 13572 | 2.41 |
| | Completely Processed rFVIIIFc (purified from an engineered cell line) | 15170 | 10.42 |
| FVIII/VWF-depleted plasma supplemented with human VWF | rFVIIIFc | 7742 | 7.4 |
| | SC rFVIIIFc | 3133 | 4.9 |
| | Completely-processed rFVIIIFc | 8495 | 4.0 |

In one-stage clotting assay (APTT), SC rFVIIIFc demonstrated a 60% decrease in activity when the plasma has normal VWF level, suggesting the potential role of VWF in the activation of SC rFVIIIFc. This observation was further confirmed by addition of human VWF back to the FVIII/VWF-depleted plasma (Table 3), where the coagulant activity of SC rFVIIIFc was reduced to the same level as in congenital FVIII-deficient plasma.

### Activity in Xase Complex

FVIII activity was also measured in the context of the Xase complex, by incubating activated FIX and thrombin-activated REFACTO^{®} or rFVIIIFc protein on a phospholipid surface in the presence of calcium, and monitoring the conversion of FX to FXa as measured by cleavage of a chromogenic or fluorogenic substrate, from which FXa generation rates were determined. This assay was then modified by varying one component of the assay while keeping the others constant in order to examine the interactions with each individual component.

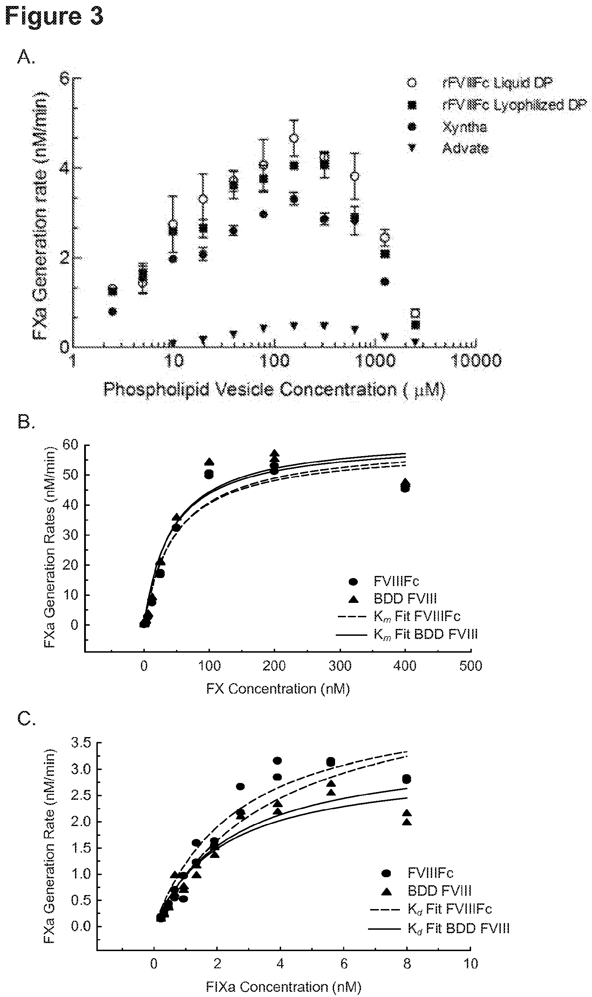

The FXa generation rate was determined as a function of varying phospholipid concentrations for rFVIIIFc DS, rBDD FVIII, and single chain rFVIIIFc (Figure 3A), using synthetic phospholipid vesicles (25% phosphotadyl serine/75% phosphotadyl choline). Both proteins were found to have a similar activity profile, with peak activity at approximately 156 µM phospholipids.

The FXa generation rate was then determined as a function of varying FX concentrations, and Km and Vmax values calculated (Figure 3B). The activity profiles for rFVIIIFc DS, rBDD FVIII, and single chain rFVIIIFc were found to be similar, with similar Km and Vmax (Table 4). Finally, the FXa generation rate was determined as a function of varying FIX concentrations (Figure 3C). The activity profiles appeared similar, with similar Kd and Vmax (Table 4). Similar results were obtained using platelets as a phospholipid source (unpublished data, June 2009).

**TABLE 4. FXa Generation Parameters for FVIII Proteins on Phospholipids**

| Lipid Source | Molecule | Km (nM) | Vmax (nM/min) |
|---|---|---|---|
| | rFVIIIFc DS | 55.0 ± 5.9 | 65.6 ± 8.6 |
| 25%PS-75%PC | rBDD FVIII | 51.0 ± 8.7 | 73.5 ± 10.1 |
| | NP rFVIIIFc | 53.2 ± 7.5 | 56.0 ± 13.8 |

**TABLE 5. FIXa Interactions with FVIII Proteins**

| Lipid Source | Molecule | Km (nM) | Vmax (nM/min) |
|---|---|---|---|
| | rFVIIIFc DS | 2.8 ± 0.4 | 4.5 ± 0.3 |
| 25%PS-75%PC | rBDD FVIII | 2.5 ± 0.3 | 4.0 ± 1.0 |
| | NP rFVIIIFc | 2.3 ± 0.2 | 3.8 ± 0.4 |

### Inactivation by APC

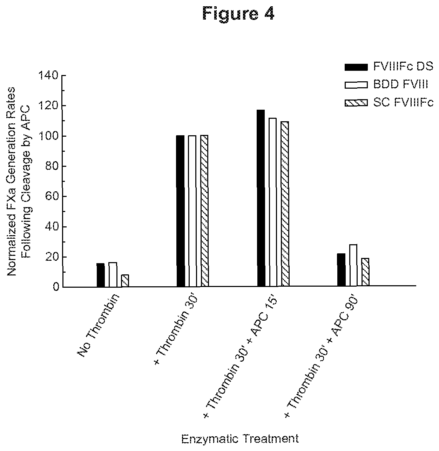

Once active, FVIII is inactivated by cleavage by activated protein C (APC), as well as by dissociation of the A2 domain. rFVIIIFc and rBDD FVIII were both activated by thrombin, then incubated with APC for different times and activity determined in a FXa generation assay (Figure 4). In the absence of thrombin activation, little FXa generation was detected, and this was increased significantly with thrombin digestion. Treatment with APC for 90 min led to a significant decrease in FXa generation rates, similar to non-activated samples, and these results were similar for rFVIIIFc DS, rBDD FVIII, and single chain rFVIIIFc.

### Affinity for vWF

FVIII interactions with von Willebrand factor (vWF) were measured by real-time biomolecular interaction analysis (BIAcore), based on surface Plasmon resonance (SPR) technology, to determine the kinetics of binding of rFVIIIFc and rBDD FVIII towards vWF (Table 6). Kinetic rate parameters of Ka (on-rate) and Kd (off-rate), and the affinity KD (Kd/Ka), were determined for each FVIII interaction under identical conditions. Both rFVIIIFc and rBDD FVIII were found to have a low nM binding affinity (KD) for vWF, of 1.64±0.37 and 0.846±0.181 nM, respectively. The proteins had similar off-rates, with a two fold difference in on-rate resulting in a two fold difference in the affinity.

**TABLE 6. Biocore Binding Analysis of FVIII Proteins to vWF**

| | | | Kinetic rate parameters | | Off-rate/On-rate |
|---|---|---|---|---|---|
| Analyte | Ligand | N | On-rate (M-1s-1) | Off-rate (s-1) | KD(M) |
| rFVIIIFc DS | hvWf | 5 | 7.92 ± 1.51x10⁵ | 1.25 ± 1.12x10⁻³ | 1.64 ± 0.37x10⁻⁹ |
| NP rFVIIIFc | hvWf | 5 | 8.66 ± 1.10x10⁵ | 1.09 ± 0.09x10⁻³ | 1.28 ± 0.22x10⁻⁹ |
| rBDD FVIII | hvWf | 5 | 13.7 ± 1.50x10⁵ | 1.14 ± 0.12x10⁻³ | 0.846 ± 0.181x10⁻⁹ |

As shown in Table 6, the affinity of rFVIII:Fc DS or single chain rFVIIIFc with vWF was found to be in the low nM range, approximately two fold greater than that of BDD FVIII alone. At physiological concentrations, this would result in a slight decrease in the percentage of rFVIIIFc (processed or single chain) complexed with vWF as compared to free FVIII, however in vivo studies have indicated that the half life of rFVIIIFc is significantly prolonged over full length or BDD FVIII despite this slightly lower affinity, and therefore this does not appear to compromise the half life of the molecule. It may be possible that the free rFVIIIFc is more efficiently recycled through the FcRn pathway and therefore this may contribute to a greater prolongation of half life.

### Affinity for vWF and Thrombin-mediated Release from vWF

Recombinant B-domain deleted Factor VIIIFc (rFVIIIFc) was expressed in HEK293 cells. During biosynthesis in HEK293 cells, most of the rFVIIIFc is processed by limited proteolysis to generate a FVIII heavy chain (HC) and a FVIII light chain (LC) to which the Fc moiety is attached. Spontaneous disassociation of the HC and LC in plasma, and during storage of FVIII drug products, is thought to contribute to a loss of FVIII activity. The remaining portion of the biosynthesized rFVIIIFc, which is not processed, forms a single chain isoform of rFVIIIFc (SC rFVIIIFc), which may provide enhanced manufacturability and stability compared to the processed rFVIIIFc.

This example describes an assay comparing the interaction of SC rFVIIIFc with von Willebrand factor (vWF) in relation to the interaction of rFVIIIFc with vWF. Interactions with vWF were measured by real-time biomolecular interaction analysis (BIAcore), based on surface Plasmon resonance (SPR) technology, to determine the kinetics of binding of rFVIIIFc and SC rFVIIIFc towards vWF (Table 11 and Figure 16A). FVIII-free human plasma derived vWF was immobilized by amine coupling on the surface of a biosensor at levels low enough to prevent mass transport limitation. rFVIIIFc and SC rFVIIIFc were sequentially injected in single-cycle kinetics mode at concentrations ranging from 0.13 to 5.0 nM. Sensorgram data was fit to a 1:1 interaction model.

Kinetic rate parameters of Ka (on-rate) and Kd (off-rate), and the affinity KD (Kd/Ka), were determined for each FVIII interaction under identical conditions. Both rFVIIIFc and SC rFVIIIFc were found to have a low nM binding affinity (KD) for vWF, of 0.34±0.1 and 0.31±0.1 nM, respectively. Both isoforms also had similar on-rates and off-rates.

**TABLE 11. Biocore Binding Analysis of FVIII Proteins to vWF**

| | | | Kinetic rate parameters | | Off-rate/On-rate |
|---|---|---|---|---|---|
| Analyte | Ligand | N | On-rate (M-1s-1) | Off-rate (s-1) | KD(M) |
| rFVIIIFc | hvWf | 6 | 2.6 ± 0.4x10⁵ | 8.9 ± 1.3x10⁻⁴ | 3.4 ± 0.1x10⁻¹⁰ |
| SC rFVIIIFc | hvWf | 6 | 2.7 ± 0.1x10⁵ | 8.4 ± 0.4x10⁻⁴ | 3.1 ± 0.1x10⁻¹⁰ |

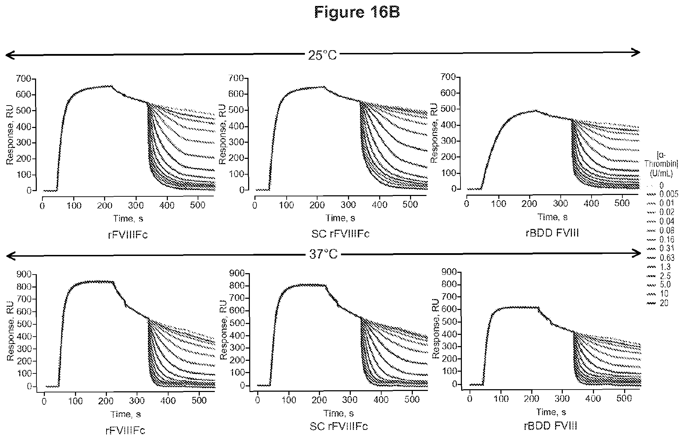

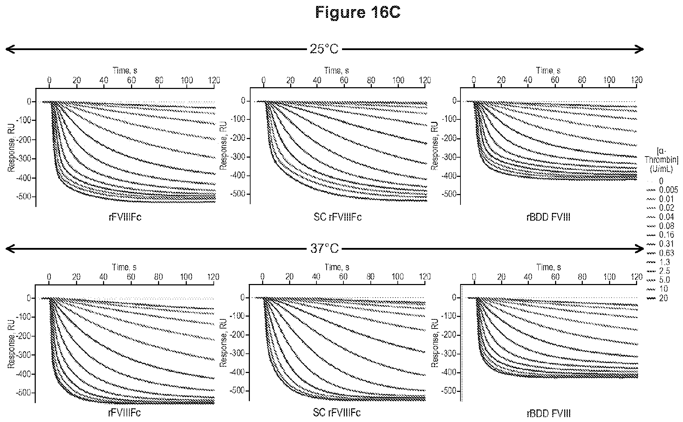

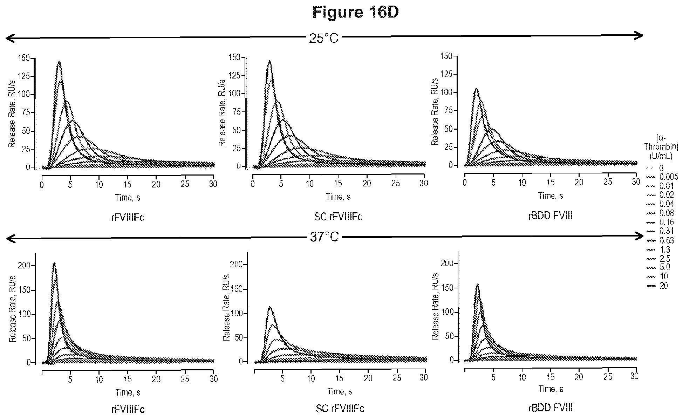

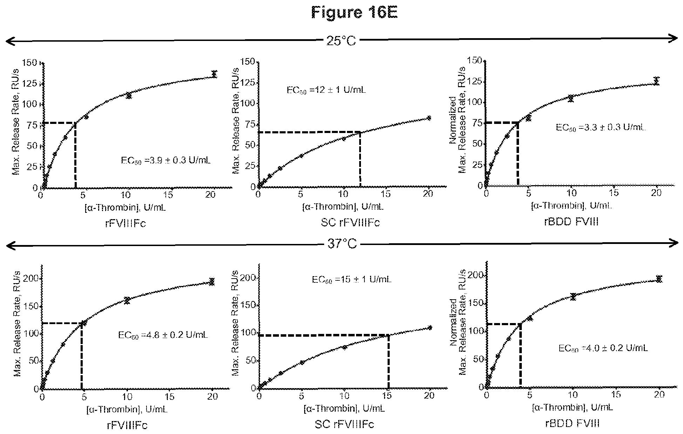

Next, thrombin-mediated release of rFVIIIFc, SC rFVIIIFc, and B-domain deleted FVIII lacking Fc moieties (rBDD FVIII) was measured at both 25° C and 37° C. Human vWF was immobilized by amine coupling at similar levels on three flow cells on the biosensor surface. The remaining flow cell served as a blank for reference purposes. The rFVIIIFc and SC rFVIIIFc proteins were captured by vWF and allowed to slowly disassociate, and the concentrations of rFVIIIFc and SC rFVIIIFc were adjusted to obtain equimolar capture levels by the end of the dissociation phase. Human α-thrombin solutions were prepared by 2-fold serial dilution and applied at concentrations that ranged from 0.005 to 20 U/mL, resulting in proteolytic release of FVIIIa species from vWF. Thrombin mediated release of activated rFVIIIFc, SC rFVIIIFc, and rBDD FVIII from vWF was monitored in real-time using an SPR-based optical biosensor (Figure 16B). Following blank reference subtraction (Figure 16C), the release rate as a function of α-thrombin concentration was determined (Figure 16D). The thrombin half maximal effective concentration (EC₅₀) for SC rFVIIIFc was 12±1 U/mL compared to 3.9±0.3 U/mL for rFVIIIFc at 25° C and was 15±1 U/mL for SC rFVIIIFc compared to 4.8±0.2 U/mL for rFVIIIFc at 37° C (Figure 16E). rFVIIIFc had a similar thrombin EC₅₀ value compared to rBDD FVIII, having values of 3.9±0.3 U/mL and 3.3±0.3 U/mL, respectively at 25° C and values of 4.8±0.2 U/mL and 4.0±0.2 U/mL, respectively at 37° C. SC rFVIIIFc had a thrombin EC₅₀ value that was approximately 3-fold higher than rFVIIIFc. This impairment of thrombin mediated release from vWF may underlie the specific reduction in specific activity for SC rFVIIIFc observed in the aPTT assay in which vWF was present (Table 3B).

### Example 3

A Phase I/IIa, open-label, crossover, dose-escalation, multi-center, and first-in-human study was designed to evaluate the safety, tolerability, and pharmacokinetics of a single dose of rFVIIIFc in subjects with severe (defined as <1 IU/dL [1%] endogenous factor VIII [FVIII]) hemophilia A. A total of approximately 12 previously treated patients were enrolled and dosed with rFVIIIFc at 25 or 65 IU/kg. After the screening (scheduled within 28 days prior to the first dose of the ADVATE^{®} [rFVIII], the reference comparator agent) and a minimum of 4-days (96 hours) elapsing with no FVIII treatment prior to the first injection, approximately 6 subjects received a single 25 IU/kg dose of ADVATE^{®} followed by a 3-day (72 hours) pharmacokinetic (PK) profile then crossover and receive a 25 IU/kg single, open-label dose of rFVIIIFc for a 7-day (168 hours) PK profiling. The first 3 subjects were dosed sequentially. For the first three (3) subjects dosed with 25 IU/kg of rFVIIIFc, each subject underwent an inhibitor assessment at 14-days (336 hours) post-injection of rFVIIIFc. Dosing of the next subject (for the first three subjects only) occurred once the inhibitor testing is completed. After the 3rd subject completed the 14 day inhibitor assessment, the remaining three subjects at 25 IU/kg and the six subjects at 65 IU/kg began enrollment sequentially at least 1 day apart within each dose group.

One week after the last subject received the 25 IU/kg dose of the rFVIIIFc, approximately 6 unique subjects were recruited for the 65 IU/kg cohort. Each subject in the 65 IU/kg cohort received a single 65 IU/kg dose of ADVATE^{®} followed by a 4-day (96 hours) PK profiling then crossover and receive a 65 IU/kg single, open-label dose of rFVIIIFc for a 10-day (240 hours) profiling. If a bleeding episode occurred before the first injection of rFVIIIFc in any cohort, subject's pre-study FVIII product was used for treatment and an interval of at least 4 days had to pass before receiving the first injection of rFVIIIFc for the PK profile.

All subjects were followed for a 14-day (336 hours) and 28 day safety evaluation period after administration of rFVIIIFc 25 IU/kg or 65 IU/kg for safety. All subjects underwent pharmacokinetic sampling pre- and post-dosing along with blood samples for analysis of FVIII activity at designated time points.

The pharmacokinetic data for the Phase I/IIa clinical trial demonstrated the following results for FVIIIFc. FVIIIFc had about a 50% increase in systemic exposure (AUC_{INF}), about 50% reduction in clearance (Cl), and about 50-70% increase in elimination half-life and MRT compared to ADVATE^{®} (full length rFVIII). In addition, FVIIIFc showed increased C168, TBLP1, TBLP3, and TBLP5 values compared to ADVATE^{®}.
- AUC_{INF}: Area under the concentration-time curve from zero to infinity
- Beta HL: Elimination phase half-life; also referred to as t_{1/2β}
- C168: Estimated FVIIIFc activity above baseline at approximately 168 h after dose
- Cl: Clearance
- MRT: Mean residence time
- TBLP1: Model-predicted time after dose when FVIIIFc activity has declined to approximately 1 IU/dL above baseline
- TBLP3: Model-predicted time after dose when FVIIIFc activity has declined to approximately 3 IU/dL above baseline
- TBLP5: Model-predicted time after dose when FVIIIFc activity has declined to approximately 5 IU/dL above baseline

### Example 4

A recombinant B-domain-deleted factor VIII-Fc (rFVIIIFc) fusion protein has been created as an approach to extend the half-life of FVIII. The pharmacokinetics (PK) of rFVIIIFc were compared to rFVIII in hemophilia A mice. We found that the terminal half-life was twice as long for rFVIIIFc compared to rFVIII. In order to confirm that the underlying mechanism for the extension of half-life was due to the protection of rFVIIIFc by FcRn, the PK were evaluated in FcRn knockout and human FcRn transgenic mice. A single intravenous dose (125 IU/kg) was administered and the plasma concentration measured using a chromogenic activity assay. The Cmax was similar between rFVIIIFc and rFVIII (XYNTHA^{®}) in both mouse strains. However, while the half-life for rFVIIIFc was comparable to that of rFVIII in the FcRn knockout mice, the half-life for rFVIIIFc was extended to approximately twice longer than that for rFVIII in the hFcRn transgenic mice. These results confirm that FcRn mediates or is responsible for the prolonged half-life of rFVIIIFc compared to rFVIII. Since hemostasis in whole blood measured by rotation thromboelastometry (ROTEM^{®}) has been shown to correlate with the efficacy of coagulation factors in bleeding models of hemophilia mice as well as in clinical applications, we sought to evaluate the ex vivo efficacy of rFVIIIFc in the hemophilia A mice using ROTEM^{®}. Hemophilia A mice were administered a single intravenous dose of 50 IU/kg rFVIIIFc, XYNTHA^{®} (FVIII) or ADVATE^{®} (FVIII). At 5 minutes post dose, clot formation was similar with respect to clotting time (CT), clot formation time (CFT) and α-angle. However, rFVIIIFc showed significantly improved CT at 72 and 96 hr post dose, and CFT and α-angle were also improved at 96 hrs compared to both XYNTHA^{®} (FVIII) and ADVATE^{®} (FVIII), consistent with prolonged PK of rFVIIIFc. Therefore construction of an Fc fusion of FVIII produces a molecule with a defined mechanism of action that has an increased half-life and the potential to provide prolonged protection from bleeding.

### Example 5

This Example presents final analysis results for FVIII activity from 16 patients treated with 25 and 65 IU/kg FVIII products. See Example 3.

In this Example, rFVIIIFc is a recombinant fusion protein comprised of a single molecule of recombinant B-domain deleted human FVIII (BDD-rFVIII) fused to the dimeric Fc domain of the human IgG1, with no intervening linker sequence. This protein construct is also referred to herein as rFVIIIFc heterodimeric hybrid protein, FVIIIFc monomeric Fc fusion protein, FVIIIFc monomer hybrid, monomeric FVIIIIFc hybrid, and FVIIIFc monomer-dimer. See Example 1, Fig. 1, and Table 2A.

Preclinical studies with rFVIIIFc have shown an approximately 2-fold prolongation of the half-life of rFVIII activity compared to commercially available rFVIII products. The rationale for this study was to evaluate the safety and tolerability of a single dose of rFVIIIFc in frozen liquid formulation and provide data on the PK in severe hemophilia A subjects. For this study, 16 evaluable subjects were available for PK evaluation. Single administration of two doses of both rFVIIIFc and ADVATE^{®} at a nominal dose of 25 (n=6) and 65 IU/kg of body weight (n=10) were infused intravenously over approximately 10 minutes. Blood samples for plasma PK assessments were obtained before infusion, as well as up to 10 days after dosing. The PK of FVIII activity for both ADVATE^{®} and rFVIIIFc were characterized in this study using a model-dependent method.

### OBJECTIVES

The primary objective of this study was to assess the safety and tolerability of single administration of two doses of rFVIIIFc (25 and 65 IU/kg) in previously treated patients (PTPs) aged 12 and above with severe hemophilia A.

The secondary objectives were to determine the pharmacokinetics (PK) parameters determined by pharmacodynamic (PD) activity of FVIII over time after a single administration of 25 or 65 IU/kg of rFVIIIFc compared to ADVATE^{®} in one-stage clotting and chromogenic assays.

### Study Design (See Example 3)

Blood samples were collected for FVIII activity PK evaluations at the screening visit (within 28 days prior to dosing ADVATE^{®}); on Day 0 (injection of ADVATE^{®}) pre-injection and at 10 and 30 minutes and 1, 3, 6, and 9 hours post-injection; on Day 1 at 24 hours post-injection of ADVATE^{®}; on Day 2 at 48 hours post-injection of ADVATE^{®}; on Day 3 at 72 hours post-injection of ADVATE^{®}; and on Day 4 at 96 hours post-injection of high dose of ADVATE^{®} (Cohort B only).

Blood samples were collected for FVIII activity PK evaluations on the day of rFVIIIFc injection just prior to the administration of rFVIIIFc, at 10 and 30 minutes and 1, 3, 6, and 9 hours post-injection of rFVIIIFc; on Day 1 at 24 hours post-injection of rFVIIIFc; on Days 2 through 5 at 48, 72, 96, and 120 hours post-injection of rFVIIIFc; on Day 7 at 168 hours post-injection of rFVIIIFc; on Days 8, 9, and 10 at 192, 216, and 240 hours post-injection of high dose of rFVIIIFc (Cohort B only). FVIII activity was also measured at the final study visit (28 days post-injection of rFVIIIFc) at 672 hours post-injection of rFVIIIFc.

### Pharmacokinetic Modeling and Calculations

### Abbreviations

TBLP1 = Model-predicted time after dose when FVIII activity has declined to approximately 1 IU/dL above baseline.
TBLP3 = Model-predicted time after dose when FVIII activity has declined to approximately 3 IU/dL above baseline $KV_M = Cmax_M / Actual Dose \left(IU/kg\right)$ $KV_OB = Cmax_OB / Actual Dose \left(IU/kg\right)$ $IVR_M = 100 \times Cmax_M \times Plasma Volume \left(dL\right) / Total Dose in IU ;$ where plasma volume in mL = (23.7 × Ht in cm) + (9.0 × Wt in kg) - 1709. $IVR_OB = 100 \times Cmax_OB \times Plasma Volume \left(dL\right) / Total Dose in IU ;$ where plasma volume in mL = (23.7 × Ht in cm) + (9.0 × Wt in kg) - 1709.

### RESULTS

### Single-Dose Pharmacokinetics (One-Stage Assay)

Observed FVIII activity increased sharply after the short IV infusion of either ADVATE^{®} or rFVIIIFc, with mean (±SD) model-predicted Cmax values of 56.6 ± 4.74 and 121 ± 28.2 IU/dL for ADVATE^{®} and 55.6 ± 8.18 and 108 ± 16.9 IU/dL for rFVIIIFc for the 25 and 65 IU/kg dose groups, respectively. All ADVATE^{®}- and rFVIIIFc-treated patients had dose-related increases in FVIII activity. The observed increase in both Cmax and AUC_{INF} was slightly less than proportional to dose over the dose range evaluated.

After the end of the infusion, the decline of the observed FVIII activity exhibited monoexponential decay characteristics until the baseline level was reached. The rate of decline in FVIII activity was slower for rFVIIIFc than for ADVATE^{®} with mean (±SD) model-predicted elimination half-life values of 11.9 ± 2.98 and 10.4 ± 3.03 hr for ADVATE^{®} and 18.0 ± 3.88 and 18.4 ± 6.99 hr for rFVIIIFc for the 25 and 65 IU/kg dose groups, respectively. Elimination half-life values appeared to be dose-independent over the dose range evaluated for both FVIII products.

Total systemic FVIII exposure (assessed by AUC_{INF}) was ~ 48% and 61% greater following rFVIIIFc administration than ADVATE^{®} at 25 and 65 IU/kg dose levels, respectively. Mean (±SD) model-predicted AUC_{INF} values were 974 ± 259 and 1810 ± 606 hr*IU/dL for ADVATE^{®} and 1440 ± 316 and 2910 ± 1320 hr*IU/dL for rFVIIIFc for the 25 and 65 IU/kg dose groups, respectively.

Similar to elimination half-life, the MRT was prolonged for rFVIIIFc relative to ADVATE^{®}. Mean (±SD) model-predicted MRT values were 17.1 ± 4.29 and 14.9 ± 4.38 hr for ADVATE^{®} and 25.9 ± 5.60 and 26.5 ± 10.1 hr for rFVIIIFc for the 25 and 65 IU/kg dose groups, respectively. MRT values appeared to be dose-independent over the dose range evaluated for both FVIII products.

In addition, primary PK parameter values for CL and V were determined. CL values for rFVIIIFc only accounted for ~ 66% of those observed for ADVATE^{®} at equivalent doses. Mean (±SD) model-predicted CL values were 2.70 ± 0.729 and 4.08 ± 1.69 mL/hr/kg for ADVATE^{®} and 1.80 ± 0.409 and 2.69 ± 1.25 mL/hr/kg for rFVIIIFc for the 25 and 65 IU/kg dose groups, respectively. V values were comparable between ADVATE^{®} and rFVIIIFc with mean (±SD) model-predicted V values of 43.9 ± 4.27 and 56.1 ± 13.4 mL/kg for ADVATE^{®} and 45.3 ± 7.23 and 61.6 ± 10.6 mL/kg for rFVIIIFc for the 25 and 65 IU/kg dose groups, respectively. Slight increases in mean CL and V values were noted with increasing dose of ADVATE^{®} and rFVIIIFc; however, the increase in standard deviations at the 65 IU/kg dose coupled with limited dose levels confounded an assessment of the dose-dependency of these parameters. For example, the CV% geometric mean CL value for the rFVIIIFc treatment group increased from 23.0% (25 IU/kg) to 48.6% (65 IU/kg).

In addition to the primary PK parameters, secondary PK parameters (e.g. K-values, IVR, etc.) were determined to evaluate FVIII duration of effect. Evidence of PK difference was also observed with rFVIIIFc demonstrating increased TBLP1 and TBLP3 values compared to ADVATE^{®} at equivalent doses. IVR and K-values for ADVATE^{®} and rFVIIIFc appeared to be comparable. A slight increase in TBLP1 and TBLP3 values were observed with increasing dose of ADVATE^{®} and rFVIIIFc. In contrast, slight decreases in mean IVR and K-values were noted with increasing dose of ADVATE^{®} and rFVIIIFc. As previously indicated, an assessment of the dose dependency of these parameters is confounded by limited dose levels.

Mean (±SD) observed TBLP1 were 2.88 ± 0.733 and 2.93 ± 0.848 IU/dL per IU/kg for ADVATE^{®} and 4.28 ± 0.873 and 5.16 ± 2.02 IU/dL per IU/kg for rFVIIIFc for the 25 and 65 IU/kg dose groups, respectively. Mean (±SD) observed TBLP3 were 2.06 ± 0.527 and 2.26 ± 0.666 IU/dL per IU/kg for ADVATE^{®} and 3.09 ± 0.623 and 3.93 ± 1.59 IU/dL per IU/kg for rFVIIIFc for the 25 and 65 IU/kg dose groups, respectively.

Mean IVR and K-values calculated using observed Cmax values (subtracted with baseline and residual drug within the model) were generally greater than values determined using model-predicted Cmax values; consistent with slight underestimation of the observed peak activity using the one-compartment model. Mean (±SD) observed K-values were 2.57 ± 0.198 and 2.13 ± 0.598 IU/dL per IU/kg for ADVATE^{®} and 2.46 ± 0.330 and 1.85 ± 0.332 IU/dL per IU/kg for rFVIIIFc for the 25 and 65 IU/kg dose groups, respectively. Mean (±SD) observed IVR values were 94.1 ± 15.6 and 85.8 ± 16.5 % for ADVATE^{®} and 89.5 ± 11.9 and 74.8 ± 6.72 % for rFVIIIFc for the 25 and 65 IU/kg dose groups, respectively.

### Single-Dose Pharmacokinetics (Chromogenic Assay)

Observed FVIII activity increased sharply after the short IV infusion of either ADVATE^{®} or rFVIIIFc, with mean (±SD) model-predicted Cmax values of 70.2 ± 9.60 and 157 ± 38.6 IU/dL for ADVATE^{®} and 70.3 ± 10.0 and 158 ± 34.7 IU/dL for rFVIIIFc for the 25 and 65 IU/kg dose groups, respectively.

All ADVATE^{®}- and rFVIIIFc-treated patients had dose-related increases in FVIII activity. The observed increase in both Cmax and AUC_{INF} was slightly less than proportional to dose over the dose range evaluated.

After the end of the infusion, the decline of the observed FVIII activity exhibited monoexponential decay characteristics until the baseline level was reached. The rate of decline in FVIII activity was slower for rFVIIIFc than for ADVATE^{®} with mean (±SD) model-predicted elimination half-life values of 10.7 ± 1.98 and 10.3 ± 3.27 hr for ADVATE^{®} and 16.2 ± 2.92 and 19.0 ± 7.94 hr for rFVIIIFc for the 25 and 65 IU/kg dose groups, respectively. Elimination half-life values appeared to be dose-independent over the dose range evaluated for both FVIII products.

Total systemic FVIII exposure (assessed by AUC_{INF}) was ~ 53% and 84% greater following rFVIIIFc administration than ADVATE^{®} at 25 and 65 IU/kg dose levels, respectively. Mean (±SD) model-predicted AUC_{INF} values were 1080 ± 236 and 2320 ± 784 hr*IU/dL for ADVATE^{®} and 1650 ± 408 and 4280 ± 1860 hr*IU/dL for rFVIIIFc for the 25 and 65 IU/kg dose groups, respectively.

Similar to elimination half-life, the MRT was prolonged for rFVIIIFc relative to ADVATE^{®}. Mean (±SD) model-predicted MRT values were 15.3 ± 2.86 and 14.8 ± 4.72 hr for ADVATE^{®} and 23.4 ± 4.22 and 27.3 ± 11.4 hr for rFVIIIFc for the 25 and 65 IU/kg dose groups, respectively. MRT values appeared to be dose-independent over the dose range evaluated for both FVIII products.

In addition, primary PK parameter values for CL and V were determined. CL values for rFVIIIFc only accounted for ~ 58-66% of those observed for ADVATE^{®} at equivalent doses. Mean (±SD) model-predicted CL values were 2.39 ± 0.527 and 3.21 ± 1.40 mL/hr/kg for ADVATE^{®} and 1.57 ± 0.349 and 1.86 ± 0.970 mL/hr/kg for rFVIIIFc for the 25 and 65 IU/kg dose groups, respectively. V values were comparable between ADVATE^{®} and rFVIIIFc with mean (±SD) model-predicted V values of 35.8 ± 5.52 and 43.6 ± 11.2 mL/kg for ADVATE^{®} and 35.9 ± 6.65 and 42.7 ± 8.91 mL/kg for rFVIIIFc for the 25 and 65 IU/kg dose groups, respectively. Increases in mean CL and V values were noted with increasing dose of ADVATE^{®} and rFVIIIFc; however, the increase in standard deviations at 65 IU/kg coupled with limited dose levels confounded an assessment of the dose-dependency of these parameters.

In addition to the primary PK parameters, secondary PK parameters (e.g. K-values, IVR, etc.) were determined to evaluate FVIII duration of effect. Evidence of PK difference was also observed with rFVIIIFc demonstrating increased TBLP1 and TBLP3 values compared to ADVATE^{®} at equivalent doses. IVR and K-values for ADVATE^{®} and rFVIIIFc appeared to be comparable.

A slight increase in TBLP1 and TBLP3 values were observed with increasing dose of ADVATE^{®} and rFVIIIFc. In contrast, slight decreases in mean IVR and K-values were noted with increasing dose of ADVATE^{®} and rFVIIIFc. As previously indicated, an assessment of the dose dependency of these parameters is confounded by limited dose levels.

Mean (±SD) observed TBLP1 were 2.70 ± 0.511 and 3.09 ± 0.978 IU/dL per IU/kg for ADVATE^{®} and 4.06 ± 0.798 and 5.66 ± 2.38 IU/dL per IU/kg for rFVIIIFc for the 25 and 65 IU/kg dose groups, respectively. Mean (±SD) observed TBLP3 were 1.98 ± 0.377 and 2.39 ± 0.718 IU/dL per IU/kg for ADVATE^{®} and 3.04 ± 0.598 and 4.44 ± 1.84 IU/dL per IU/kg for rFVIIIFc for the 25 and 65 IU/kg dose groups, respectively.

Mean IVR and K-values calculated using observed Cmax values (subtracted with baseline and residual drug within the model) were generally greater than values determined using model-predicted Cmax values; consistent with slight underestimation of the observed peak activity using the one-compartment model. Mean (±SD) observed K-values were 3.08 ± 0.429 and 2.85 ± 0.721 IU/dL per IU/kg for ADVATE^{®} and 3.12 ± 0.451 and 2.92 ± 0.985 IU/dL per IU/kg for rFVIIIFc for the 25 and 65 IU/kg dose groups, respectively. Mean (±SD) observed IVR values were 112 ± 14.5 and 116 ± 26.9 % for ADVATE^{®} and 113 ± 16.3 and 117 ± 33.6 % for rFVIIIFc for the 25 and 65 IU/kg dose groups, respectively.

### CONCLUSIONS

All ADVATE^{®}- and rFVIIIFc-treated patients had comparable dose-related increases in Cmax and AUCINF over the dose range evaluated. Peak plasma levels of ADVATE^{®} and rFVIIIFc activity were generally observed within the first hour after the end of the infusion and remained detectable for several days after dosing. After the end of infusion, the decline in baseline corrected FVIII activity exhibited monoexponential decay until the baseline was reached for both products. Parameter values for elimination half-life and MRT appeared to be dose-independent over the dose range evaluated for both FVIII products. Slight increases in mean CL and V values were noted with increasing dose of ADVATE^{®} and rFVIIIFc; however, increased intersubject variability at the 65 IU/kg coupled with limited dose levels confounded an assessment of the dose-dependency of these parameters.

Comparison of rFVIIIFc and ADVATE^{®} activity PK revealed an approximate 48-61% (One-Stage Assay) or 53-84% (Chromogenic Assay) increase in systemic exposure, approximate 30-40% reduction in clearance, and an approximate 50-80% increase in both elimination half-life and MRT for rFVIIIFc relative to ADVATE^{®} at comparable doses. Evidence of PK difference was also observed with rFVIIIFc demonstrating increased TBLP1 and TBLP3 values compared to ADVATE^{®} at equivalent doses. IVR and K-values for ADVATE^{®} and rFVIIIFc appeared to be comparable.

The PK parameters obtained from the Chromogenic Assay results generally agreed with those from the One-Stage Assay, except that the Chomogenic Assay yielded a higher estimation of exposure parameters (e.g., Cmax, AUCINF, etc.).

With the observed improvements in PK, rFVIIIFc may provide a prolonged protection from bleeding, allowing less frequent injections for individuals with Hemophilia A.

### Example 6

On the basis of the interim PK analysis from the first in-human study of rFVIII:Fc (Example 3), the A-LONG study was designed. A-LONG is an open label, multi-center evaluation of the safety, pharmacokinetics, and efficacy of recombinant Factor VIII Fc fusion (FVIII:Fc) in the prevention and treatment of bleeding in previously treated subjects with severe hemophilia A (defined as <1 IU/dL [<1%] endogenous FVIII).

Approximately 106 subjects will be enrolled into one of three regimens: a tailored prophylaxis regimen (arm 1), a weekly dosing regimen (arm 2), and an on-demand regimen (arm 3).

### Arm 1: Tailored Prophylaxis Regimen

Arm 1 will include an overall group and a PK subgroup. Approximately 66 subjects will be enrolled. The initial regimen will be twice weekly at 25 IU/kg on the first day, followed by 50 IU/kg on the fourth day of the week. Subjects will administer rFVIIIFc on this weekly prophylaxis regimen until PK results for rFVIIIFc are available. Based on these results, a tailored prophylaxis regimen will be established for each individual, in which the dose and interval will be determined to maintain a trough level of 1-3% FVIII activity. Each subject will then administer his individually tailored prophylaxis regimen throughout the study.

Subjects will be monitored throughout the study and ongoing dose and interval adjustments will be made. Adjustments will only be made when a subject experiences unacceptable bleeding episodes defined as ≥2 spontaneous bleeding episodes over a rolling two-month period. In this case, adjustment will target trough levels of 3-5%.

### Arm 2: Weekly Dosing Regimen

Approximately 20 subjects will be enrolled/randomized and undergo abbreviated rFVIIIFc PK profiling as follows: Washout of at least 96 hours; a single dose of rFVIIIFc 65 IU/kg; Abbreviated sampling beginning on rFVIIIFc Day 0, including pre-injection and 10 (±2) minutes, 3 hours (± 15 minutes), 72 (± 2) hours [Day 3], and 96 (± 2) hours [Day 4] from the start of injection. Following the abbreviated PK profiling, subjects will then administer a fixed dose of 65 IU/kg rFVIIIFc every 7 days at least for 28 weeks and up to 52 weeks.

### Arm 3: On-demand Regimen

A minimum of 10 major surgeries in at least 5 subjects will be evaluated in the study. Major surgery is defined as any surgical procedure (elective or emergent) that involves general anesthesia and/or respiratory assistance in which a major body cavity is penetrated and exposed, or for which a substantial impairment of physical or physiological functions is produced (e.g., laparotomy, thoracotomy, craniotomy, joint replacement, and limb amputation).

For prophylaxis during surgery, subjects will be treated with 20 to 50 IU/kg rFVIIIFc every 12 to 24 hours. Prior to surgery, the physician will review the subject's rFVIIIFc PK profile and assess the dose regimen of Factor VIII replacement generally required for the type of planned surgery and the clinical status of the subject. Recommendation for the appropriate dosing of rFVIIIFc in the surgical treatment period, including any rehabilitation time, will take these factors into consideration.

The primary objectives of this study are (a) to evaluate the safety and tolerability of rFVIIIFc administered as prophylaxis, on-demand, and surgical treatment regimens; and (b) to evaluate the efficacy of rFVIIIFc administered as prophylaxis, on-demand, and surgical treatment regimens. The secondary objectives of this study are (a) to characterize the PK profile of rFVIIIFc and compare the PK of FVIIIFc with the currently marketed product, ADVATE^{®}; (b) to evaluate individual responses with FVIIIFc; and (c) to evaluate FVIIIFc consumption.

### Primary Objectives

- To evaluate safety and tolerability of rFVIIIFc administered as prophylaxis, weekly, on-demand, and surgical treatment regimens
- To evaluate the efficacy of rFVIIIFc administered as tailored prophylaxis, on-demand, and surgical treatment regimens

### Secondary Objectives

- To characterize the PK profile of rFVIIIFc and compare the PK of rFVIIIFc with the currently marketed product, ADVATE^{®}
- To evaluate individual responses with rFVIIIFc
- To characterize the range of dose and schedules required to adequately prevent bleeding in a prophylaxis regimen; maintain homeostasis in a surgical setting; or to treat bleeding episodes in an on-demand, weekly treatment, or prophylaxis setting
- To evaluate rFVIIIFc consumption (e.g., total annualized rFVIIIFc consumption per subject)

### Example 7

### Clinical ROTEM^{®} Assessment

In the study in Example 7, in addition to the measurement of plasma FVIII activity by one-stage activated partial thromboplastin time (aPTT) assay, whole blood rotational thromboelastometry (ROTEM^{®}) has also been explored to assess the improvement in global hemostasis by rFVIIIFc and ADVATE^{®} in 2 subjects, specifically, 1 in the low dose cohort and 1 in the high dose cohort.

rFVIIIFc and ADVATE^{®} appear to be comparably active in clot formation when spiked into subjects' blood prior to rFVIIIFc treatment. The clotting time (CT) was linear with respect to the dose of rFVIIIFc and ADVATE^{®} in the range of approximately 1% of 100% of normal, and the dose response was comparable between rFVIIIFc and ADVATE^{®} in the same subject.

Following dosing with ADVATE^{®} and subsequently rFVIIIFc, citrated whole blood was sampled at various time points and the clot formation following recalcification was monitored by ROTEM^{®}. Despite the variable baseline CT due to residue FVIII levels prior to ADVATE^{®} or rFVIIIFc dosing, both products effectively corrected the CT to comparable levels 30 minutes post-injection. In addition, the improvement in CT was better sustained at and after 3 hours post-injection of 25 IU/kg of rFVIIIFc relative to ADVATE^{®} in the subject dosed at this low dose. However, the differential improvement of rFVIIIFc versus ADVATE^{®} was much less appreciable at the 65 IU/kg dose.

### Example 8

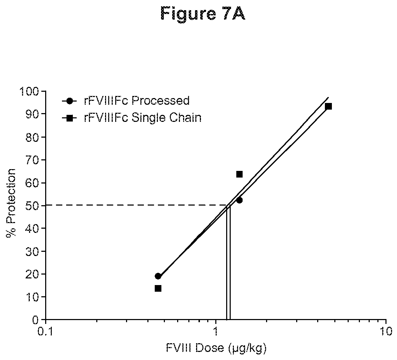

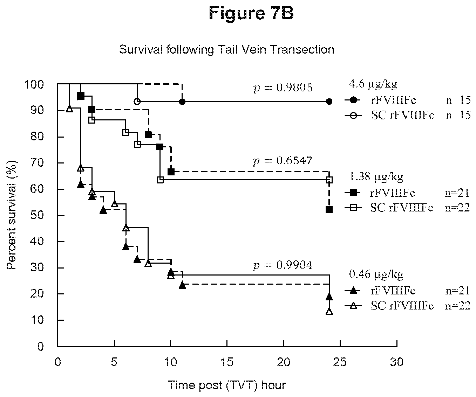

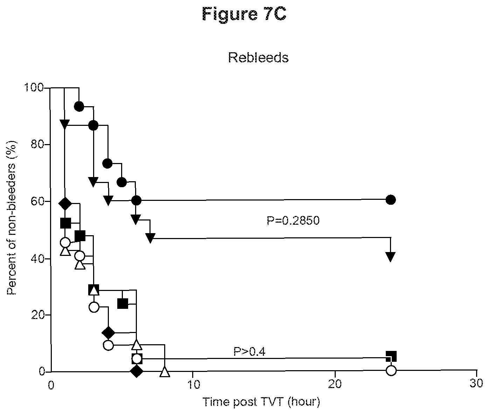

HemA mice were used for tail clip studies. The mice were first anesthetized and then injected with 4.6 µg/kg, 1.38 µg/kg, or 0.46 µg/kg of either processed rFVIIIFc (Drug Substance, which contain about 75%-85% processed rFVIIIFc) and purified single chain rFVIIIFc. After the injection, the tail was cut from the tip and immediately placed into a tube to collect blood. Percentage of protection on survival was measured for rFVIIIFc processed (drug substance) and single chain FVIIIFc as shown in Table 7 and Figure 7.

**TABLE 7. In Vivo Efficacy of rFVIII:Fc DS and Single chain rFVIIIFc**

| Dose (µg/kg) | | 4.6 | 1.38 | 0.46 |
|---|---|---|---|---|
| % of Protection on Survival | FVIIIFc DS | 93 | 52 | 19 |
| | Single chain rFVIIIFc | 93 | 64 | 14 |

As shown in Table 7 and Figure 7, the protection on survival by single chain rFVIIIFc is comparable to processed rFVIIIFc (DS).

### Clotting activity by in vitro ROTEM

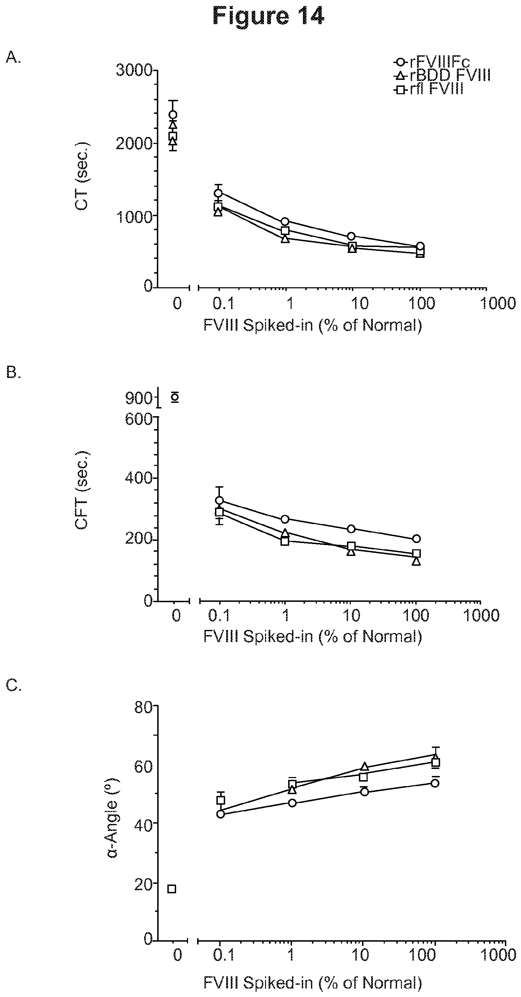

The clotting potency of rFVIIIFc was further explored in whole blood Rotational Thromboelastometry (ROTEM) over a range of concentrations, and compared to both rBDD FVIII (Xyntha) and recombinant full length FVIII (rflFVIII; Advate). For in vitro ROTEM, rFVIII proteins were spiked in triplicate into citrated pooled blood collected from the vena cava of 5-6 male HemA mice to the final concentration of 0, 0.1, 1, 10, and 100% of normal plasma FVIII level. The clot was initiated by the addition of CaCl₂ (NATEM) and clotting time (CT), clot formation time (CFT), alpha-angle and maximum clot firmness (MCF) were recorded on the ROTEM system (Pentapharm GmbH, Munich, Germany). The clotting time (CT), clot formation time (CFT), and alpha angle for the three proteins spiked in HemA mouse blood at escalating doses from 0.1 - 100 % of normal FVIII levels are shown in Figure 14. In the wide range of 0.1 to 100% of normal, the CT and CFT are comparable among rFVIIIFc, rBDD FVIII and rflFVIII. The alpha angle is only significantly different (p<0.05) between rFVIIIFc and rBDD FVIII at 10%.

### Clotting activity by ex vivo ROTEM

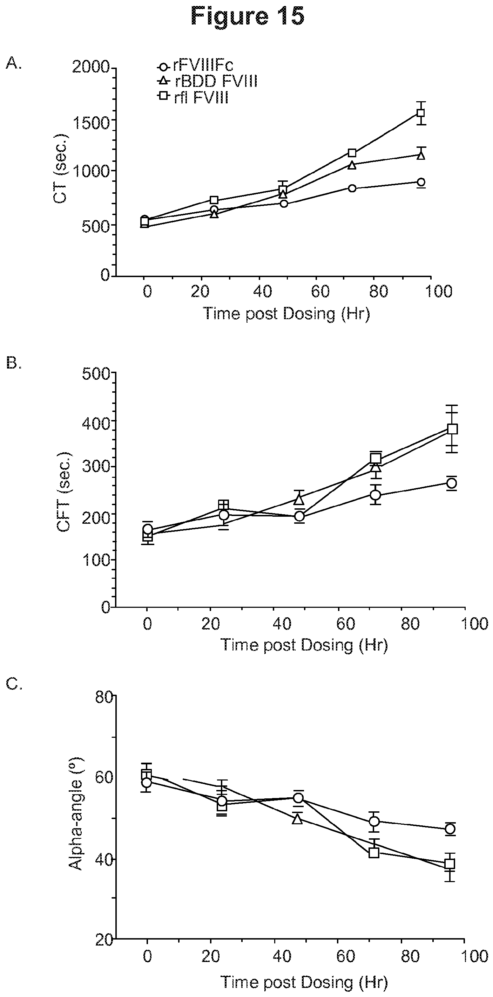

The pharmacodynamics of rFVIIIFc, as measured by ROTEM, was compared to rBDD FVIII and rflFVIII after a single intravenous injection into Hemophilia A mice. For ex vivo ROTEM, male HemA mice were injected intravenously with a single dose of 50 IU/kg rFVIIIFc, ADVATE^{®}, or XYNTHA^{®}, and 5 mice sacrificed at each time point (5 minutes, 24, 48, 72 and 96 hours post dosing). Individual citrated whole blood collected from the vena cava was immediately analyzed by NATEM on the ROTEM system and parameters measured as above. The CT, CFT, and alpha angle were determined for samples taken from 5 min to 96 hours after dosing, and shown in Figure 15. At 5 min, all are comparably effective resulting in similar CT, CFT and alpha angle (Figure 15A - C). However, rFVIIIFc demonstrated a significantly improved (p<0.05) CT at 72 and 96 hrs, CFT and alpha angle at 96 hrs (Figures 15A - C) relative to rBDD FVIII and rflFVIII .

### Example 9

Recombinant factor VIIIFc (rFVIIIFc) is comprised of a B domain deleted (BDD) rFVIII protein genetically fused to the Fc domain of human immunoglobulin G1 (IgG1). Prior to secretion from HEK 293 cells, most of the rFVIIIFc is processed into a FVIII heavy chain (HC) and light chain (LC+Fc). In circulation, rFVIIIFc is complexed with von Willebrand factor (VWF) and released upon activation in a manner that is indistinguishable from native FVIII. Spontaneous dissociation of the HC and LC is thought to contribute to the loss of FVIII activity in plasma and during storage of FVIII drug products. Here we describe a single chain non-processed isoform of rFVIIIFc (SC rFVIIIFc), which may provide superior manufacturability and enhanced stability compared to native FVIII.

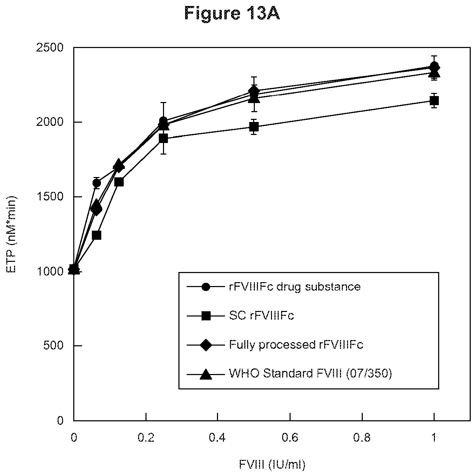

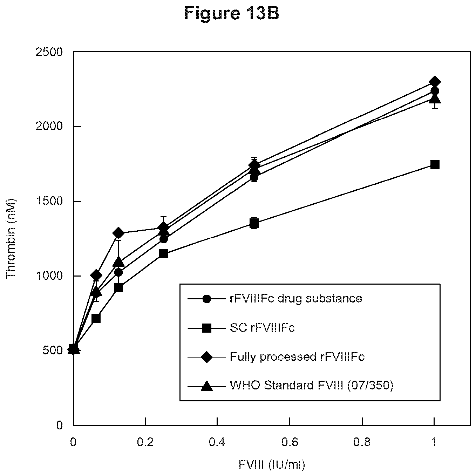

SC rFVIIIFc was purified from rFVIIIFc, which contains a fraction of the non-processed isoform. Compared to rFVIIIFc, SC rFVIIIFc showed equivalent chromogenic activity but approximately 60% reduced activity by the one stage (aPTT) assay, (Table 3A-B). Thrombin generation assay (TGA) was performed using calibrated automated thrombogram (from Thrombinoscope^{®}). In a thrombin generation assay (TGA), SC rFVIIIFc also showed a reduced thrombin potential (Figure 13A), and peak thrombin (Figure 13B) compared to rFVIIIFc. However, as shown in Table 3B, full activity of SC rFVIIIFc by aPTT was observed in the absence of vWF, suggesting release from vWF may be delayed due to covalent linkage of the a3 acidic region to the HC after Arg 1680 cleavage in SC rFVIIIFc, in contrast to a3 release and dissociation from fully processed FVIII. Delayed dissociation from vWF may explain the reduced activity observed in the aPTT assay and TGA, while full activity was observed in the two-stage chromogenic assay. A reduced rate of activation in the presence of vWF was confirmed in a modified chromogenic substrate assay with limiting thrombin as FVIII activator.

In vivo function of SC rFVIIIFc was assessed in the HemA mouse tail vein transection (TVT) model. HemA male mice were treated with indicated doses of either rFVIIIFc drug product or SC rFVIIIFc 48 hours prior to TVT. Tail re-bleeding and survival were monitored hourly up to 12 hours post TVT with final observation performed at 24-hour post TVT. SC rFVIIIFc and the rFVIIIFc demonstrated equivalent in vivo efficacy in this model, with an ED50 of 1.17 µg/kg and 1.23 µg /kg respectively when TVT was performed at 48 hours post infusion (Figure 7(A)). Comparable 24 hour post TVT survival curves (p ≥ 0.65) (Figure 7(B)) and re-bleed rates (Figure 7(C)) in HemA mice were observed for the SC rFVIIIFc and rFVIIIFc at each tested dose level, indicating that SC rFVIIIFc was equally effective as rFVIIIFc despite its lower apparent aPTT activity. The delayed in vitro activation of SC rFVIIIFc in the presence of vWF therefore appears to have no significant impact on its in vivo efficacy. Thus, SC rFVIIIFc represents a novel and efficacious isoform of rFVIIIFc with potential clinical applications. Further studies will be required to demonstrate enhanced product stability in the context of this Fc fusion protein.

### Example 10

Current factor VIII (FVIII) products display a half-life (t_{1/2}) of approximately 8-12 hours, requiring frequent intravenous injections for prophylaxis and treatment of hemophilia A patients. rFVIIIFc is a recombinant fusion protein composed of a single molecule of FVIII covalently linked to the Fc domain of human IgG₁ to extend circulating rFVIII half-life. This first-in-human study in previously-treated male subjects with severe hemophilia A investigated safety and pharmacokinetics of rFVIIIFc. Sixteen subjects received a single dose of ADVATE^{®} at 25 or 65 IU/kg followed by an equal dose of rFVIIIFc. Most adverse events were unrelated to study drug. None of the study subjects developed anti-FVIIIFc antibodies or inhibitors. Across dose levels, as compared with ADVATE^{®}, rFVIIIFc showed 1.54 to 1.71-fold longer elimination t_{1/2} and mean residence time, 1.49 to 1.56-fold lower clearance, and 1.48 to 1.56-fold higher total systemic exposure. ADVATE^{®} and rFVIIIFc had comparable dose-dependent peak plasma concentrations and recoveries. Time to 1% FVIII activity above baseline was approximately 1.53 to 1.68-fold longer than ADVATE^{®} across dose levels. Thus, rFVIIIFc may offer a viable therapeutic approach to achieve prolonged hemostatic protection and less frequent dosing in patients with hemophilia A.

Hemophilia A is an inherited bleeding disorder that results in frequent spontaneous and traumatic bleeding into the joints and soft tissues. Mannucci PM, Tuddenham EGD, N Engl J Med, 344:1773-1779 (2001). When inadequately treated, this bleeding leads to chronic arthropathy, disability, and increased risk of death. Soucie JM et al., Blood. 96(2):437-442 (2000).

Plasma-derived FVIII (pdFVIII) and recombinant human FVIII (rFVIII) products are utilized for treatment (on-demand therapy) and prevention (prophylaxis therapy) of bleeding episodes. rFVIII was developed to reduce the risk of blood-borne pathogen transmission following the widespread contamination of plasma products with HIV and hepatitis viruses, and to secure an adequate supply of FVIII product. However, hemostatic protection with current FVIII products is temporally limited due to a short half-life (t_{1/2}) of approximately 8-12 hours, requiring prophylactic injections three times per week or every other day for most patients in order to maintain FVIII levels above 1%, a level that has been established as protective against most spontaneous bleeding episodes. Manco-Johnson et al., New Engl J Med. 357(6):535-44 (2007).

Many studies have shown that, even at high doses, on-demand therapy is not effective in preventing arthropathy. Aledort L. et al., J Intern Med. 236:391-399 (1994); Petrini P. et al., Am J Pediatr Hematol Oncol. 13:280-287 (1991). The benefits of prophylactic therapy have been demonstrated in numerous clinical studies ^{4, 6-15} and Manco-Johnson *et al., supra,* established that children started on primary prophylaxis after their first joint bleed had significantly fewer bleeds and less joint damage than children treated on-demand.

Compared to on-demand treatment, prophylactic therapy also decreases disability, hospitalization rate, and time lost from school or work; ^{6,16} and improves quality of life for patients and their families.¹⁷ However, prophylactic therapy often requires use of central venous access devices in children, and their attendant risks of infection, sepsis, and thrombosis. In addition, despite the benefits, acceptance of and compliance with prophylaxis decreases with age, in part because of inconvenience and invasiveness.^{18,19} Thus, a rFVIII product with a prolonged plasma t_{1/2} would potentially be of benefit. Lillicrap D., Current Opinion in Hematology 17:393-397 (2010).

rFVIIIFc is a recombinant fusion protein composed of a single molecule of B-domain deleted rFVIII covalently linked to the human IgG₁ Fc domain. Potential advantages of Fc-fusion proteins include better tolerability and prolonged hemostatic protection, and the Fc domain represents a natural molecule with no known inherent toxicity.^{21,22} Attachment to the IgG₁ Fc domain permits binding to the neonatal Fc receptor (FcRn), which is expressed in many cell types, including endothelial cells. FcRn expression remains stable throughout life and is responsible for protecting IgG₁ and Fc-fusion proteins from lysosomal degradation, thus prolonging the t_{1/2} of the protein.^{21,23} Numerous proteins within the circulation are internalized into the cells lining the vasculature via nonspecific pinocytosis and are trafficked to endosomal and lysosomal degradation pathways.

Fc proteins interact with FcRn, resident within endosomes. Endosomes containing FcRn direct the Fc fusion proteins back to the plasma membrane, releasing them into circulation in a pH-dependent manner,²⁴ thereby avoiding lysosomal degradation. This recycling approach has been used successfully to extend the t_{1/2} of therapeutic biologics; a number of Fc fusion-based drugs have been approved for clinical use (eg etanercept, romiplostim) and others are in development.^{25,26}

Preclinical data for rFVIIIFc indicate that FVIII can be rescued from degradation by a natural protective pathway mediated by FcRn, thus extending t_{1/2}. In Hemophilia A mice and dogs, terminal plasma t_{1/2} for rFVIIIFc was approximately 2 times longer than with rFVIII.^{27,28} Based on these data, we conducted a first-in-human clinical study to investigate the safety and PK of a long-lasting rFVIIIFc fusion protein in subjects with hemophilia A.

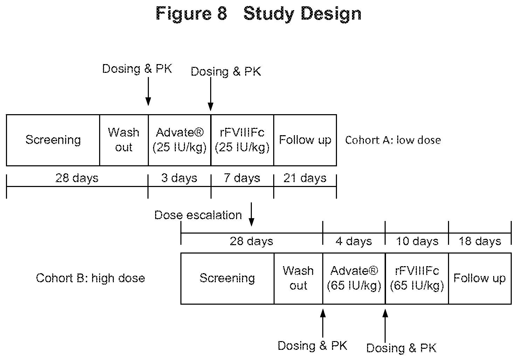

Study Design: This open-label, dose-escalation, multicenter Phase 1/2a study in previously treated patients with severe hemophilia A investigated the safety of rFVIIIFc and its pharmacokinetics (PK) compared with ADVATE^{®} (antihemophilic factor [recombinant], plasma/albumin-free method, octocog alfa, Baxter Healthcare). This study was performed in accordance with the US CFR and ICH Guidelines on Good Clinical Practices. Prior to any testing, approval from participating Institutional Review Boards and written informed consents from all subjects were obtained. The study design was sequential; a single dose of ADVATE^{®} was administered at 25 or 65 IU/kg followed by an equal dose of rFVIIIFc (FIG. 8). Both drugs were injected intravenously over approximately 10 minutes. The two dose levels were expected to bracket the typical therapeutic dose ranges. Subjects were followed for 28 days after receiving rFVIIIFc for safety analyses, including testing for anti-FVIII antibodies and inhibitors at 14 and 28 days post-injection. Plasma FVIII activity was measured in subjects before injection, 10 and 30 minutes, 1, 3, 6, 9, 24, 48, 72, 96, 120, and 168 hours (7 days) after rFVIIIFc injection, with additional samples at 192, 216, and 240 hours (10 days) for subjects dosed at 65 IU/kg of rFVIIIFc. Plasma FVIII activity was measured at the same time points after ADVATE^{®} treatment, through 72 hours for the 25 IU/kg group and 96 hours for the 65 IU/kg group.

Subjects: Male subjects were at least 12 years of age with severe hemophilia A (defined as FVIII activity level < 1%) and had at least 100 documented prior exposure days to FVIII concentrates (pdFVIII or rFVIII). Subjects with known hypersensitivity to mouse or hamster protein, history of inhibitor or detectable inhibitor titer at screening, or who were taking any medications that could affect hemostasis or systemic immunosuppressive drugs, or who experienced an active bacterial or viral infection (other than hepatitis or HIV) within 30 days of screening were excluded. Subject's genotype was recorded at study entry, when known.

Treatment Product: The human rFVIIIFc and Fc transgenes were stably transfected into HEK293 cells and the cell line was extensively tested for stability, sterility, and viral contamination to ensure safety. The purified drug product is composed of a monomeric B-domain-deleted FVIII covalently linked through its carboxy-terminus to the N-terminus of an Fc monomer, which forms a disulfide bond with a second Fc monomer during synthesis and secretion from the cells. rFVIIIFc was purified by chromatography and nanofiltration, and was fully active in one-stage and chromogenic clotting assays relative to commercially available rFVIII preparations. It was supplied as a frozen liquid containing 1000 IU per 2 mL of solution and formulated with L-histidine (pH 7), sodium chloride, calcium chloride, sucrose, mannitol, and Polysorbate 20. For injection, the product was diluted with saline solution (0.9% NaCl).

Outcome Measures: The primary objective of the study was safety, evaluated through physical examination, reporting of treatment-emergent adverse events (AEs), development of antibodies, and laboratory monitoring over time. The secondary objectives included parameters derived from PK analyses. Laboratory assessments included prothrombin time, activated partial thromboplastin time (aPTT), international normalized ratio, levels of D-dimer, von Willebrand factor (vWF) antigen, standard hematology and blood chemistry tests, and urinalysis.

FVIII activity was measured by the one-stage clotting (aPTT) assay on a Siemens BCS-XP analyzer using commercial reagents (Dade Actin FSL) with calibration against a normal reference plasma (Precision Biologics CRYOcheck^{™}) traceable to the World Health Organization (WHO) 5^{th} International Standard (IS) for human plasma. In addition to the aPTT assay, FVIII activity was measured by a chromogenic substrate assay²⁹ using a commercially available kit (Aniara BIOPHEN FVIII:C) that complies with European Pharmacopoeia recommendations. The chromogenic assay was calibrated against normal human reference plasma (Instrumentation Laboratories ORKE45), which also had a potency assigned against the WHO 5th IS human plasma standard.

The lower limit of quantification (LLOQ) for the one-stage and chromogenic assays was 0.5 IU/dL and 0.4 IU/dL, respectively. FVIII inhibitors were measured by the -*Nijmegen-modified Bethesda assay and less than 0.6 BU/mL was considered negative. Anti-rFVIIIFc antibodies were assessed using a specific bridging electrochemiluminescent immunoassay which uses biotin and sulfo-tagged rFVIIIFc. Assay sensitivity was determined to be 89 ng/mL using an anti-human FVIII monoclonal antibody as a surrogate control. Exploratory whole blood rotation thromboelastometry (ROTEM^{®}) was performed in two subjects, one from each dose level, at various time points to assess the improvement in global hemostasis following injection with ADVATE^{®} and rFVIIIFc.

Pharmacokinetic Analyses: A user-defined one-compartment disposition model, which automatically estimates the endogenous FVIII level and subsequent residual decay, was utilized in WinNonLin for analysis of the individual subject plasma FVIII activity-versus-time data following a single administration of ADVATE^{®} or rFVIIIFc. Actual sampling times, doses, and duration of injection were used for calculations of parameters including maximum activity (Cmax), t_{1/2}, clearance (CL), volume of distribution at steady-state (Vₛₛ), area under the curve (time zero extrapolated to infinity [AUC_{INF}]), mean residence time (MRT), and incremental recovery.

Monte Carlo Simulation of rFVIIIFc Activity-Versus-Time Profile - To construct FVIII activity-time profiles following dosing regimens of 25 IU/kg or 65 IU/kg, a Monte Carlo simulation was conducted using the population PK model of ADVATE^{®} and rFVIIIFc. The mean estimates of model parameters (CL, volume of distribution) in the tested population, the inter-individual variance, and the residual variability were estimated based on the one-stage (aPTT) clotting assay activity data of ADVATE^{®} and rFVIIIFc from 16 subjects in this Phase 1/2a study. Five hundred subjects were simulated with 15 sampling points for each subject for each dosing regimen. The percentage of the population with FVIII activity above or equal to 1% and 3% at different times following different dosing regimens of ADVATE^{®} or rFVIIIFc was estimated.

Statistical Analyses - Selected PK parameters for rFVIIIFc and ADVATE^{®} were compared using an analysis of variance model. PK parameters were log-transformed for these analyses and estimated means, mean differences, and confidence intervals on the log-scale were transformed to obtain estimates for geometric means, geometric mean ratios (GMR), and confidence intervals, respectively, on the original scale. The GMR is the geometric mean of the intra-subject ratio of the rFVIIIFc PK parameter value to the ADVATE^{®} PK parameter value.

### Results

Subject Disposition- Nineteen subjects were enrolled in the study; 16 underwent PK evaluation for both ADVATE^{®} and rFVIIIFc. One subject self-administered his previous product prior to completing the wash-out period following the dose with ADVATE^{®} and was thus excluded from the PK analysis, but was included in the safety analysis. Three subjects were discontinued from the study before receiving either study drug: one voluntarily withdrew; a second was withdrawn by the Investigator for non-compliance; and one was withdrawn at the Sponsor's request due to completion of study enrollment. Of the subjects dosed, six subjects received 25 IU/kg and 10 subjects received 65 IU/kg of both ADVATE^{®} and rFVIIIFc. Mean age was 40.3 years (23 to 61 years). Genotypic identification was collected for seven subjects; inversion of intron 22 was reported in six subjects; and a frame-shift defect was reported in one subject. The genotype was unknown for nine subjects. Thirteen subjects had hepatitis C antibodies, four of whom were also positive for HIV.

Safety - Forty-four treatment-emergent AEs were reported by 11 (69%) subjects during the treatment and follow-up periods. This included the day of dosing with Advate or rFVIIIFc through a 28-day post-dosing observation period. The majority of events were considered mild and none led to withdrawal from the study. One event, dysgeusia, occurred transiently in one subject while receiving a 65 IU/kg dose of rFVIIIFc and was considered related to rFVIIIFc.

One subject experienced an anxiety attack after receiving 65 IU/kg of rFVIIIFc which resulted in 21 AEs, 19 of which were graded as mild, and two of which (headache and photophobia) were rated as moderate. Neither was deemed related to rFVIIIFc by the Investigator.

No serious bleeding episodes were reported. No evidence of allergic reactions to injection was detected. All plasma samples tested negative for FVIII inhibitors and anti-rFVIIIFc antibodies. No signs of injection site reactions were observed. No clinically meaningful changes in abnormal laboratory values were reported.

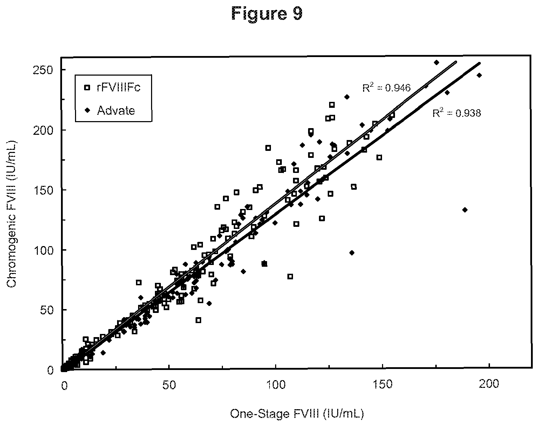

Pharmacokinetics: Correlation Between aPTT and Chromogenic Activity for rFVIIIFc in Plasma - ADVATE^{®} and rFVIIIFc activities were determined in the same assays using commercially available reagents and calibration against normal human plasma standards. There was a strong correlation between the results obtained by the one-stage clotting assay and the chromogenic assay in samples that had an activity above the LLOQ. Correlation coefficients (Pearson R²) of 0.94 and 0.95 were observed between the two assay results for 151 samples following ADVATE^{®} dosing and 185 samples following rFVIIIFc dosing, respectively. Compared to the aPTT results, the chromogenic FVIII activities were, on average, 21% higher for ADVATE^{®} and 32% higher for rFVIIIFc, not statistically significant (Figure 9). This observation led to a slightly higher estimation of exposure parameters by the chromogenic assessment for both drugs. The apparent higher FVIII recoveries by the chromogenic assay are typical for recombinant FVIII products tested in clinical assays, and are in agreement with most other marketed FVIII products.³⁰⁻³²

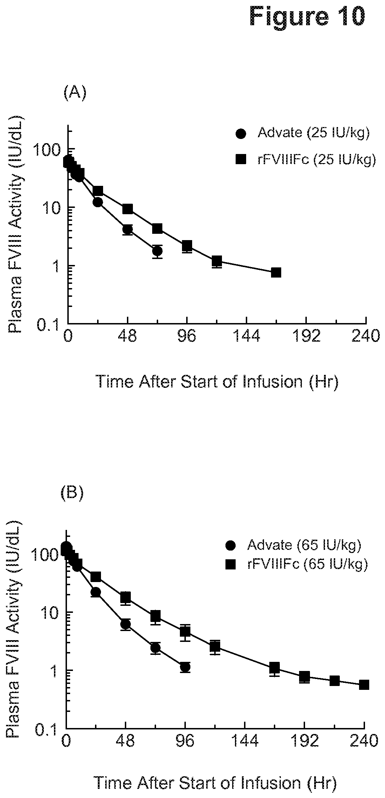

Improved Pharmacokinetics for rFVIIIFc - The primary PK estimates were derived from one-stage (aPTT) clotting assay activity data. In subjects who received 25 or 65 IU/kg of ADVATE^{®} followed by an equal dose of rFVIIIFc, the plasma FVIII activity rose sharply and reached Cₘₐₓ within the first hour following dosing. The subsequent decline of the observed FVIII activity exhibited monoexponential decay characteristics until the baseline FVIII activity was reached (Figure 10). The Cₘₐₓ increased proportionally to the dose, but was comparable between equal doses of ADVATE^{®} and rFVIIIFc (Table 8) The total exposure (AUC_{INF}) also increased proportionally to the dose. However, the AUC_{INF} of rFVIIIFc was 1.48 and 1.56-fold greater than that of ADVATE^{®} at 25 IU/kg (p=0.002) and 65 IU/kg (p<0.001), respectively (Table 8).

**Table 8. PK Parameters by One-Stage (aPTT) Assay for rFVIIIFc and ADVATE^{®} Per Dose Group**

| Parameter | Dose: 25 IU/kg (N=6) | | | Dose: 65 IU/kg (N=9) | | |
|---|---|---|---|---|---|---|
| | ADVATE^{®} Geom. Mean [95% CI] | rFVIIIFc Geom. Mean [95% CI] | Geom. Mean Ratio [95% CI] (p-value) | ADVATE^{®} Geom. Mean [95% CI] | rFVIIIFc Geom. Mean [95% CI] | Geom. Mean Ratio [95% CI] (p-value) |
| Cₘₐₓ_OB S (IU/dL) | 63.6 [59.1, 68.3] | 60.5 [53.1, 69.0] | 0.952 [0.819, 1.11] (p = 0.440) | 133 [105, 168] | 119 [103, 136] | 0.895 [0.795, 1.01] (p = 0.061) |
| AUC_{INF} (hr*IU/d L) | 994 [723, 1370] | 1480 [1160, 1880] | 1.48 [1.26,1.76] (p = 0.002) | 1800 [1350, 2400] | 2800 [1980, 3970] | 1.56 [1.33, 1.83] (p < 0.001) |
| t_{1/2} (hr) | 12.2 [9.14, 16.3] | 18.8 [14.8, 23.8] | 1.54 [1.40,1.69] (p < 0.001) | 11.0 [8.76, 13.9] | 18.8 [14.3, 24.5] | 1.70 [1.54, 1.89] (p < 0.001) |
| MRT (hr) | 17.5 [13.1, 23.4] | 27.0 [21.3, 34.2] | 1.54 [1.40,1.69] (p < 0.001) | 15.8 [12.6, 19.9] | 27.0 [20.6, 35.3] | 1.71 [1.54, 1.89] (p < 0.001) |
| CL (mL/hour /kg) | 2.49 [1.80, 3.45] | 1.68 [1.31, 2.15] | 0.673 [0.569, 0.796] (p = 0.002) | 3.61 [2.71, 4.83] | 2.32 [1.64, 3.29] | 0.642 [0.547, 0.753] (p < 0.001) |
| Vₛₛ (mL/kg) | 43.9 [39.3, 49.0] | 45.4 [39.3, 52.5] | 1.04 [0.947,1.13] (p = 0.357) | 57.4 [48.3, 68.3] | 62.8 [55.2, 71.5] | 1.09 [0.976,1.22] (p = 0.107) |
| Incremental Recovery (IU/dL per IU/kg) | 2.56 [2.36, 2.78] | 2.44 [2.12, 2.81] | 0.952 [0.819,1.11] (p = 0.444) | 2.04 [1.61, 2.59] | 1.83 [1.59, 2.10] | 0.894 [0.795,1.01] (p = 0.060) |

| | | | | | | |
|---|---|---|---|---|---|---|
| CI = Confidence Interval; Geom. Mean = Geometric Mean; OBS = observed. Estimated means, 95% CI for means, and mean differences were transformed to obtain estimated geometric means, 95% CI for geometric means, and geometric mean ratios, respectively. | | | | | | |

The t_{1/2}, MRT, CL, and Vₛₛ appeared to be independent of dose (Table 8). The geometric mean t_{1/2} of rFVIIIFc was 18.8 hours for both the 25 IU/kg and 65 IU/kg dose groups. This represents a 1.54 and 1.70-fold improvement over that of ADVATE^{®} (12.2 hours and 11.0 hours) at equivalent doses (p<0.001), respectively (Table 8). The same intra-subject improvement was observed in the MRT of rFVIIIFc (27.0 hours for both dose groups) compared with ADVATE^{®} (17.5 hours for the 25 IU/kg and 15.8 hours for the 65 IU/kg) (p<0.001). Consistent with improvement in the t_{1/2} and MRT was a corresponding 1.49 and 1.56-fold reduction in intra-subject CL at doses of 25 IU/kg (p=0.002) and 65 IU/kg (p<0.001), respectively. There were no significant differences in Vₛₛ and incremental recovery between ADVATE^{®} and rFVIIIFc. Therefore, within each subject, rFVIIIFc demonstrated an improved PK profile compared with ADVATE^{®}. It was also observed that the patients with shorter half-life on ADVATE^{®} had shorter half-life on rFVIIIFc, and patients with longer half-life on ADVATE^{®} had longer half-life on rFVIIIFc.

The improved PK profile of rFVIIIFc resulted in increased time post-dosing to 1% FVIII activity which was 1.53 and 1.68-fold longer respectively, than with ADVATE^{®} at 25 IU/kg (p<0.001) and 65 IU/kg (p<0.001) (data not shown), suggesting a potentially longer therapeutic duration for rFVIIIFc.

The favorable PK profile of rFVIIIFc relative to ADVATE^{®} was also demonstrated by FVIII activity measured in the chromogenic assay (Table 9), which was comparable to data derived from aPTT assays. The estimation of exposure, ie, Cₘₐₓ and AUC_{INF}, was slightly higher, however, based on the chromogenic assay than on the one-stage (aPTT) clotting assay for both ADVATE^{®} and rFVIIIFc.

**Table 9. PK Parameters by Two-Stage (Chromogenic) Assay for rFVIIIFc and ADVATE^{®} Per Dose Group**

| Parameter | Dose: 25 IU/kg (N=6) | | | Dose: 65 IU/kg (N=9) | | |
|---|---|---|---|---|---|---|
| | ADVATE^{®} Geom. Mean [95% CI] | rFVIIIFc Geom. Mean [95% CI] | Geom. Mean Ratio [95% CI] (p-value) | ADVATE^{®} Geom. Mean [95% CI] | rFVIIIFc Geom. Mean [95% CI] | Geom. Mean Ratio [95% CI] (p-value) |
| C_{max_}OBS (IU/dL) | 75.5 [65.5, 87.1] | 76.5 [64.9, 90.1] | 1.01 [0.940, 1.09] (p = 0.686) | 175 [143, 215] | 182 [146, 227] | 1.04 [0.900, 1.20] (p = 0.571) |
| AUC_{INF} (hr*IU/dL ) | 1060 [822, 1360] | 1660 [1300, 2120] | 1.57 [1.38, 1.80] (p < 0.001) | 2270 [1670, 3070] | 4280 [2960, 6190] | 1.89 [1.61, 2.21] (p < 0.001) |
| t_{1/2} (hr) | 10.5 [8.49, 12.9] | 16.7 [13.8, 20.1] | 1.59 [1.35, 1.87] (p < 0.001) | 10.8 [8.16, 14.2] | 19.8 [14.3, 27.5] | 1.84 [1.60, 2.12] (p < 0.001) |
| MRT (hr) | 15.0 [12.2, 18.6] | 23.9 [19.8, 28.9] | 1.59 [1.35, 1.87] (p < 0.001) | 15.4 [11.7, 20.4] | 28.5 [20.5, 39.6] | 1.85 [1.61, 2.12] (p < 0.001) |
| CL (mL/hour/ kg) | 2.35 [1.80, 3.06] | 1.49 [1.16, 1.92] | 0.636 [0.557, 0.727] (p < 0.001) | 2.87 [2.12, 3.89] | 1.52 [1.05, 2.20] | 0.530 [0.453, 0.620] (p < 0.001) |
| Vss (mL/kg) | 35.5 [30.5, 41.3] | 35.9 [30.4, 42.3] | 1.01 [0.898, 1.14] (p = 0.822) | 44.5 [36.7, 54.1] | 43.4 [38.2, 49.4] | 0.975 [0.863,1.10] (p = 0.653) |
| Increment al Recovery (IU/dL per IU/kg) | 3.05 [2.62, 3.54] | 3.09 [2.61, 3.66] | 1.01 [0.940, 1.09] (p = 0.679) | 2.70 [2.20, 3.31] | 2.80 [2.24, 3.50] | 1.04 [0.900,1.20] (p = 0.571) |

| | | | | | | |
|---|---|---|---|---|---|---|
| CI = Confidence Interval; Geom. Mean = Geometric Mean; OBS = observed. Estimated means, 95% CI for means, and mean differences were transformed to obtain estimated geometric means, 95% CI for geometric means, and geometric mean ratios, respectively. | | | | | | |

Correlation Between von Willebrand Factor and Disposition of rFVIIIFc - Because the majority of FVIII in circulation is in complex with VWF³³ and because the genome-wide association study has identified that the genetic determinants of FVIII levels are primarily dependent on VWF levels,³⁴ we examined the association between VWF and rFVIIIFc. A strong correlation was observed between VWF levels and CL and t_{1/2} for both rFVIIIFc and ADVATE^{®}. As shown in Figure 10, as the level of VWF increased, the CL of rFVIIIFc (p=0.0016) and of ADVATE^{®} (p=0.0012) decreased.

The opposite relationship was observed between the level of VWF and t_{1/2}. As the level of VWF increased, the t_{1/2} of rFVIIIFc (p=0.0003) and of ADVATE^{®} (p<0.0001) increased. This correlation suggests that the Fc moiety of rFVIIIFc does not alter the role of VWF in protecting FVIII from clearance.

Effects of Prolonged PK of rFVIIIFc on Whole Blood ROTEM^{®} - Prior to administration of study drug, blood from one subject in each dose group was spiked with an equal dose of rFVIIIFc or ADVATE^{®} and analyzed by whole blood ROTEM^{®}. Clotting time (CT) was linear with respect to the dose in the range of approximately 1% to 100% of normal, and the dose response was comparable between rFVIIIFc and ADVATE^{®} in the same subject (data not shown), indicating comparable potency of rFVIIIFc and ADVATE^{®} in clot formation.

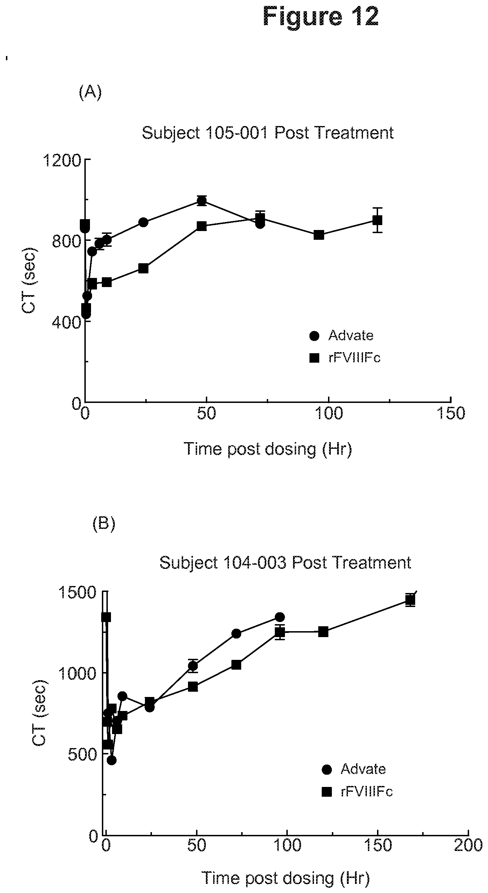

Despite the variable baseline CT due to residual FVIII levels prior to the administration of ADVATE^{®} or rFVIIIFc, both products effectively corrected the CT to comparable levels 30 minutes post-dosing (Figure 12). The improvement in CT was better sustained by rFVIIIFc than ADVATE^{®} after 3 hours following a dose of 25 IU/kg (Figure 12A), and after 24 hours following a dose of 65 IU/kg (Figure 12B).

rFVIIIFc was well tolerated by subjects at both doses. There were no clinically significant changes observed in hematology, blood chemistry, or urinalysis parameters. The majority of AEs were mild, unrelated to rFVIIIFc, and resolved without sequelae. No serious AEs or deaths occurred during the study, and no subjects at either dose developed neutralizing or binding antibodies to rFVIIIFc.

rFVIIIFc demonstrated a significantly improved FVIII activity PK profile relative to ADVATE^{®}, with t_{1/2} and MRT across dose levels being 1.54 to 1.71-fold longer, as measured by the one-stage (aPTT) clotting assay and 1.59 to 1.84-fold longer by the two-stage chromogenic assay. The prolonged activity of rFVIIIFc predicts possible prolonged efficacy, allowing for a less frequent dosing regimen in the prophylactic treatment of patients with Hemophilia A.

Adopting the PK parameters derived from this study, the Monte Carlo simulation predicts that a higher percentage of patients receiving rFVIIIFc will sustain FVIII levels above 1% or 3% as compared with patients receiving equal doses of ADVATE^{®} (Table 10). For example, at a dose of 25 IU/kg, 12.2% of ADVATE^{®} patients versus 71.2% of rFVIIIFc patients are predicted to have FVIII trough levels above 1% on Day 4; at a dose of 65 IU/kg, 11.0% of ADVATE^{®} patients versus 66.4% of rFVIIIFc patients are predicted to have FVIII levels above 3% on Day 4. Clinical trials in larger numbers of patients are planned to confirm results from this Phase 1/2a study and from the Monte Carlo simulation predictions.

**Table 10. Predicted Percentage of Subjects Achieving FVIII Trough Levels Above 1% and 3% of Normal at a Specified Dose Regimen of ADVATE^{®} or rFVIIIFc**

| Timepoint following dosing (Day) | ADVATE^{®} | | rFVIIIFc | |
|---|---|---|---|---|
| | 25 IU/kg | 65 IU/kg | 25 IU/kg | 65 IU/kg |
| | Percent of Subjects with FVIII Trough Levels above 1% | | | |
| 3 | 40.0 | 67.8 | 92.6 | 99.0 |
| 4 | 12.2 | 31.0 | 71.2 | 90.0 |
| 5 | 4.20 | 13.6 | 39.4 | 71.6 |
| 7 | 0.200 | 1.40 | 7.80 | 26.4 |

| | Percent of Subjects with FVIII Trough Levels above 3% | | | |
|---|---|---|---|---|
| 3 | 10.6 | 34.6 | 62.2 | 91.0 |
| 4 | 1.60 | 11.0 | 25.4 | 66.4 |
| 5 | 0.200 | 3.20 | 7.00 | 36.2 |
| 7 | 0 | 0.200 | 0.400 | 6.60 |

Despite the success of Fc fusion technology in prolonging circulating t_{1/2} for a variety of protein therapeutics, rFVIII was considered too large to successfully produce dimeric Fc fusions. We thus created a monomeric Fc fusion protein whereby a single effector molecule was covalent linked to a dimeric Fc, enabling binding to intracellular FcRn and subsequent recycling.^{21,22} *In vitro* coagulation assays demonstrate no loss of specific activity for rFVIIIFc, compared to B-domain deleted or native FVIII, by either clotting or chromogenic assays, using commercially available reagents and commonly used FVIII reference standards (JAD, TL, SCL, et al., manuscript submitted August, 2011). In addition, these results indicate that rFVIIIFc can be reliably assayed in a clinic setting by either the one-stage assay or the chromogenic method.

In summary, this Phase 1/2a clinical trial is the first trial to demonstrate the safety and prolonged t_{1/2} of rFVIIIFc in patients with severe hemophilia A. A pivotal Phase 3 study is ongoing with rFVIIIFc to establish effective prophylaxis dosing regimens for individuals with hemophilia A.

### Example 11

A novel single-chain (SC) isoform of factor VIII (FVIII), resulting from incomplete proteolysis at residue R1648 during biosynthesis, may provide superior manufacturability and stability relative to native FVIII. A single recombinant B domain deleted factor VIII molecule fused to an immunoglobulin Fc domain (rFVIIIFc) and its purified SC counterpart (SC-rFVIIIFc) exhibited similar specific activity in one stage clotting assays using plasma depleted of von Willebrand factor (VWF), but SC-rFVIIIFc exhibited lower specific activity in the presence of VWF. This study was undertaken to determine if VWF-bound rFVIIIFc, SC-rFVIIIFc and rBDD-FVIII (XYNTHA^{®}, REFACTO AF^{®}) differ with respect to thrombin-mediated proteolytic release from VWF.

Equimolar amounts of rFVIIIFc, SC-rFVIIIFc, and rBDD-FVIII were captured on an optical biosensor chip on which human VWF had been immobilized by amine coupling. Human α-thrombin at a range of concentrations was infused over the chip surface, and the rates of FVIII release from immobilized VWF were monitored in real time. The half maximal effective concentration (EC₅₀) of α-thrombin was determined for each FVIII species.

α-thrombin EC₅₀ values for rFVIIIFc and rBDD-FVIII were comparable (3.7 ± 0.2 U/mL and 3.2 ± 0.3 U/mL, respectively), whereas the EC₅₀ value for SC-rFVIIIFc was greater than 3-fold higher (11.7 ± 0.9 U/mL). This finding that SC-rFVIIIFc is released more slowly from VWF than are either rFVIIIFc or rBDD-FVIII is consistent with a previously observed finding regarding the activities of rFVIIIFc and SC-rFVIIIFc in a one-stage clotting assay (aPTT) in which SC-FVIIIFc had a lower apparent activity only when VWF was present in the assay plasma sample. However, all samples possessed equivalent activities in a mouse bleeding model, indicating that responsiveness of FVIII preparations to thrombin in the release of FVIII from VWF does not correlate with efficacy *in vivo.*

**TABLE 1: Polynucleotide Sequences**

| **A. B-Domain Deleted FVIIIFc** |
|---|
| (i) B-Domain Deleted FVIIIFc Chain DNA Sequence (FVIII signal peptide underlined. Fc region in bold) (SEQ ID NO:1, which encodes SEQ ID NO:2) |
| 661 A TGCAAATAGA GCTCTCCACC TGCTTCTTTC 721 TGTGCCTTTT GCGATTCTGC TTTAGTGCCA CCAGAAGATA CTACCTGGGT GCAGTGGAAC 781 TGTCATGGGA CTATATGCAA AGTGATCTCG GTGAGCTGCC TGTGGACGCA AGATTTCCTC 841 CTAGAGTGCC AAAATCTTTT CCATTCAACA CCTCAGTCGT GTACAAAAAG ACTCTGTTTG 901 TAGAATTCAC GGATCACCTT TTCAACATCG CTAAGCCAAG GCCACCCTGG ATGGGTCTGC 961 TAGGTCCTAC CATCCAGGCT GAGGTTTATG ATACAGTGGT CATTACACTT AAGAACATGG 1021 CTTCCCATCC TGTCAGTCTT CATGCTGTTG GTGTATCCTA CTGGAAAGCT TCTGAGGGAG 1081 CTGAATATGA TGATCAGACC AGTCAAAGGG AGAAAGAAGA TGATAAAGTC TTCCCTGGTG 1141 GAAGCCATAC ATATGTCTGG CAGGTCCTGA AAGAGAATGG TCCAATGGCC TCTGACCCAC 1201 TGTGCCTTAC CTACTCATAT CTTTCTCATG TGGACCTGGT AAAAGACTTG AATTCAGGCC 1261 TCATTGGAGC CCTACTAGTA TGTAGAGAAG GGAGTCTGGC CAAGGAAAAG ACACAGACCT 1321 TGCACAAATT TATACTACTT TTTGCTGTAT TTGATGAAGG GAAAAGTTGG CACTCAGAAA 1381 CAAAGAACTC CTTGATGCAG GATAGGGATG CTGCATCTGC TCGGGCCTGG CCTAAAATGC 1441 ACACAGTCAA TGGTTATGTA AACAGGTCTC TGCCAGGTCT GATTGGATGC CACAGGAAAT 1501 CAGTCTATTG GCATGTGATT GGAATGGGCA CCACTCCTGA AGTGCACTCA ATATTCCTCG 1561 AAGGTCACAC ATTTCTTGTG AGGAACCATC GCCAGGCGTC CTTGGAAATC TCGCCAATAA 1621 CTTTCCTTAC TGCTCAAACA CTCTTGATGG ACCTTGGACA GTTTCTACTG TTTTGTCATA 1681 TCTCTTCCCA CCAACATGAT GGCATGGAAG CTTATGTCAA AGTAGACAGC TGTCCAGAGG 1741 AACCCCAACT ACGAATGAAA AATAATGAAG AAGCGGAAGA CTATGATGAT GATCTTACTG 1801 ATTCTGAAAT GGATGTGGTC AGGTTTGATG ATGACAACTC TCCTTCCTTT ATCCAAATTC 1861 GCTCAGTTGC CAAGAAGCAT CCTAAAACTT GGGTACATTA CATTGCTGCT GAAGAGGAGG 1921 ACTGGGACTA TGCTCCCTTA GTCCTCGCCC CCGATGACAG AAGTTATAAA AGTCAATATT 1981 TGAACAATGG CCCTCAGCGG ATTGGTAGGA AGTACAAAAA AGTCCGATTT ATGGCATACA 2041 CAGATGAAAC CTTTAAGACT CGTGAAGCTA TTCAGCATGA ATCAGGAATC TTGGGACCTT 2101 TACTTTATGG GGAAGTTGGA GACACACTGT TGATTATATT TAAGAATCAA GCAAGCAGAC 2161 CATATAACAT CTACCCTCAC GGAATCACTG ATGTCCGTCC TTTGTATTCA AGGAGATTAC 2221 CAAAAGGTGT AAAACATTTG AAGGATTTTC CAATTCTGCC AGGAGAAATA TTCAAATATA 2281 AATGGACAGT GACTGTAGAA GATGGGCCAA CTAAATCAGA TCCTCGGTGC CTGACCCGCT 2341 ATTACTCTAG TTTCGTTAAT ATGGAGAGAG ATCTAGCTTC AGGACTCATT GGCCCTCTCC 2401 TCATCTGCTA CAAAGAATCT GTAGATCAAA GAGGAAACCA GATAATGTCA GACAAGAGGA 2461 ATGTCATCCT GTTTTCTGTA TTTGATGAGA ACCGAAGCTG GTACCTCACA GAGAATATAC 2521 AACGCTTTCT CCCCAATCCA GCTGGAGTGC AGCTTGAGGA TCCAGAGTTC CAAGCCTCCA 2581 ACATCATGCA CAGCATCAAT GGCTATGTTT TTGATAGTTT GCAGTTGTCA GTTTGTTTGC 2641 ATGAGGTGGC ATACTGGTAC ATTCTAAGCA TTGGAGCACA GACTGACTTC CTTTCTGTCT 2701 TCTTCTCTGG ATATACCTTC AAACACAAAA TGGTCTATGA AGACACACTC ACCCTATTCC 2761 CATTCTCAGG AGAAACTGTC TTCATGTCGA TGGAAAACCC AGGTCTATGG ATTCTGGGGT 2821 GCCACAACTC AGACTTTCGG AACAGAGGCA TGACCGCCTT ACTGAAGGTT TCTAGTTGTG 2881 ACAAGAACAC TGGTGATTAT TACGAGGACA GTTATGAAGA TATTTCAGCA TACTTGCTGA 2941 GTAAAAACAA TGCCATTGAA CCAAGAAGCT TCTCTCAAAA CCCACCAGTC TTGAAACGCC 3001 ATCAACGGGA AATAACTCGT ACTACTCTTC AGTCAGATCA AGAGGAAATT GACTATGATG 3061 ATACCATATC AGTTGAAATG AAGAAGGAAG ATTTTGACAT TTATGATGAG GATGAAAATC 3121 AGAGCCCCCG CAGCTTTCAA AAGAAAACAC GACACTATTT TATTGCTGCA GTGGAGAGGC 3181 TCTGGGATTA TGGGATGAGT AGCTCCCCAC ATGTTCTAAG AAACAGGGCT CAGAGTGGCA 3241 GTGTCCCTCA GTTCAAGAAA GTTGTTTTCC AGGAATTTAC TGATGGCTCC TTTACTCAGC 3301 CCTTATACCG TGGAGAACTA AATGAACATT TGGGACTCCT GGGGCCATAT ATAAGAGCAG 3361 AAGTTGAAGA TAATATCATG GTAACTTTCA GAAATCAGGC CTCTCGTCCC TATTCCTTCT 3421 ATTCTAGCCT TATTTCTTAT GAGGAAGATC AGAGGCAAGG AGCAGAACCT AGAAAAAACT 3481 TTGTCAAGCC TAATGAAACC AAAACTTACT TTTGGAAAGT GCAACATCAT ATGGCACCCA 3541 CTAAAGATGA GTTTGACTGC AAAGCCTGGG CTTATTTCTC TGATGTTGAC CTGGAAAAAG 3601 ATGTGCACTC AGGCCTGATT GGACCCCTTC TGGTCTGCCA CACTAACACA CTGAACCCTG |
| 3661 CTCATGGGAG ACAAGTGACA GTACAGGAAT TTGCTCTGTT TTTCACCATC TTTGATGAGA 3721 CCAAAAGCTG GTACTTCACT GAAAATATGG AAAGAAACTG CAGGGCTCCC TGCAATATCC 3781 AGATGGAAGA TCCCACTTTT AAAGAGAATT ATCGCTTCCA TGCAATCAAT GGCTACATAA 3841 TGGATACACT ACCTGGCTTA GTAATGGCTC AGGATCAAAG GATTCGATGG TATCTGCTCA 3901 GCATGGGCAG CAATGAAAAC ATCCATTCTA TTCATTTCAG TGGACATGTG TTCACTGTAC 3961 GAAAAAAAGA GGAGTATAAA ATGGCACTGT ACAATCTCTA TCCAGGTGTT TTTGAGACAG 4021 TGGAAATGTT ACCATCCAAA GCTGGAATTT GGCGGGTGGA ATGCCTTATT GGCGAGCATC 4081 TACATGCTGG GATGAGCACA CTTTTTCTGG TGTACAGCAA TAAGTGTCAG ACTCCCCTGG 4141 GAATGGCTTC TGGACACATT AGAGATTTTC AGATTACAGC TTCAGGACAA TATGGACAGT 4201 GGGCCCCAAA GCTGGCCAGA CTTCATTATT CCGGATCAAT CAATGCCTGG AGCACCAAGG 4261 AGCCCTTTTC TTGGATCAAG GTGGATCTGT TGGCACCAAT GATTATTCAC GGCATCAAGA 4321 CCCAGGGTGC CCGTCAGAAG TTCTCCAGCC TCTACATCTC TCAGTTTATC ATCATGTATA 4381 GTCTTGATGG GAAGAAGTGG CAGACTTATC GAGGAAATTC CACTGGAACC TTAATGGTCT 4441 TCTTTGGCAA TGTGGATTCA TCTGGGATAA AACACAATAT TTTTAACCCT CCAATTATTG 4501 CTCGATACAT CCGTTTGCAC CCAACTCATT ATAGCATTCG CAGCACTCTT CGCATGGAGT 4561 TGATGGGCTG TGATTTAAAT AGTTGCAGCA TGCCATTGGG AATGGAGAGT AAAGCAATAT 4621 CAGATGCACA GATTACTGCT TCATCCTACT TTACCAATAT GTTTGCCACC TGGTCTCCTT 4681 CAAAAGCTCG ACTTCACCTC CAAGGGAGGA GTAATGCCTG GAGACCTCAG GTGAATAATC 4741 CAAAAGAGTG GCTGCAAGTG GACTTCCAGA AGACAATGAA AGTCACAGGA GTAACTACTC 4801 AGGGAGTAAA ATCTCTGCTT ACCAGCATGT ATGTGAAGGA GTTCCTCATC TCCAGCAGTC 4861 AAGATGGCCA TCAGTGGACT CTCTTTTTTC AGAATGGCAA AGTAAAGGTT TTTCAGGGAA 4921 ATCAAGACTC CTTCACACCT GTGGTGAACT CTCTAGACCC ACCGTTACTG ACTCGCTACC 4981 TTCGAATTCA CCCCCAGAGT TGGGTGCACC AGATTGCCCT GAGGATGGAG GTTCTGGGCT 5041 GCGAGGCACA GGACCTCTAC GACAAAACTC ACACATGCCC ACCGTGCCCA GCTCCAGAAC 5101 TCCTGGGCGG ACCGTCAGTC TTCCTCTTCC CCCCAAAACC CAAGGACACC CTCATGATCT 5161 CCCGGACCCC TGAGGTCACA TGCGTGGTGG TGGACGTGAG CCACGAAGAC CCTGAGGTCA 5221 AGTTCAACTG GTACGTGGAC GGCGTGGAGG TGCATAATGC CAAGACAAAG CCGCGGGAGG 5281 AGCAGTACAA CAGCACGTAC CGTGTGGTCA GCGTCCTCAC CGTCCTGCAC CAGGACTGGC 5341 TGAATGGCAA GGAGTACAAG TGCAAGGTCT CCAACAAAGC CCTCCCAGCC CCCATCGAGA 5401 AAACCATCTC CAAAGCCAAA GGGCAGCCCC GAGAACCACA GGTGTACACC CTGCCCCCAT 5461 CCCGGGATGA GCTGACCAAG AACCAGGTCA GCCTGACCTG CCTGGTCAAA GGCTTCTATC 5521 CCAGCGACAT CGCCGTGGAG TGGGAGAGCA ATGGGCAGCC GGAGAACAAC TACAAGACCA 5581 CGCCTCCCGT GTTGGACTCC GACGGCTCCT TCTTCCTCTA CAGCAAGCTC ACCGTGGACA 5641 AGAGCAGGTG GCAGCAGGGG AACGTCTTCT CATGCTCCGT GATGCATGAG GCTCTGCACA 5701 ACCACTACAC GCAGAAGAGC CTCTCCCTGT CTCCGGGTAA A |
| (ii) Fc DNA sequence (mouse Ier signal peptide underlined) (SEQ ID NO:3. which encodes SEQ ID NO:4) |
| 7981                    ATGGA GACAGACACA 8041    CTCCTGCTAT GGGTACTGCT GCTCTGGGTT CCAGGTTCCA CTGGTGACAA AACTCACACA 8101    TGCCCACCGT GCCCAGCACC TGAACTCCTG GGAGGACCGT CAGTCTTCCT CTTCCCCCCA 8161    AAACCCAAGG ACACCCTCAT GATCTCCCGG ACCCCTGAGG TCACATGCGT GGTGGTGGAC 8221    GTGAGCCACG AAGACCCTGA GGTCAAGTTC AACTGGTACG TGGACGGCGT GGAGGTGCAT 8281    AATGCCAAGA CAAAGCCGCG GGAGGAGCAG TACAACAGCA CGTACCGTGT GGTCAGCGTC 8341    CTCACCGTCC TGCACCAGGA CTGGCTGAAT GGCAAGGAGT ACAAGTGCAA GGTCTCCAAC 8401    AAAGCCCTCC CAGCCCCCAT CGAGAAAACC ATCTCCAAAG CCAAAGGGCA GCCCCGAGAA 8461    CCACAGGTGT ACACCCTGCC CCCATCCCGC GATGAGCTGA CCAAGAACCA GGTCAGCCTG 8521    ACCTGCCTGG TCAAAGGCTT CTATCCCAGC GACATCGCCG TGGAGTGGGA GAGCAATGGG 8581    CAGCCGGAGA ACAACTACAA GACCACGCCT CCCGTGTTGG ACTCCGACGG CTCCTTCTTC 8641    CTCTACAGCA AGCTCACCGT GGACAAGAGC AGGTGGCAGC AGGGGAACGT CTTCTCATGC 8701    TCCGTGATGC ATGAGGCTCT GCACAACCAC TACACGCAGA AGAGCCTCTC CCTGTCTCCG 8761    GGTAAA |

| **B. Full Length FVIIIFc** |
|---|
| (i) Full Length FVIIIFc DNA Sequence (FVIII signal peptide underlined. Fc region in bold) (SEQ ID NO:5. which encodes SEQ ID NO:6) |
| 661 ATG CAAATAGAGC TCTCCACCTG 721 CTTCTTTCTG TGCCTTTTGC GATTCTGCTT TAGTGCCACC AGAAGATACT ACCTGGGTGC 781 AGTGGAACTG TCATGGGACT ATATGCAAAG TGATCTCGGT GAGCTGCCTG TGGACGCAAG 841 ATTTCCTCCT AGAGTGCCAA AATCTTTTCC ATTCAACACC TCAGTCGTGT ACAAAAAGAC 901 TCTGTTTGTA GAATTCACGG ATCACCTTTT CAACATCGCT AAGCCAAGGC CACCCTGGAT 961 GGGTCTGCTA GGTCCTACCA TCCAGGCTGA GGTTTATGAT ACAGTGGTCA TTACACTTAA 1021 GAACATGGCT TCCCATCCTG TCAGTCTTCA TGCTGTTGGT GTATCCTACT GGAAAGCTTC 1081 TGAGGGAGCT GAATATGATG ATCAGACCAG TCAAAGGGAG AAAGAAGATG ATAAAGTCTT 1141 CCCTGGTGGA AGCCATACAT ATGTCTGGCA GGTCCTGAAA GAGAATGGTC CAATGGCCTC 1201 TGACCCACTG TGCCTTACCT ACTCATATCT TTCTCATGTG GACCTGGTAA AAGACTTGAA 1261 TTCAGGCCTC ATTGGAGCCC TACTAGTATG TAGAGAAGGG AGTCTGGCCA AGGAAAAGAC 1321 ACAGACCTTG CACAAATTTA TACTACTTTT TGCTGTATTT GATGAAGGGA AAAGTTGGCA 1381 CTCAGAAACA AAGAACTCCT TGATGCAGGA TAGGGATGCT GCATCTGCTC GGGCCTGGCC 1441 TAAAATGCAC ACAGTCAATG GTTATGTAAA CAGGTCTCTG CCAGGTCTGA TTGGATGCCA 1501 CAGGAAATCA GTCTATTGGC ATGTGATTGG AATGGGCACC ACTCCTGAAG TGCACTCAAT 1561 ATTCCTCGAA GGTCACACAT TTCTTGTGAG GAACCATCGC CAGGCGTCCT TGGAAATCTC- 1621 GCCAATAACT TTCCTTACTG CTCAAACACT CTTGATGGAC CTTGGACAGT TTCTACTGTT 1681 TTGTCATATC TCTTCCCACC AACATGATGG CATGGAAGCT TATGTCAAAG TAGACAGCTG 1741 TCCAGAGGAA CCCCAACTAC GAATGAAAAA TAATGAAGAA GCGGAAGACT ATGATGATGA 1801 TCTTACTGAT TCTGAAATGG ATGTGGTCAG GTTTGATGAT GACAACTCTC CTTCCTTTAT 1861 CCAAATTCGC TCAGTTGCCA AGAAGCATCC TAAAACTTGG GTACATTACA TTGCTGCTGA 1921 AGAGGAGGAC TGGGACTATG CTCCCTTAGT CCTCGCCCCC GATGACAGAA GTTATAAAAG 1981 TCAATATTTG AACAATGGCC CTCAGCGGAT TGGTAGGAAG TACAAAAAAG TCCGATTTAT 2041 GGCATACACA GATGAAACCT TTAAGACTCG TGAAGCTATT CAGCATGAAT CAGGAATCTT 2101 GGGACCTTTA CTTTATGGGG AAGTTGGAGA CACACTGTTG ATTATATTTA AGAATCAAGC 2161 AAGCAGACCA TATAACATCT ACCCTCACGG AATCACTGAT GTCCGTCCTT TGTATTCAAG 2221 GAGATTACCA AAAGGTGTAA AACATTTGAA GGATTTTCCA ATTCTGCCAG GAGAAATATT 2281 CAAATATAAA TGGACAGTGA CTGTAGAAGA TGGGCCAACT AAATCAGATC CTCGGTGCCT 2341 GACCCGCTAT TACTCTAGTT TCGTTAATAT GGAGAGAGAT CTAGCTTCAG GACTCATTGG 2401 CCCTCTCCTC ATCTGCTACA AAGAATCTGT AGATCAAAGA GGAAACCAGA TAATGTCAGA 2461 CAAGAGGAAT GTCATCCTGT TTTCTGTATT TGATGAGAAC CGAAGCTGGT ACCTCACAGA 2521 GAATATACAA CGCTTTCTCC CCAATCCAGC TGGAGTGCAG CTTGAGGATC CAGAGTTCCA 2581 AGCCTCCAAC ATCATGCACA GCATCAATGG CTATGTTTTT GATAGTTTGC AGTTGTCAGT 2641 TTGTTTGCAT GAGGTGGCAT ACTGGTACAT TCTAAGCATT GGAGCACAGA CTGACTTCCT 2701 TTCTGTCTTC TTCTCTGGAT ATACCTTCAA ACACAAAATG GTCTATGAAG ACACACTCAC 2761 CCTATTCCCA TTCTCAGGAG AAACTGTCTT CATGTCGATG GAAAACCCAG GTCTATGGAT 2821 TCTGGGGTGC CACAACTCAG ACTTTCGGAA CAGAGGCATG ACCGCCTTAC TGAAGGTTTC 2881 TAGTTGTGAC AAGAACACTG GTGATTATTA CGAGGACAGT TATGAAGATA TTTCAGCATA 2941 CTTGCTGAGT AAAAACAATG CCATTGAACC AAGAAGCTTC TCCCAGAATT CAAGACACCC 3001 TAGCACTAGG CAAAAGCAAT TTAATGCCAC CACAATTCCA GAAAATGACA TAGAGAAGAC 3061 TGACCCTTGG TTTGCACACA GAACACCTAT GCCTAAAATA CAAAATGTCT CCTCTAGTGA 3121 TTTGTTGATG CTCTTGCGAC AGAGTCCTAC TCCACATGGG CTATCCTTAT CTGATCTCCA 3181 AGAAGCCAAA TATGAGACTT TTTCTGATGA TCCATCACCT GGAGCAATAG ACAGTAATAA 3241 CAGCCTGTCT GAAATGACAC ACTTCAGGCC ACAGCTCCAT CACAGTGGGG ACATGGTATT 3301 TACCCCTGAG TCAGGCCTCC AATTAAGATT AAATGAGAAA CTGGGGACAA CTGCAGCAAC 3361 AGAGTTGAAG AAACTTGATT TCAAAGTTTC TAGTACATCA AATAATCTGA TTTCAACAAT 3421 TCCATCAGAC AATTTGGCAG CAGGTACTGA TAATACAAGT TCCTTAGGAC CCCCAAGTAT 3481 GCCAGTTCAT TATGATAGTC AATTAGATAC CACTCTATTT GGCAAAAAGT CATCTCCCCT 3541 TACTGAGTCT GGTGGACCTC TGAGCTTGAG TGAAGAAAAT AATGATTCAA AGTTGTTAGA 3601 ATCAGGTTTA ATGAATAGCC AAGAAAGTTC ATGGGGAAAA AATGTATCGT CAACAGAGAG 3661 TGGTAGGTTA TTTAAAGGGA AAAGAGCTCA TGGACCTGCT TTGTTGACTA AAGATAATGC 3721 CTTATTCAAA GTTAGCATCT CTTTGTTAAA GACAAACAAA ACTTCCAATA ATTCAGCAAC 3781 TAATAGAAAG ACTCACATTG ATGGCCCATC ATTATTAATT GAGAATAGTC CATCAGTCTG 3841 GCAAAATATA TTAGAAAGTG ACACTGAGTT TAAAAAAGTG ACACCTTTGA TTCATGACAG 3901 AATGCTTATG GACAAAAATG CTACAGCTTT GAGGCTAAAT CATATGTCAA ATAAAACTAC 3961 TTCATCAAAA AACATGGAAA TGGTCCAACA GAAAAAAGAG GGCCCCATTC CACCAGATGC 4021 ACAAAATCCA GATATGTCGT TCTTTAAGAT GCTATTCTTG CCAGAATCAG CAAGGTGGAT 4081 ACAAAGGACT CATGGAAAGA ACTCTCTGAA CTCTGGGCAA GGCCCCAGTC CAAAGCAATT 4141 AGTATCCTTA GGACCAGAAA AATCTGTGGA AGGTCAGAAT TTCTTGTCTG AGAAAAACAA 4201 AGTGGTAGTA GGAAAGGGTG AATTTACAAA GGACGTAGGA CTCAAAGAGA TGGTTTTTCC |
| 4261 AAGCAGCAGA AACCTATTTC TTACTAACTT GGATAATTTA CATGAAAATA ATACACACAA 4321 TCAAGAAAAA AAAATTCAGG AAGAAATAGA AAAGAAGGAA ACATTAATCC AAGAGAATGT 4381 AGTTTTGCCT CAGATACATA CAGTGACTGG CACTAAGAAT TTCATGAAGA ACCTTTTCTT 4441 ACTGAGCACT AGGCAAAATG TAGAAGGTTC ATATGACGGG GCATATGCTC CAGTACTTCA 4501 AGATTTTAGG TCATTAAATG ATTCAACAAA TAGAACAAAG AAACACACAG CTCATTTCTC 4561 AAAAAAAGGG GAGGAAGAAA ACTTGGAAGG CTTGGGAAAT CAAACCAAGC AAATTGTAGA 4621 GAAATATGCA TGCACCACAA GGATATCTCC TAATACAAGC CAGCAGAATT TTGTCACGCA 4681 ACGTAGTAAG AGAGCTTTGA AACAATTCAG ACTCCCACTA GAAGAAACAG AACTTGAAAA 4741 AAGGATAATT GTGGATGACA CCTCAACCCA GTGGTCCAAA AACATGAAAC ATTTGACCCC 4801 GAGCACCCTC ACACAGATAG ACTACAATGA GAAGGAGAAA GGGGCCATTA CTCAGTCTCC 4861 CTTATCAGAT TGCCTTACGA GGAGTCATAG CATCCCTCAA GCAAATAGAT CTCCATTACC 4921 CATTGCAAAG GTATCATCAT TTCCATCTAT TAGACCTATA TATCTGACCA GGGTCCTATT 4981 CCAAGACAAC TCTTCTCATC TTCCAGCAGC ATCTTATAGA AAGAAAGATT CTGGGGTCCA 5041 AGAAAGCAGT CATTTCTTAC AAGGAGCCAA AAAAAATAAC CTTTCTTTAG CCATTCTAAC 5101 CTTGGAGATG ACTGGTGATC AAAGAGAGGT TGGCTCCCTG GGGACAAGTG CCACAAATTC 5161 AGTCACATAC AAGAAAGTTG AGAACACTGT TCTCCCGAAA CCAGACTTGC CCAAAACATC 5221 TGGCAAAGTT GAATTGCTTC CAAAAGTTCA CATTTATCAG AAGGACCTAT TCCCTACGGA 5281 AACTAGCAAT GGGTCTCCTG GCCATCTGGA TCTCGTGGAA GGGAGCCTTC TTCAGGGAAC 5341 AGAGGGAGCG ATTAAGTGGA ATGAAGCAAA CAGACCTGGA AAAGTTCCCT TTCTGAGAGT 5401 AGCAACAGAA AGCTCTGCAA AGACTCCCTC CAAGCTATTG GATCCTCTTG CTTGGGATAA 5461 CCACTATGGT ACTCAGATAC CAAAAGAAGA GTGGAAATCC CAAGAGAAGT CACCAGAAAA 5521 AACAGCTTTT AAGAAAAAGG ATACCATTTT GTCCCTGAAC GCTTGTGAAA GCAATCATGC 5581 AATAGCAGCA ATAAATGAGG GACAAAATAA GCCCGAAATA GAAGTCACCT GGGCAAAGCA 5641 AGGTAGGACT GAAAGGCTGT GCTCTCAAAA CCCACCAGTC TTGAAACGCC ATCAACGGGA 5701 AATAACTCGT ACTACTCTTC AGTCAGATCA AGAGGAAATT GACTATGATG ATACCATATC 5761 AGTTGAAATG AAGAAGGAAG ATTTTGACAT TTATGATGAG GATGAAAATC AGAGCCCCCG 5821 CAGCTTTCAA AAGAAAACAC GACACTATTT TATTGCTGCA GTGGAGAGGC TCTGGGATTA 5881 TGGGATGAGT AGCTCCCCAC ATGTTCTAAG AAACAGGGCT CAGAGTGGCA GTGTCCCTCA 5941 GTTCAAGAAA GTTGTTTTCC AGGAATTTAC TGATGGCTCC TTTACTCAGC CCTTATACCG 6001 TGGAGAACTA AATGAACATT TGGGACTCCT GGGGCCATAT ATAAGAGCAG AAGTTGAAGA 6061 TAATATCATG GTAACTTTCA GAAATCAGGC CTCTCGTCCC TATTCCTTCT ATTCTAGCCT 6121 TATTTCTTAT GAGGAAGATC AGAGGCAAGG AGCAGAACCT AGAAAAAACT TTGTCAAGCC 6181 TAATGAAACC AAAACTTACT TTTGGAAAGT GCAACATCAT ATGGCACCCA CTAAAGATGA 6241 GTTTGACTGC AAAGCCTGGG CTTATTTCTC TGATGTTGAC CTGGAAAAAG ATGTGCACTC 6301 AGGCCTGATT GGACCCCTTC TGGTCTGCCA CACTAACACA CTGAACCCTG CTCATGGGAG 6361 ACAAGTGACA GTACAGGAAT TTGCTCTGTT TTTCACCATC TTTGATGAGA CCAAAAGCTG 6421 GTACTTCACT GAAAATATGG AAAGAAACTG CAGGGCTCCC TGCAATATCC AGATGGAAGA 6481 TCCCACTTTT AAAGAGAATT ATCGCTTCCA TGCAATCAAT GGCTACATAA TGGATACACT 6541 ACCTGGCTTA GTAATGGCTC AGGATCAAAG GATTCGATGG TATCTGCTCA GCATGGGCAG 6601 CAATGAAAAC ATCCATTCTA TTCATTTCAG TGGACATGTG TTCACTGTAC GAAAAAAAGA 6661 GGAGTATAAA ATGGCACTGT ACAATCTCTA TCCAGGTGTT TTTGAGACAG TGGAAATGTT 6721 ACCATCCAAA GCTGGAATTT GGCGGGTGGA ATGCCTTATT GGCGAGCATC TACATGCTGG 6781 GATGAGCACA CTTTTTCTGG TGTACAGCAA TAAGTGTCAG ACTCCCCTGG GAATGGCTTC 6841 TGGACACATT AGAGATTTTC AGATTACAGC TTCAGGACAA TATGGACAGT GGGCCCCAAA 6901 GCTGGCCAGA CTTCATTATT CCGGATCAAT CAATGCCTGG AGCACCAAGG AGCCCTTTTC 6961 TTGGATCAAG GTGGATCTGT TGGCACCAAT GATTATTCAC GGCATCAAGA CCCAGGGTGC 7021 CCGTCAGAAG TTCTCCAGCC TCTACATCTC TCAGTTTATC ATCATGTATA GTCTTGATGG 7081 GAAGAAGTGG CAGACTTATC GAGGAAATTC CACTGGAACC TTAATGGTCT TCTTTGGCAA 7141 TGTGGATTCA TCTGGGATAA AACACAATAT TTTTAACCCT CCAATTATTG CTCGATACAT 7201 CCGTTTGCAC CCAACTCATT ATAGCATTCG CAGCACTCTT CGCATGGAGT TGATGGGCTG 7261 TGATTTAAAT AGTTGCAGCA TGCCATTGGG AATGGAGAGT AAAGCAATAT CAGATGCACA 7321 GATTACTGCT TCATCCTACT TTACCAATAT GTTTGCCACC TGGTCTCCTT CAAAAGCTCG 7381 ACTTCACCTC CAAGGGAGGA GTAATGCCTG GAGACCTCAG GTGAATAATC CAAAAGAGTG 7441 GCTGCAAGTG GACTTCCAGA AGACAATGAA AGTCACAGGA GTAACTACTC AGGGAGTAAA 7501 ATCTCTGCTT ACCAGCATGT ATGTGAAGGA GTTCCTCATC TCCAGCAGTC AAGATGGCCA 7561 TCAGTGGACT CTCTTTTTTC AGAATGGCAA AGTAAAGGTT TTTCAGGGAA ATCAAGACTC 7621 CTTCACACCT GTGGTGAACT CTCTAGACCC ACCGTTACTG ACTCGCTACC TTCGAATTCA 7681 CCCCCAGAGT TGGGTGCACC AGATTGCCCT GAGGATGGAG GTTCTGGGCT GCGAGGCACA 7741 GGACCTCTAC GACAAAACTC ACACATGCCC ACCGTGCCCA GCTCCAGAAC TCCTGGGCGG 7801 ACCGTCAGTC TTCCTCTTCC CCCCAAAACC CAAGGACACC CTCATGATCT CCCGGACCCC 7861 TGAGGTCACA TGCGTGGTGG TGGACGTGAG CCACGAAGAC CCTGAGGTCA AGTTCAACTG 7921 GTACGTGGAC GGCGTGGAGG TGCATAATGC CAAGACAAAG CCGCGGGAGG AGCAGTACAA 7981 CAGCACGTAC CGTGTGGTCA GCGTCCTCAC CGTCCTGCAC CAGGACTGGC TGAATGGCAA 8041 GGAGTACAAG TGCAAGGTCT CCAACAAAGC CCTCCCAGCC CCCATCGAGA AAACCATCTC 8101 CAAAGCCAAA GGGCAGCCCC GAGAACCACA GGTGTACACC CTGCCCCCAT CCCGGGATGA 8161 GCTGACCAAG AACCAGGTCA GCCTGACCTG CCTGGTCAAA GGCTTCTATC CCAGCGACAT 8221 CGCCGTGGAG TGGGAGAGCA ATGGGCAGCC GGAGAACAAC TACAAGACCA CGCCTCCCGT |
| 8281 GTTGGACTCC GACGGCTCCT TCTTCCTCTA CAGCAAGCTC ACCGTGGACA AGAGCAGGTG 8341 GCAGCAGGGG AACGTCTTCT CATGCTCCGT GATGCATGAG GCTCTGCACA ACCACTACAC 8401 GCAGAAGAGC CTCTCCCTGT CTCCGGGTAA A |
| (ii) Fc (same sequence as A (ii) (SEQ ID NO:3))] |

| Table 2: Polypeptide Sequences |
|---|
| **A. B-Domain Deleted FVIII-Fc Monomer Hybrid (BDD FVIIIFc monomer dimer): created by coexpressing BDD FVIIIFc and Fc chains.** |
| Construct = HC-LC-Fc fusion. An Fc expression cassette is cotransfected with BDDFVIII-Fc to generate the BDD FVIIIFc monomer-. For the BDD FVIIIFc chain, the Fc sequence is shown in bold; HC sequence is shown in double underline; remaining B domain sequence is shown in italics. Signal peptides are underlined. |
| i) B domain deleted FVIII-Fc chain (19 amino acid signal sequence underlined) (SEQ ID NO:2) |
| MQIELSTCFFLCLLRFCFS ATRRYYLGAVELSWDYMQSDLGELPVDARFPPRVPKSFPFNTSVVYKKTLFVEFTDHLFNIAKPRPPWMGL LGPTIQAEVYDTVVITLKNMASHPVSLHAVGVSYWKASEGAEYDDQTSQREKEDDKVFPGGSHTYVWQVLK ENGPMASDPLCLTYSYLSHVDLVKDLNSGLIGALLVCREGSLAKEKTQTLHKFILLFAVFDEGKSWHSETK NSLMQDRDAASARAWPKMHTVNGYVNRSLPGLIGCHRKSVYWHVIGMGTTPEVHSIFLEGHTFLVRNHRQA SLEISPITFLTAQTLLMDLGQFLLFCHISSHQHDGMEAYVKVDSCPEEPQLRMKNNEEAEDYDDDLTDSEM DVVRFDDDNSPSFIQIRSVAKKHPKTWVHYIAAEEEDWDYAPLVLAPDDRSYKSQYLNNGPQRIGRKYKKV RFMAYTDETFKTREAIQHESGILGPLLYGEVGDTLLIIFKNQASRPYNIYPHGITDVRPLYSRRLPKGVKH LKDFPILPGEIFKYKWTVTVEDGPTKSDPRCLTRYYSSFVNMERDLASGLIGPLLICYKESVDQRGNQIMS DKRNVILFSVFDENRSWYLTENIQRFLPNPAGVQLEDPEFQASNIMHSINGYVFDSLQLSVCLHEVAYWYI LSIGAQTDFLSVFFSGYTFKHKMVYEDTLTLFPFSGETVFMSMENPGLWILGCHNSDFRNRGMTALLKVSS CDKNTGDYYEDSYEDISAYLLSKNNAIEPRSFSQNPPVLKRHQREITRTTLQSDQEEIDYDDTISVEMKKE DFDIYDEDENQSPRSFQKKTRHYFIAAVERLWDYGMSSSPHVLRNRAQSGSVPQFKKVVFQEFTDGSFTQP LYRGELNEHLGLLGPYIRAEVEDNIMVTFRNQASRPYSFYSSLISYEEDQRQGAEPRKNFVKPNETKTYFW KVQHHMAPTKDEFDCKAWAYFSDVDLEKDVHSGLIGPLLVCHTNTLNPAHGRQVTVQEFALFFTIFDETKS WYFTENMERNCRAPCNIQMEDPTFKENYRFHAINGYIMDTLPGLVMAQDQRIRWYLLSMGSNENIHSIHFS GHVFTVRKKEEYKMALYNLYPGVFETVEMLPSKAGIWRVECLIGEHLHAGMSTLFLVYSNKCQTPLGMASG HIRDFQITASGQYGQWAPKLARLHYSGSINAWSTKEPFSWIKVDLLAPMIIHGIKTQGARQKFSSLYISQF IIMYSLDGKKWQTYRGNSTGTLMVFFGNVDSSGIKHNIFNPPIIARYIRLHPTHYSIRSTLRMELMGCDLN SCSMPLGMESKAISDAQITASSYFTNMFATWSPSKARLHLQGRSNAWRPQVNNPKEWLQVDFQKTMKVTGV TTQGVKSLLTSMYVKEFLISSSQDGHQWTLFFQNGKVKVFQGNQDSFTPVVNSLDPPLLTRYLRIHPQSWV HQIALRMEVLGCEAQDLYDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEKTISKAKGQPR EPQVYTLPPSRDELTKNQVSLTCLVKGFYPSDIAVEWESNGQPENNYKTTPPVLDSDGSFFLYSKLTVDKS RWQQGNVFSCSVMHEALHNHYTQKSLSLSPGK |
| ii) Fc chain (20 amino acid heterologous signal peptide from mouse Igκ chain underlined) (SEQ ID NO:4) |
| METDTLLLWVLLLWVPGSTG DKTHTCPPCPAPELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTVDKSRWQQGNVFSCSVMHEALH NHYTQKSLSLSPGK |

| **B. Full length FVIIIFc monomer hybrid (Full length FVIIIFc monomer dimer): created by coexpressing FVIIIFc and Fc chains.** |
|---|
| Construct = HC-B-LC-Fc fusion. An Fc expression cassette is cotransfected with full length FVIII-Fc to generate the full length FVIIIFc monomer. For the FVIIIFc chain, the Fc sequence is shown in bold; HC sequence is shown in double underline; B domain sequence is shown in italics. Signal peptides are underlined. |
| i) Full length FVIIIFc chain (FVIII signal peptide underlined (SEQ ID NO:6) |
| MQIELSTCFFLCLLRFCFS ATRRYYLGAVELSWDYMQSDLGELPVDARFPPRVPKSFPFNTSVVYKKTLFVEFTDHLFNIAKPRPPWMGL LGPTIQAEVYDTVVITLKNMASHPVSLHAVGVSYWKASEGAEYDDQTSQREKEDDKVFPGGSHTYVWQVLK ENGPMASDPLCLTYSYLSHVDLVKDLNSGLIGALLVCREGSLAKEKTQTLHKFILLFAVFDEGKSWHSETK NSLMQDRDAASARAWPKMHTVNGYVNRSLPGLIGCHRKSVYWHVIGMGTTPEVHSIFLEGHTFLVRNHRQA SLEISPITFLTAQTLLMDLGQFLLFCHISSHQHDGMEAYVKVDSCPEEPQLRMKNNEEAEDYDDDLTDSEM DVVRFDDDNSPSFIQIRSVAKKHPKTWVHYIAAEEEDWDYAPLVLAPDDRSYKSQYLNNGPQRIGRKYKKV RFMAYTDETFKTREAIQHESGILGPLLYGEVGDTLLIIFKNQASRPYNIYPHGITDVRPLYSRRLPKGVKH LKDFPILPGEIFKYKWTVTVEDGPTKSDPRCLTRYYSSFVNMERDLASGLIGPLLICYKESVDQRGNQIMS DKRNVILFSVFDENRSWYLTENIQRFLPNPAGVQLEDPEFQASNIMHSINGYVFDSLQLSVCLHEVAYWYI LSIGAQTDFLSVFFSGYTFKHKMVYEDTLTLFPFSGETVFMSMENPGLWILGCHNSDFRNRGMTALLKVSS CDKNTGDYYEDSYEDISAYLLSKNNAIEPRSFSQNSRHPSTRQKQFNATTIPENDIEKTDPWFAHRTPMPK IQNVSSSDLLMLLRQSPTPHGLSLSDLQEAKYETFSDDPSPGAIDSNNSLSEMTHFRPQLHHSGDMVFTPE SGLQLRLNEKLGTTAATELKKLDFKVSSTSNNLISTIPSDNLAAGTDNTSSLGPPSMPVHYDSQLDTTLFG KKSSPLTESGGPLSLSEENNDSKLLESGLMNSQESSWGKNVSSTESGRLFKGKRAHGPALLTKDNALFKVS ISLLKTNKTSNNSATNRKTHIDGPSLLIENSPSVWQNILESDTEFKKVTPLIHDRMLMDKNATALRLNHMS NKTTSSKNMEMVQQKKEGPIPPDAQNPDMSFFKMLFLPESARWIQRTHGKNSLNSGQGPSPKQLVSLGPEK SVEGQNFLSEKNKVVVGKGEFTKDVGLKEMVFPSSRNLFLTNLDNLHENNTHNQEKKIQEEIEKKETLIQE NVVLPQIHTVTGTKNFMKNLFLLSTRQNVEGSYDGAYAPVLQDFRSLNDSTNRTKKHTAHFSKKGEEENLE GLGNQTKQIVEKYACTTRISPNTSQQNFVTQRSKRALKQFRLPLEETELEKRIIVDDTSTQWSKNMKHLTP STLTQIDYNEKEKGAITQSPLSDCLTRSHSIPQANRSPLPIAKVSSFPSIRPIYLTRVLFQDNSSHLPAAS YRKKDSGVQESSHFLQGAKKNNLSLAILTLEMTGDQREVGSLGTSATNSVTYKKVENTVLPKPDLPKTSGK VELLPKVHIYQKDLFPTETSNGSPGHLDLVEGSLLQGTEGAIKWNEANRPGKVPFLRVATESSAKTPSKLL DPLAWDNHYGTQIPKEEWKSQEKSPEKTAFKKKDTILSLNACESNHAIAAINEGQNKPEIEVTWAKQGRTE RLCSQNPPVLKRHQREITRTTLQSDQEEIDYDDTISVEMKKEDFDIYDEDENQSPRSFQKKTRHYFIAAVE RLWDYGMSSSPHVLRNRAQSGSVPQFKKVVFQEFTDGSFTQPLYRGELNEHLGLLGPYIRAEVEDNIMVTF RNQASRPYSFYSSLISYEEDQRQGAEPRKNFVKPNETKTYFWKVQHHMAPTKDEFDCKAWAYFSDVDLEKD VHSGLIGPLLVCHTNTLNPAHGRQVTVQEFALFFTIFDETKSWYFTENMERNCRAPCNIQMEDPTFKENYR FHAINGYIMDTLPGLVMAQDQRIRWYLLSMGSNENIHSIHFSGHVFTVRKKEEYKMALYNLYPGVFETVEM LPSKAGIWRVECLIGEHLHAGMSTLFLVYSNKCQTPLGMASGHIRDFQITASGQYGQWAPKLARLHYSGSI NAWSTKEPFSWIKVDLLAPMIIHGIKTQGARQKFSSLYISQFIIMYSLDGKKWQTYRGNSTGTLMVFFGNV DSSGIKHNIFNPPIIARYIRLHPTHYSIRSTLRMELMGCDLNSCSMPLGMESKAISDAQITASSYFTNMFA TWSPSKARLHLQGRSNAWRPQVNNPKEWLQVDFQKTMKVTGVTTQGVKSLLTSMYVKEFLISSSQDGHQWT LFFQNGKVKVFQGNQDSFTPVVNSLDPPLLTRYLRIHPQSWVHQIALRMEVLGCEAQDLYDKTHTCPPCPA PELLGGPSVFLFPPKPKDTLMISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVYTLPPSRDELTKNQVSLTCLVKGFY PSDIAVEWESNGQPENNYKTTPPVLDSDGSFFLYSKLTVDKSRWQQGNVFSCSVMHEALHNHYTQKSLSLS PGK |
| ii) Fc chain (20 amino acid heterologous signal peptide from mouse Igκ chain underlined) (SEQ ID NO:4) |
| METDTLLLWVLLLWVPGSTG DKTHTCPPCPAPELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTVDKSRWQQGNVFSCSVMHEALH NHYTQKSLSLSPGK |

### Cited References

4. Aledort L. et al., J Intern Med.:236:391-399 (1994)
5. Petrini P. et al., Am J Pediatr Hematol Oncol. 13:280-287 (1991).
6. Aznar J. et al., Haemophilia 6(3):170-176 (2000).
7. Feldman B. et al., J Thromb Haemost. 4:1228-1236 (2006).
8. Kreuz W. et al., Haemophilia 4:413-417 (1998).
9. Liesner R. et al., B J Haem. 92:973-978 (1996).
10. Ljung R., Haemophilia. 4(4):409-412 (1998).
11. Löfquist T, et al., J Intern Med 241:395-400 (1997).
12. Nilsson I, et al., B. J Int Med 232:25-32 (1992).
13. Risebrough N. et al., Haemophilia. 14:743-752 (2008).
14. Van Den Berg H. et al., Haemophilia 9 (Suppl. 1):27-31 (2003).
15. Van Den Berg H. et al., Haematologica 89(6):645-650 (2004).
16. Molho P. et al., Haemophilia 6(1):23-32 (2000).
17. Coppola A. et al., Blood Transfus. 6(2): 4-11 (2008).
18. Geraghty S. et al., Haemophilia 12:75-81 (2006).
19. Hacker M. et al., Haemophilia 7(4):392-396 (2001).
20. Lillicrap D., Current Opinion in Hematology 17:393-397 (2010).
21. Dumont J.A. et al., BioDrugs 20(3): 151-60 (2006).
22. Dumont J.A. et al., "Monomeric Fc fusion technology: an approach to create long lasting clotting factors," in: Kontermann R., ed., Therapeutic Proteins - Strategies to Modulate Half-Life, Chapter 11, Wiley VCH publisher; in press.
23. Roopenian D.C. et al., Nat Rev Immunol. 7(9):715-25 (Epub 2007 Aug 17).
24. Lencer W.I. and Blumberg R.S., Trends Cell Biol. 15(1):5-9 (2005).
25. Huang C., Curr Opin Biotechnol. 20(6):692-9. (Epub 2009 Nov 4).
26. Schmidt S.R., Curr Opin Drug Discov Devel. 12(2):284-295 (2009).
27. Dumont J. et al., Blood. 116(21) Abstract 545 (2009).
28. Liu T. et al., J Thromb Haemost. 9(S2):561 (2011).
29. Rosen S., Scand J Haematol Suppl. 33(Suppl 40):139-45 (1984).
30. Lee C.A. et al., Thromb Haemost. 82(6):1644-7 (Dec. 1999).
31. Mikaelsson M. and Oswaldsson U., Semin Thromb Hemost. 28(3):257-64 (June 2002).
32. Stroobants A.K. et al., J Thromb Haemost. 9 (Suppl 2) (2011).
33. Lenting P.J. et al., J Thromb Haemost. 5: 1353-60 (2007).
34. Smith N.L. et al., Circulation 121:1382-1392 (2010).

## Claims

1. A pharmaceutical composition, comprising
(i) a chimeric polypeptide, which comprises a Factor VIII (FVIII) portion and a second portion, and
(ii) at least one pharmaceutically acceptable excipient,
wherein
in 1% to 40% of the FVIII portion, the FVIII is in the form of a single chain FVIII, and wherein in 60 to 99% of the FVIII portion, the FVIII portion is processed, and
wherein the single chain FVIII comprises a FVIII heavy chain and a FVIII light chain on a single polypeptide chain,
wherein the single chain FVIII comprises an amino acid sequence at least 90% or 95% identical to amino acids 20 to 1457 of SEQ ID NO: 2, and
wherein the second portion comprises an amino acid sequence at least 90% or 95% identical to amino acids 21 to 247 of SEQ ID NO: 4.

2. The pharmaceutical composition of claim 1, wherein
(a) 15% of the FVIII portion of the chimeric polypeptide is single chain FVIII,
(b) 20% of the FVIII portion of the chimeric polypeptide is single chain FVIII,
(c) 25% of the FVIII portion of the chimeric polypeptide is single chain FVIII,
(d) 30% of the FVIII portion of the chimeric polypeptide is single chain FVIII,
(e) 35% of the FVIII portion of the chimeric polypeptide is single chain FVIII, or
(f) 40% of the FVIII portion of the chimeric polypeptide is single chain FVIII.

3. The pharmaceutical composition of claim 1 or 2, wherein the chimeric polypeptide is a rFVIIIFc monomer dimer hybrid.

4. The pharmaceutical composition of any one of claims 1 to 3, wherein the single chain FVIII comprises an amino acid sequence identical to amino acids 20 to 1457 of SEQ ID NO: 2.

5. The pharmaceutical composition of any one of claims 1 to 4, wherein the rFVIIIFc monomer dimer hybrid has been produced by human embryonic kidney 293 (HEK293) cells.

6. The pharmaceutical composition of claim 5, wherein the second portion comprises an amino acid sequence identical to amino acids 21 to 247 of SEQ ID NO: 4.

7. The pharmaceutical composition of any one of claims 1 to 6, which is suitable for subcutaneous or intravenous injection into a human.

8. The pharmaceutical composition of any one of claims 1 to 7, for use in reducing bleeding in a subject who has hemophilia A.

9. The pharmaceutical composition for use according to claim 8, for use in preventing, decreasing, or treating a bleeding episode in the subject, prophylactic treatment of a bleeding episode in the subject, on-demand treatment of a bleeding episode in the subject.

10. The pharmaceutical composition for use according to claim 9, wherein the bleeding episode is associated with hemarthrosis, muscle bleed, oral bleed, hemorrhage, hemorrhage into muscles, oral hemorrhage, trauma, trauma capitis (head trauma), gastrointestinal bleeding, intracranial hemorrhage, intraabdominal hemorrhage, intrathoracic hemorrhage, bone fracture, central nervous system bleeding, bleeding in the retropharyngeal space, bleeding in the retroperitoneal space, or bleeding in the iliopsoas sheath.

11. The pharmaceutical composition for use according to claim 9, wherein the subject is in need of surgery.

12. The pharmaceutical composition for use according to claim 11, wherein the surgery is minor surgery, major surgery, tooth extraction, tonsillectomy, inguinal hemiotomy, synovectomy, total knee replacement, craniotomy, osteosynthesis, trauma surgery, intracranial surgery, intra-abdominal surgery, intrathoracic surgery, emergency surgery, or joint replacement surgery.

13. The pharmaceutical composition of any one of claims 1 to 7, or the pharmaceutical composition for use according to any one of claims 8 to 12, which is lyophilized.

## Patentansprüche

1. Pharmazeutische Zusammensetzung, umfassend:
(i) ein chimäres Polypeptid, das einen Faktor VIII(FVIII)-Teil und einen zweiten Teil umfasst, und
(ii) mindestens einen pharmazeutisch akzeptablen Exzipienten,
wobei
bei 1 % bis 40 % des FVIII-Teils der FVIII in Form eines einkettigen FVIII vorliegt und
wobei bei 60 bis 99 % des FVIII-Teils der FVIII-Teil prozessiert ist und
wobei der einkettige FVIII eine schwere FVIII-Kette und eine leichte FVIII-Kette auf einer einzigen Polypeptidkette umfasst,
wobei der einkettige FVIII eine Aminosäuresequenz umfasst, die mindestens zu 90 % oder 95 % identisch mit den Aminosäuren 20 bis 1457 von SEQ ID NO: 2 ist, und
wobei der zweite Teil eine Aminosäuresequenz umfasst, die mindestens zu 90 % oder 95 % identisch mit den Aminosäuren 21 bis 247 von SEQ ID NO: 4 ist.

2. Pharmazeutische Zusammensetzung nach Anspruch 1, wobei
(a) 15 % des FVIII-Teils des chimären Polypeptids als einkettiger FVIII vorliegen,
(b) 20 % des FVIII-Teils des chimären Polypeptids als einkettiger FVIII vorliegen,
(c) 25 % des FVIII-Teils des chimären Polypeptids als einkettiger FVIII vorliegen,
(d) 30 % des FVIII-Teils des chimären Polypeptids als einkettiger FVIII vorliegen,
(e) 35 % des FVIII-Teils des chimären Polypeptids als einkettiger FVIII vorliegen oder
(f) 40 % des FVIII-Teils des chimären Polypeptids als einkettiger FVIII vorliegen.

3. Pharmazeutische Zusammensetzung nach Anspruch 1 oder 2, wobei es sich bei dem chimären Polypeptid um ein rFVIIIFc-Monomer-Dimer-Hybrid handelt.

4. Pharmazeutische Zusammensetzung nach einem der Ansprüche 1 bis 3, wobei der einkettige FVIII eine mit den Aminosäuren 20 bis 1457 von SEQ ID NO: 2 identische Aminosäuresequenz umfasst.

5. Pharmazeutische Zusammensetzung nach einem der Ansprüche 1 bis 4, wobei das rFVIIIFc-Monomer-Dimer-Hybrid von HEK293(Human Embryonic Kidney 293)-Zellen produziert wurde.

6. Pharmazeutische Zusammensetzung nach Anspruch 5, wobei der zweite Teil eine mit den Aminosäuren 21 bis 247 von SEQ ID NO: 4 identische Aminosäuresequenz umfasst.

7. Pharmazeutische Zusammensetzung nach einem der Ansprüche 1 bis 6, die zur subkutanen oder intravenösen Injektion in einen Menschen geeignet ist.

8. Pharmazeutische Zusammensetzung nach einem der Ansprüche 1 bis 7 zur Verwendung bei der Verringerung von Blutungen bei einem Individuum, das an Hämophilie A leidet.

9. Pharmazeutische Zusammensetzung zur Verwendung gemäß Anspruch 8, zur Verwendung bei der Vorbeugung, Verkürzung oder Behandlung einer Blutungsepisode bei dem Individuum, prophylaktischen Behandlung einer Blutungsepisode bei dem Individuum, bedarfsgerechten Behandlung einer Blutungsepisode bei dem Individuum.

10. Pharmazeutische Zusammensetzung zur Verwendung gemäß Anspruch 9, wobei die Blutungsepisode mit Hämarthrose, Muskelblutung, Mundblutung, Hämorrhagie, Hämorrhagie in die Muskulatur, oraler Hämorrhagie, Trauma, Trauma capitis (Kopftrauma), Magen-DarmBlutungen, intrakranieller Hämorrhagie, intraabdominaler Hämorrhagie, intrathorakaler Hämorrhagie, Knochenfraktur, Blutungen des Zentralnervensystems, Blutungen im retropharyngealen Raum, Blutungen im Retroperitoneum oder Blutungen in der Iliopsoas-Scheide assoziiert ist.

11. Pharmazeutische Zusammensetzung zur Verwendung gemäß Anspruch 9, wobei das Individuum eine Operation benötigt.

12. Pharmazeutische Zusammensetzung zur Verwendung gemäß Anspruch 11, wobei es sich bei der Operation um eine kleinere Operation, größere Operation, Zahnextraktion, Tonsillektomie, Leistenbruchoperation, Synovektomie, Knie-Vollprothese, Kraniotomie, Osteosynthese, Unfallchirurgie, intrakranielle Operation, intraabdominale Operation, intrathorakale Operation, Notoperation oder Gelenkersatzoperation handelt.

13. Pharmazeutische Zusammensetzung nach einem der Ansprüche 1 bis 7 oder pharmazeutische Zusammensetzung zur Verwendung gemäß einem der Ansprüche 8 bis 12, die lyophilisiert ist.

## Revendications

1. Composition pharmaceutique, comprenant
(i) un polypeptide chimérique, qui comprend une partie de facteur VIII (FVIII) et une deuxième partie, et
(ii) au moins un excipient pharmaceutiquement acceptable,
dans laquelle
dans 1 à 40 % de la partie FVIII, le FVIII est sous la forme d'un FVIII monocaténaire, et
dans laquelle, dans 60 à 99 % de la partie FVIII, la partie FVIII est transformée, et
dans laquelle la chaîne FVIII monocaténaire comprend une chaîne lourde de FVIII et une chaîne légère de FVIII sur une chaîne polypeptidique unique,
dans laquelle le FVIII monocaténaire comprend une séquence d'acides aminés au moins 90 % ou 95 % identique aux acides aminés 20 à 1457 de SEQ ID NO: 2, et
dans laquelle la deuxième partie comprend une séquence d'acides aminés au moins 90 % ou 95 % identique aux acides aminés 21 à 247 de SEQ ID NO: 4.

2. Composition pharmaceutique selon la revendication 1, dans laquelle
(a) 15 % de la partie FVIII du polypeptide chimérique est FVIII monocaténaire,
(b) 20 % de la partie FVIII du polypeptide chimérique est FVIII monocaténaire,
(c) 25 % de la partie FVIII du polypeptide chimérique est FVIII monocaténaire,
(d) 30 % de la partie FVIII du polypeptide chimérique est FVIII monocaténaire,
(e) 35 % de la partie FVIII du polypeptide chimérique est FVIII monocaténaire, ou
(f) 40 % de la partie FVIII du polypeptide chimérique est FVIII monocaténaire.

3. Composition pharmaceutique selon la revendication 1 ou 2, dans laquelle le polypeptide chimérique est un hybride dimère monomère de rFVIIIFc.

4. Composition pharmaceutique selon l'une quelconque des revendications 1 à 3, dans laquelle le FVIII monocaténaire comprend une séquence d'acides aminés identique aux acides aminés 20 à 1457 de SEQ ID NO: 2.

5. Composition pharmaceutique selon l'une quelconque des revendications 1 à 4, dans laquelle l'hybride dimère monomère de rFVIIIFc a été produit par des cellules rénales embryonnaires humaines 293 (HEK293).

6. Composition pharmaceutique selon la revendication 5, dans laquelle la deuxième partie comprend une séquence d'acides aminés identique aux acides aminés 21 à 247 de SEQ ID NO: 4.

7. Composition pharmaceutique selon l'une quelconque des revendications 1 à 6, qui convient pour une injection sous-cutanée ou intraveineuse chez un humain.

8. Composition pharmaceutique selon l'une quelconque des revendications 1 à 7, destinée à être utilisée pour réduire un saignement chez un sujet atteint d'hémophilie A.

9. Composition pharmaceutique destinée à être utilisée selon la revendication 8, destinée à être utilisée dans la prévention, la diminution ou le traitement d'un épisode de saignement chez le sujet, le traitement prophylactique d'un épisode de saignement chez le sujet, le traitement à la demande d'un épisode de saignement chez le sujet.

10. Composition pharmaceutique destinée à être utilisée selon la revendication 9, dans laquelle l'épisode de saignement est associé à une hémarthrose, un saignement musculaire, un saignement buccal, une hémorragie, une hémorragie dans les muscles, une hémorragie buccale, un traumatisme, un traumatisme crânien, un saignement gastro-intestinal, une hémorragie intracrânienne, une hémorragie intra-abdominale, une hémorragie intrathoracique, une fracture osseuse, un saignement du système nerveux central, un saignement dans l'espace rétropharyngé, un saignement dans l'espace rétropéritonéal ou un saignement dans la gaine de l'iliopsoas.

11. Composition pharmaceutique destinée à être utilisée selon la revendication 9, dans laquelle le sujet a besoin d'une intervention chirurgicale.

12. Composition pharmaceutique destinée à être utilisée selon la revendication 11, dans laquelle l'intervention chirurgicale est une intervention chirurgicale mineure, une intervention chirurgicale majeure, une extraction dentaire, une amygdalectomie, une hémiotomie inguinale, une synovectomie, une prothèse totale du genou, une craniotomie, une ostéosynthèse, une chirurgie traumatologique, une chirurgie intracrânienne, une chirurgie intra-abdominale, une chirurgie intrathoracique, une chirurgie d'urgence ou une chirurgie de remplacement articulaire.

13. Composition pharmaceutique selon l'une quelconque des revendications 1 à 7, ou composition pharmaceutique destinée à être utilisée selon l'une quelconque des revendications 8 à 12, qui est lyophilisée.
